# EUROPEAN PATENT SPECIFICATION

(11) **EP 2 173 358 B1**
(45) Date of publication and mention of the grant of the patent: **21.10.2015**
(21) Application number: 08771759.1
(22) Date of filing: 23.06.2008
(51) Int. Cl.: C12N 15/113, C12N 15/11

(54) **DOUBLE STRAND COMPOSITIONS COMPRISING DIFFERENTIALLY MODIFIED STRANDS FOR USE IN GENE MODULATION**
DOPPELSTRÄNGIGE ZUSAMMENSETZUNGEN MIT UNTERSCHIEDLICH MODIFIZIERTEN STRÄNGEN ZUR VERWENDUNG BEI DER GENMODULATION
COMPOSITIONS DOUBLE BRIN COMPRENANT DES BRINS MODIFIÉS DE MANIÈRE DIFFÉRENTIELLE POUR UTILISATION DANS LA MODULATION DE GÈNE

(30) Priority: 22.06.2007 US 945837 P
(43) Date of publication of application: 14.04.2010
(73) Proprietor: Isis Pharmaceuticals, Inc., Carlsbad, CA 92010 (US)
(72) Inventor: BHAT, Balkrishen, Carlsbad, CA 92009 (US); PRAKASH, Thazha, P., Carlsbad, CA 92009 (US); ALLERSON, Charles, San Diego, CA 92130 (US); KINBERGER, Garth, A., San Diego, CA 92130 (US); MARCUSSON, Eric, G., San Francisco, CA 94107 (US); SWAYZE, Eric, E., Encinitas, CA 92024 (US)
(74) Representative: Warner, James Alexander
(86) International application number: PCT/US2008/067932
(87) International publication number: WO 2009/002944

(56) References cited:
- WO-A2-2004/113496
- WO-A2-2005/121372
- US-A1- 2001 029 035
- US-A1- 2005 261 218
- US-A1- 2007 049 547
- US-A1- 2007 123 484
- DANDE PRASAD ET AL: "Improving RNA interference in mammalian cells by 4'-thio-modified small interfering RNA (siRNA): effect on siRNA activity and nuclease stability when used in combination with 2'-O-alkyl modifications", JOURNAL OF MEDICINAL CHEMISTRY, AMERICAN CHEMICAL SOCIETY, US, vol. 49, no. 5, 9 March 2006 (2006-03-09), pages 1624-1634, XP002611368, ISSN: 0022-2623, DOI: 10.1021/JM050822C [retrieved on 2006-02-08]
- ALLERSON C R ET AL: "Fully 2'-modified oligonucleotide duplexes with improved in vitro potency and stability compared to unmodified small interfering RNA", JOURNAL OF MEDICINAL CHEMISTRY, AMERICAN CHEMICAL SOCIETY, US, vol. 48, no. 4, 20 January 2005 (2005-01-20), pages 901-904, XP002404763, ISSN: 0022-2623, DOI: 10.1021/JM049167J

### SEQUENCE LISTING

<110> Isis Pharmaceuticals, Inc.
   Bhat, Balkrishen
   Prakash, Thazha P.
   Allerson, Charles
   Kinberger, Garth A.
   Marcusson, Eric G.
   Swayze, Eric E.
<120> DOUBLE STRAND COMPOSITIONS COMPRISING DIFFERENTIALLY MODIFIED STRANDS FOR USE IN GENE MODULATION
<130> CORE0074WO
<150> 60/945,837 <151> 2007-06-22
<160> 68
<170> FastSEQ for Windows Version 4.0
<210> 1
   <211> 20
   <212> DNA
   <213> Artificial Sequence
<220>
   <223> Synthetic Oligonucleotide
<400> 1
   tccgtcatcg ctcctcaggg 20
<210> 2
   <211> 20
   <212> DNA
   <213> Artificial Sequence
<220>
   <223> Synthetic Oligonucleotide
<400> 2
   gtgcgcgcga gcccgaaatc 20
<210> 3
   <211> 20
   <212> DNA
   <213> Artificial Sequence
<220>
   <223> Synthetic Oligonucleotide
<400> 3
   atgcattctg cccccaagga 20
<210> 4
   <211> 3160
   <212> DNA
   <213> H. Sapiens
<400> 4
   cctcccctcg cccggcgcgg tcccgtccgc ctctcgctcg cctcccgcct cccctcggtc 60
   ttccgaggcg cccgggctcc cggcgcggcg gcggaggggg cgggcaggcc ggcgggcggt 120
   gatgtggcag gactctttat gcgctgcggc aggatacgcg ctcggcgctg ggacgcgact 180
   gcgctcagtt ctctcctctc ggaagctgca gccatgatgg aagtttgaga gttgagccgc 240
   tgtgaggcga ggccgggctc aggcgaggga gatgagagac ggcggcggcc gcggcccgga 300
   gcccctctca gcgcctgtga gcagccgcgg gggcagcgcc ctcggggagc cggccggcct 360
   gcggcggcgg cagcggcggc gtttctcgcc tcctcttcgt cttttctaac cgtgcagcct 420
   cttcctcggc ttctcctgaa agggaaggtg gaagccgtgg gctcgggcgg gagccggctg 480
   aggcgcggcg gcggcggcgg cggcacctcc cgctcctgga gcggggggga gaagcggcgg 540
   cggcggcggc cgcggcggct gcagctccag ggagggggtc tgagtcgcct gtcaccattt 600
   ccagggctgg gaacgccgga gagttggtct ctccccttct actgcctcca acacggcggc 660
   ggcggcggcg gcacatccag ggacccgggc cggttttaaa cctcccgtcc gccgccgccg 720
   caccccccgt ggcccgggct ccggaggccg ccggcggagg cagccgttcg gaggattatt 780
   cgtcttctcc ccattccgct gccgccgctg ccaggcctct ggctgctgag gagaagcagg 840
   cccagtcgct gcaaccatcc agcagccgcc gcagcagcca ttacccggct gcggtccaga 900
   gccaagcggc ggcagagcga ggggcatcag ctaccgccaa gtccagagcc atttccatcc 960
   tgcagaagaa gccccgccac cagcagcttc tgccatctct ctcctccttt ttcttcagcc 1020
   acaggctccc agacatgaca gccatcatca aagagatcgt tagcagaaac aaaaggagat 1080
   atcaagagga tggattcgac ttagacttga cctatattta tccaaacatt attgctatgg 1140
   gatttcctgc agaaagactt gaaggcgtat acaggaacaa tattgatgat gtagtaaggt 1200
   ttttggattc aaagcataaa aaccattaca agatatacaa tctttgtgct gaaagacatt 1260
   atgacaccgc caaatttaat tgcagagttg cacaatatcc ttttgaagac cataacccac 1320
   cacagctaga acttatcaaa cccttttgtg aagatcttga ccaatggcta agtgaagatg 1380
   acaatcatgt tgcagcaatt cactgtaaag ctggaaaggg acgaactggt gtaatgatat 1440
   gtgcatattt attacatcgg ggcaaatttt taaaggcaca agaggcccta gatttctatg 1500
   gggaagtaag gaccagagac aaaaagggag taactattcc cagtcagagg cgctatgtgt 1560
   attattatag ctacctgtta aagaatcatc tggattatag accagtggca ctgttgtttc 1620
   acaagatgat gtttgaaact attccaatgt tcagtggcgg aacttgcaat cctcagtttg 1680
   tggtctgcca gctaaaggtg aagatatatt cctccaattc aggacccaca cgacgggaag 1740
   acaagttcat gtactttgag ttccctcagc cgttacctgt gtgtggtgat atcaaagtag 1800
   agttcttcca caaacagaac aagatgctaa aaaaggacaa aatgtttcac ttttgggtaa 1860
   atacattctt cataccagga ccagaggaaa cctcagaaaa agtagaaaat ggaagtctat 1920
   gtgatcaaga aatcgatagc atttgcagta tagagcgtgc agataatgac aaggaatatc 1980
   tagtacttac tttaacaaaa aatgatcttg acaaagcaaa taaagacaaa gccaaccgat 2040
   acttttctcc aaattttaag gtgaagctgt acttcacaaa aacagtagag gagccgtcaa 2100
   atccagaggc tagcagttca acttctgtaa caccagatgt tagtgacaat gaacctgatc 2160
   attatagata ttctgacacc actgactctg atccagagaa tgaacctttt gatgaagatc 2220
   agcatacaca aattacaaaa gtctgaattt ttttttatca agagggataa aacaccatga 2280
   aaataaactt gaataaactg aaaatggacc tttttttttt taatggcaat aggacattgt 2340
   gtcagattac cagttatagg aacaattctc ttttcctgac caatcttgtt ttaccctata 2400
   catccacagg gttttgacac ttgttgtcca gttgaaaaaa ggttgtgtag ctgtgtcatg 2460
   tatatacctt tttgtgtcaa aaggacattt aaaattcaat taggattaat aaagatggca 2520
   ctttcccgtt ttattccagt tttataaaaa gtggagacag actgatgtgt atacgtagga 2580
   attttttcct tttgtgttct gtcaccaact gaagtggcta aagagctttg tgatatactg 2640
   gttcacatcc tacccctttg cacttgtggc aacagataag tttgcagttg gctaagagag 2700
   gtttccgaaa ggttttgcta ccattctaat gcatgtattc gggttagggc aatggagggg 2760
   aatgctcaga aaggaaataa ttttatgctg gactctggac catataccat ctccagctat 2820
   ttacacacac ctttctttag catgctacag ttattaatct ggacattcga ggaattggcc 2880
   gctgtcactg cttgttgttt gcgcattttt ttttaaagca tattggtgct agaaaaggca 2940
   gctaaaggaa gtgaatctgt attggggtac aggaatgaac cttctgcaac atcttaagat 3000
   ccacaaatga agggatataa aaataatgtc ataggtaaga aacacagcaa caatgactta 3060
   accatataaa tgtggaggct atcaacaaag aatgggcttg aaacattata aaaattgaca 3120
   atgatttatt aaatatgttt tctcaattgt aaaaaaaaaa 3160
<210> 5
   <211> 26
   <212> DNA
   <213> Artificial Sequence
<220>
   <223> Primer
<400> 5
   aatggctaag tgaagatgac aatcat 26
<210> 6
   <211> 25
   <212> DNA
   <213> Artificial Sequence
<220>
   <223> Primer
<400> 6
   tgcacatatc attacaccag ttcgt 25
<210> 7
   <211> 30
   <212> DNA
   <213> Artificial Sequence
<220>
   <223> Probe
<400> 7
   ttgcagcaat tcactgtaaa gctggaaagg 30
<210> 8
   <211> 1619
   <212> DNA
   <213> H. Sapiens
<400> 8
   ccgccagatt tgaatcgcgg gacccgttgg cagaggtggc ggcggcggca tgggtgcccc 60
   gacgttgccc cctgcctggc agccctttct caaggaccac cgcatctcta cattcaagaa 120
   ctggcccttc ttggagggct gcgcctgcac cccggagcgg atggccgagg ctggcttcat 180
   ccactgcccc actgagaacg agccagactt ggcccagtgt ttcttctgct tcaaggagct 240
   ggaaggctgg gagccagatg acgaccccat agaggaacat aaaaagcatt cgtccggttg 300
   cgctttcctt tctgtcaaga agcagtttga agaattaacc cttggtgaat ttttgaaact 360
   ggacagagaa agagccaaga acaaaattgc aaaggaaacc aacaataaga agaaagaatt 420
   tgaggaaact gcgaagaaag tgcgccgtgc catcgagcag ctggctgcca tggattgagg 480
   cctctggccg gagctgcctg gtcccagagt ggctgcacca cttccagggt ttattccctg 540
   gtgccaccag ccttcctgtg ggccccttag caatgtctta ggaaaggaga tcaacatttt 600
   caaattagat gtttcaactg tgctcctgtt ttgtcttgaa agtggcacca gaggtgcttc 660
   tgcctgtgca gcgggtgctg ctggtaacag tggctgcttc tctctctctc tctctttttt 720
   gggggctcat ttttgctgtt ttgattcccg ggcttaccag gtgagaagtg agggaggaag 780
   aaggcagtgt cccttttgct agagctgaca gctttgttcg cgtgggcaga gccttccaca 840
   gtgaatgtgt ctggacctca tgttgttgag gctgtcacag tcctgagtgt ggacttggca 900
   ggtgcctgtt gaatctgagc tgcaggttcc ttatctgtca cacctgtgcc tcctcagagg 960
   acagtttttt tgttgttgtg tttttttgtt tttttttttt ggtagatgca tgacttgtgt 1020
   gtgatgagag aatggagaca gagtccctgg ctcctctact gtttaacaac atggctttct 1080
   tattttgttt gaattgttaa ttcacagaat agcacaaact acaattaaaa ctaagcacaa 1140
   agccattcta agtcattggg gaaacggggt gaacttcagg tggatgagga gacagaatag 1200
   agtgatagga agcgtctggc agatactcct tttgccactg ctgtgtgatt agacaggccc 1260
   agtgagccgc ggggcacatg ctggccgctc ctccctcaga aaaaggcagt ggcctaaatc 1320
   ctttttaaat gacttggctc gatgctgtgg gggactggct gggctgctgc aggccgtgtg 1380
   tctgtcagcc caaccttcac atctgtcacg ttctccacac gggggagaga cgcagtccgc 1440
   ccaggtcccc gctttctttg gaggcagcag ctcccgcagg gctgaagtct ggcgtaagat 1500
   gatggatttg attcgccctc ctccctgtca tagagctgca gggtggattg ttacagcttc 1560
   gctggaaacc tctggaggtc atctcggctg ttcctgagaa ataaaaagcc tgtcatttc  1619
<210> 9
   <211> 22
   <212> DNA
   <213> Artificial Sequence
<220>
   <223> Primer
<400> 9
   caccacttcc agggtttatt cc 22
<210> 10
   <211> 25
   <212> DNA
   <213> Artificial Sequence
<220>
   <223> Primer
<400> 10
   tgatctcctt tcctaagaca ttgct 25
<210> 11
   <211> 22
   <212> DNA
   <213> Artificial Sequence
<220>
   <223> Probe
<400> 11
   accagccttc ctgtgggccc ct 22
<210> 12
   <211> 1842
   <212> DNA
   <213> H. Sapiens
<400> 12
   cgatcagatc gatctaagat ggcgactgtc gaaccggaaa ccacccctac tcctaatccc 60
   ccgactacag aagaggagaa aacggaatct aatcaggagg ttgctaaccc agaacactat 120
   attaaacatc ccctacagaa cagatgggca ctctggtttt ttaaaaatga taaaagcaaa 180
   acttggcaag caaacctgcg gctgatctcc aagtttgata ctgttgaaga cttttgggct 240
   ctgtacaacc atatccagtt gtctagtaat ttaatgcctg gctgtgacta ctcacttttt 300
   aaggatggta ttgagcctat gtgggaagat gagaaaaaca aacggggagg acgatggcta 360
   attacattga acaaacagca gagacgaagt gacctcgatc gcttttggct agagacactt 420
   ctgtgcctta ttggagaatc ttttgatgac tacagtgatg atgtatgtgg cgctgttgtt 480
   aatgttagag ctaaaggtga taagatagca atatggacta ctgaatgtga aaacagagaa 540
   gctgttacac atatagggag ggtatacaag gaaaggttag gacttcctcc aaagatagtg 600
   attggttatc agtcccacgc agacacagct actaagagcg gctccaccac taaaaatagg 660
   tttgttgttt aagaagacac cttctgagta ttctcatagg agactgcgtc aagcaatcga 720
   gatttgggag ctgaaccaaa gcctcttcaa aaagcagagt ggactgcatt taaatttgat 780
   ttccatctta atgttactca gatataagag aagtctcatt cgcctttgtc ttgtacttct 840
   gtgttcattt tttttttttt tttttggcta gagtttccac tatcccaatc aaagaattac 900
   agtacacatc cccagaatcc ataaatgtgt tcctggccca ctctgtaata gttcagtaga 960
   attaccatta attacataca gattttacct atccacaata gtcagaaaac aacttggcat 1020
   ttctatactt tacaggaaaa aaaattctgt tgttccattt tatgcagaag catattttgc 1080
   tggtttgaaa gattatgatg catacagttt tctagcaatt ttctttgttt ctttttacag 1140
   cattgtcttt gctgtactct tgctgatggc tgctagattt taatttattt gtttccctac 1200
   ttgataatat tagtgattct gatttcagtt tttcatttgt tttgcttaaa tttttttttt 1260
   ttttttcctc atgtaacatt ggtgaaggat ccaggaatat gacacaaagg tggaataaac 1320
   attaattttg tgcattcttt ggtaattttt tttgtttttt gtaactacaa agctttgcta 1380
   caaatttatg catttcattc aaatcagtga tctatgtttg tgtgatttcc taaacataat 1440
   tgtggattat aaaaaatgta acatcataat tacattccta actagaatta gtatgtctgt 1500
   ttttgtatct ttatgctgta ttttaacact ttgtattact taggttattt tgctttggtt 1560
   aaaaatggct caagtagaaa agcagtccca ttcatattaa gacagtgtac aaaactgtaa 1620
   ataaaatgtg tacagtgaat tgtcttttag acaactagat ttgtcctttt atttctccat 1680
   ctttatagaa ggaatttgta cttcttattg caggcaagtc tctatattat gtcctctttt 1740
   gtggtgtctt ccatgtgaac agcataagtt tggagcacta gtttgattat tatgtttatt 1800
   acaattttta ataaattgaa taggtagtat catatatatg ga 1842
<210> 13
   <211> 17
   <212> DNA
   <213> Artificial Sequence
<220>
   <223> Primer
<400> 13
   tggcgactgt cgaaccg 17
<210> 14
   <211> 26
   <212> DNA
   <213> Artificial Sequence
<220>
   <223> Primer
<400> 14
   agattccgtt ttctcctctt ctgtag 26
<210> 15
   <211> 26
   <212> DNA
   <213> Artificial Sequence
<220>
   <223> Probe
<400> 15
   aaaccacccc tactcctaat cccccg 26
<210> 16
   <211> 2763
   <212> DNA
   <213> Mus musculus
<400> 16
   gagatcgatc taagatggcg actgtggaac cggaaaccac ccctaccact aatcccccac 60
   ctgcagaaga ggaaaaaaca gagtctaatc aagaggttgc taacccagag cactatatta 120
   aacaccctct acagaacagg tgggcactct ggttttttaa aaatgataaa agcaaaactt 180
   ggcaagcaaa ccttcgattg atctctaagt ttgatactgt tgaagacttt tgggctctat 240
   acaaccatat ccagttgtct agtaatttaa tgcctggctg tgactactca ctttttaagg 300
   acgggattga gcctatgtgg gaagatgaga aaaacaaacg aggaggacgg tggctgatca 360
   cactgaacaa gcagcagaga cggagtgacc tcgatcgctt ctggctagag acactgctgt 420
   gccttattgg agaatctttc gatgactaca gtgatgatgt gtgtggagct gttgttaatg 480
   ttagagctaa aggcgataag atagcaatat ggactactga gagtgaaaac agagatgcag 540
   tcacacacat agggagggta tacaaggaaa ggttaggact tcctccgaag atagtgattg 600
   gttatcagtc ccacgcagac acagctacaa agagcggctc caccactaaa aataggtttg 660
   ttgtttaaaa agacaccttc tgagtattct cacaggagac tgcgtcacgc aatcgagatt 720
   gggagctgaa ccaaagcctc atcaaagcag agtggactgc actgaagttg attccatcca 780
   agtgttgcta agatataaga gaagtctcat tcgcctttgt cttgtacttc tgtgttcatt 840
   ctcctccccc acccccaatt tttgctagtg tgtccactat cccaatcaaa gaattacagt 900
   atacgtcacc ccagaacccg cagatgtgtt cctggcccgc tctgtaacag ccggttagaa 960
   ttaccatgac acacacattt gcctttccac agtattcgaa aaagaacttg catttctatt 1020
   accttagcag gaaagatctg gttttgctcc actccatgca ggagcggact ttgctggtgt 1080
   gagagtctga gtacagcttt ctagcaacct tctgtttcct ttcacagcat tgtccttgct 1140
   gtcctcttgc tgatggctgc tagatttaat ttatttgctt ccctccttga taacattagt 1200
   gattctgatt tcagtttttc atttgttttg cttttgtttt tttcctcgtg taacattggt 1260
   gaaggatcca ggaatatgac agaaaggtgg aataaacatt aaatttgtgc attctttggt 1320
   aatttttttg tttcttgtaa ctacaaagct ttgctacaaa tttatgcatt tcattcaaat 1380
   cagtgatcta tgtctgtgtg atccctaaac ataattgtgg actataaaaa tgtaacacca 1440
   taattacatt cctaactaga attagtatgt ctgcctttgt atctctatgc tgtactttaa 1500
   cactttgtat tcttaggtta ttttgctttg gttacaatgg ctcaagtaga aaagcggtcc 1560
   catccatatt aagacagtgt acaaaactgt aaataaaatg tgtacagtga attgtctttt 1620
   agacaactag atttgtcctt tatttctcca tctctagaag gaatctgtac ttcgtattgc 1680
   aaggcagtct cttgtgtctt cttagagtgt cttccccatg cacagcctca gtttggagca 1740
   ctagtttatt atgtttatta caatttttaa taaattgact aggtagtatc acatgtaatt 1800
   acactgatgt ggctatcttt ttaataaagt taaggcacag ttgctcagtc ctaggttgag 1860
   tgatggactt tgactatgtt acagttgatg aggattgggg ttttggtgca tcaccattcg 1920
   gtaggaacag cggctagaaa ctgattgttg ggtttaagat gtttttactt aatggccaga 1980
   aaattagcgt aaggaaagta tatagagaaa catgcgttag ggacattagt gttactatct 2040
   gaataaaaca caataaacaa gtattaagaa ctacttatat tggtcaattg ttgcagtatg 2100
   gttttctgta aacttgaaac cttgatctat tctttgtatc atttaaagca aacatgaaga 2160
   cattttgtct gcagtacgta attgtatagt tcagatcctg tgagatgagg tgtggctgtt 2220
   aacgccgaag ggtaagctga actgtgggta gcagagtgga aaccattggc tgagagaaaa 2280
   atgctcttta agtggtggtt gttatgaatt cacactgata acttgataaa gatccttata 2340
   aaatacatac ggaattaata gcattgctct tattatgtac gtcaagaatg tataaccgcc 2400
   tgctcttgtt gtcacagata actccctgtt cagtgctttg gaaatagcga tgctcacgat 2460
   ctcagcattc tgtaccctac atctactgtg tggatcattg agagatcttt tgacattgca 2520
   acatgatatg gtctatgttg ggctgcattc ctggctgtct tgtatgagac cccggttggc 2580
   tccctgaagc tgattgatac agtgtacagg catgaaggtg gctgatgagg ctttcttacc 2640
   aacatgtggg attctagtag ttgtatctat tagagattaa ttctcatatt ccttttcatt 2700
   catttgtaag aagtatcaac tttagaagtg aaaaaagaat cataaaatac agtttttaaa 2760
   gtt 2763
<210> 17
   <211> 19
   <212> DNA
   <213> Artificial Sequence
<220>
   <223> Primer
<400> 17
   aggacggtgg ctgatcaca 19
<210> 18
   <211> 21
   <212> DNA
   <213> Artificial Sequence
<220>
   <223> Primer
<400> 18
   tctctagcca gaagcgatcg a 21
<210> 19
   <211> 25
   <212> DNA
   <213> Artificial Sequence
<220>
   <223> Probe
<400> 19
   tgaacaagca gcagagacgg agtga 25
<210> 20
   <211> 19
   <212> RNA
   <213> Artificial Sequence
<220>
   <223> Synthetic Oligonucleotide
<400> 20
   cgagaggcgg acgggaccg 19
<210> 21
   <211> 21
   <212> DNA
   <213> Artificial Sequence
<220>
   <223> Synthetic Oligonucleotide
<220>
   <221> misc_feature
   <222> (1)...(19)
   <223> bases at these positions are RNA
<400> 21
   cgagaggcgg acgggaccgt t 21
<210> 22
   <211> 21
   <212> DNA
   <213> Artificial Sequence
<220>
   <223> Synthetic Oligonucleotide
<400> 22
   ttgctctccg cctgccctgg c 21
<210> 23
   <211> 19
   <212> DNA
   <213> Artificial Sequence
<220>
   <223> Synthetic Oligonucleotide
<400> 23
   gctctccgcc tgccctggc 19
<210> 24
   <211> 19
   <212> RNA
   <213> Artificial Sequence
<220>
   <223> Synthetic Oligonucleotide
<400> 24
   uuugaaaaug uugaucucc 19
<210> 25
   <211> 19
   <212> RNA
   <213> Artificial Sequence
<220>
   <223> Synthetic Oligonucleotide
<400> 25
   ggagaucaac auuuucaaa 19
<210> 26
   <211> 19
   <212> RNA
   <213> Artificial Sequence
<220>
   <223> Synthetic Oligonucleotide
<400> 26
   uugucucugg uccuuacuu 19
<210> 27
   <211> 19
   <212> RNA
   <213> Artificial Sequence
<220>
   <223> Synthetic Oligonucleotide
<400> 27
   aaguaaggac cagagacaa 19
<210> 28
<220>
   <223> Synthetic Oligonucleotide
<400> 28
   000
<210> 29
<220>
   <223> Synthetic Oligonucleotide
<400> 29
   000
<210> 30
   <211> 19
   <212> RNA
   <213> Artificial Sequence
<220>
   <223> Synthetic Oligonucleotide
<400> 30
   uuuagcucua acauuaaca 19
<210> 31
   <211> 19
   <212> RNA
   <213> Artificial Sequence
<220>
   <223> Synthetic Oligonucleotide
<400> 31
   uguuaauguu agagcuaaa 19
<210> 32
   <211> 19
   <212> RNA
   <213> Artificial Sequence
<220>
   <223> Synthetic Oligonucleotide
<400> 32
   uuacuagaca acuggauau 19
<210> 33
   <211> 19
   <212> RNA
   <213> Artificial Sequence
<220>
   <223> Synthetic Oligonucleotide
<400> 33
   auauccaguu gucuaguaa 19
<210> 34
   <211> 19
   <212> RNA
   <213> Artificial Sequence
<220>
   <223> Synthetic Oligonucleotide
<400> 34
   uuaaaaagug aguagucac 19
<210> 35
   <211> 19
   <212> RNA
   <213> Artificial Sequence
<220>
   <223> Synthetic Oligonucleotide
<400> 35
   gugacuacuc acuuuuuaa 19
<210> 36
   <211> 21
   <212> DNA
   <213> Artificial Sequence
<220>
   <223> Synthetic Oligonucleotide
<220>
   <221> misc_feature
   <222> (1) ... (19)
   <223> bases at these positions are RNA
<400> 36
   caaauccaga ggcuagcagt t 21
<210> 37
   <211> 21
   <212> DNA
   <213> Artificial Sequence
<220>
   <223> Synthetic Oligonucleotide
<220>
   <221> misc_feature
   <222> (1)...(19)
   <223> bases at these positions are RNA
<400> 37
   cugcuagccu cuggauuugt t 21
<210> 38
   <211> 21
   <212> RNA
   <213> Artificial Sequence
<220>
   <223> Synthetic Oligonucleotide
<400> 38
   cugcuagccu cuggauuugu u 21
<210> 39
   <211> 20
   <212> RNA
   <213> Artificial Sequence
<220>
   <223> Synthetic Oligonucleotide
<400> 39
   aaguaaggac cagagacaaa 20
<210> 40
   <211> 20
   <212> RNA
   <213> Artificial Sequence
<220>
   <223> Synthetic Oligonucleotide
<400> 40
   uuugucucug guccuuacuu 20
<210> 41
   <211> 21
   <212> RNA
   <213> Artificial Sequence
<220>
   <223> Synthetic Oligonucleotide
<400> 41
   caaauccaga ggcuagcagu u 21
<210> 42
   <211> 20
   <212> RNA
   <213> Artificial Sequence
<220>
   <223> Synthetic Oligonucleotide
<400> 42
   cugcuagccu cuggauuuga 20
<210> 43
   <211> 17
   <212> RNA
   <213> Artificial Sequence
<220>
   <223> Synthetic Oligonucleotide
<400> 43
   gucucugguc cuuacuu 17
<210> 44
   <211> 17
   <212> RNA
   <213> Artificial Sequence
<220>
   <223> Synthetic Oligonucleotide
<400> 44
   uuuugucucu gguccuu 17
<210> 45
   <211> 17
   <212> RNA
   <213> Artificial Sequence
<220>
   <223> Synthetic Oligonucleotide
<400> 45
   cugguccuua cuucccc 17
<210> 46
<220>
   <223> Synthetic Oligonucleotide
<400> 46
   000
<210> 47
   <211> 20
   <212> RNA
   <213> Artificial Sequence
<220>
   <223> Synthetic Oligonucleotide
<400> 47
   ucucuggucc uuacuucccc 20
<210> 48
   <211> 21
   <212> DNA
   <213> Artificial Sequence
<220>
   <223> Synthetic Oligonucleotide
<220>
   <221> misc_feature
   <222> (1) ... (19)
   <223> bases at these positions are RNA
<400> 48
   cugcuagccu cuggauuugu t 21
<210> 49
   <211> 20
   <212> RNA
   <213> Artificial Sequence
<220>
   <223> Synthetic Oligonucleotide
<400> 49
   uucauuccug gucucuguuu 20
<210> 50
   <211> 20
   <212> RNA
   <213> Artificial Sequence
<220>
   <223> Synthetic Oligonucleotide
<400> 50
   ugucauauuc cuggauccuu 20
<210> 51
   <211> 20
   <212> RNA
   <213> Artificial Sequence
<220>
   <223> Synthetic Oligonucleotide
<400> 51
   aaggauccag gaauaugaca 20
<210> 52
   <211> 20
   <212> RNA
   <213> Artificial Sequence
<220>
   <223> Synthetic Oligonucleotide
<400> 52
   uccuggaucc uucaccaaug 20
<210> 53
   <211> 20
   <212> RNA
   <213> Artificial Sequence
<220>
   <223> Synthetic Oligonucleotide
<400> 53
   cauuggugaa ggauccagga 20
<210> 54
   <211> 20
   <212> RNA
   <213> Artificial Sequence
<220>
   <223> Synthetic Oligonucleotide
<400> 54
   ucuuaucacc uuuagcucua 20
<210> 55
   <211> 20
   <212> RNA
   <213> Artificial Sequence
<220>
   <223> Synthetic Oligonucleotide
<400> 55
   uagagcuaaa ggugauaaga 20
<210> 56
   <211> 20
   <212> RNA
   <213> Artificial Sequence
<220>
   <223> Synthetic Oligonucleotide
<400> 56
   auacucagaa ggugucuucu 20
<210> 57
   <211> 20
   <212> RNA
   <213> Artificial Sequence
<220>
   <223> Synthetic Oligonucleotide
<400> 57
   agaagacacc uucugaguau 20
<210> 58
   <211> 19
   <212> RNA
   <213> Artificial Sequence
<220>
   <223> Synthetic Oligonucleotide
<400> 58
   ucuuaucacc uuuagcucu 19
<210> 59
   <211> 19
   <212> RNA
   <213> Artificial Sequence
<220>
   <223> Synthetic Oligonucleotide
<400> 59
   agagcuaaag gugauaaga 19
<210> 60
   <211> 19
   <212> RNA
   <213> Artificial Sequence
<220>
   <223> Synthetic Oligonucleotide
<400> 60
   ccuggauccu ucaccaaug 19
<210> 61
   <211> 20
   <212> DNA
   <213> Artificial Sequence
<220>
   <223> Synthetic Oligonucleotide
<220>
   <221> misc_feature
   <222> (1)...(20)
   <223> bases at these positions are RNA
<400> 61
   uutgtcucug guccuuacuu 20
<210> 62
   <211> 20
   <212> DNA
   <213> Artificial Sequence
<220>
   <223> Synthetic Oligonucleotide
<220>
   <221> misc_feature
   <222> (1) ... (20)
   <223> bases at these positions are RNA
<400> 62
   tttgtcucug guccuuacuu 20
<210> 63
   <211> 20
   <212> DNA
   <213> Artificial Sequence
<220>
   <223> Synthetic Oligonucleotide
<400> 63
   ctgctagcct ctggatttga 20
<210> 64
   <211> 21
   <212> RNA
   <213> Artificial Sequence
<220>
   <223> Synthetic Oligonucleotide
<400> 64
   aaguaaggac cagagacaau u 21
<210> 65
   <211> 21
   <212> RNA
   <213> Artificial Sequence
<220>
   <223> Synthetic Oligonucleotide
<400> 65
   uugucucugg uccuuacuuu u 21
<210> 66
   <211> 21
   <212> RNA
   <213> Artificial Sequence
<220>
   <223> Synthetic Oligonucleotide
<400> 66
   gugacuacuc acuuuuuaau u 21
<210> 67
   <211> 21
   <212> RNA
   <213> Artificial Sequence
<220>
   <223> Synthetic Oligonucleotide
<400> 67
   uuaaaaagug aguagucacu u 21
<210> 68
   <211> 21
   <212> RNA
   <213> Artificial Sequence
<220>
   <223> Synthetic Oligonucleotide
<400> 68
   uuugaaaaug uugaucuccu u 21

## Description

### Field of the Invention

The present invention relates to compositions comprising oligomeric compounds that modulate gene expression, as defined in the appended claims. Such modulation is via the RNA interference pathway. The modified oligomeric compounds of the invention comprise motifs that can enhance various physical properties and attributes compared to wild type nucleic acids. More particularly, the modification of the sense strand while maintaing a particular motif in the antisense strand enables enhancing the efficiency of the two strands for their particular roles in a selected pathway such as the RNAi pathway. The compositions of the present invention further include a fully modified antisense strand that has 2'-OCH₃ modified nucleosides that define a positional motif with 2'-F modified nucleosides in the remaining positions for essentially the entire length of the hybridizing region. The sense strand is a continuous sequence of nucleosides that define a motif. The compositions can include one or more 3'-capping groups, 5'-capping groups, 5'-phosphate moieties, 3'-linked conjugate groups, non hybridizing 3'-overhang regions and a non hybridizing 3'-overhang regions that are linked to a conjugate group. The compositions can further include varying numbers ofphosphorothioate internucleoside linkages that enhance the in vivo activity. The compositions are useful in one aspect for targeting selected nucleic acid molecules and modulating the expression of one or more genes. In some embodiments, the compositions of the present invention hybridize to a portion of a target RNA resulting in loss of normal function of the target RNA.

### Background of the Invention

In many species, introduction of double-stranded RNA (dsRNA) induces potent and specific gene silencing. This phenomenon occurs in both plants and animals and has roles in viral defense and transposon silencing mechanisms. This phenomenon was originally described more than a decade ago by researchers working with the petunia flower. While trying to deepen the purple color of these flowers, Jorgensen et al. introduced a pigment-producing gene under the control of a powerful.promoter. Instead of the expected deep purple color, many of the flowers appeared variegated or even white. Jorgensen named the observed phenomenon "cosuppression", since the expression of both the introduced gene and the homologous endogenous gene was suppressed (Napoli et al., Plant Cell, 1990, 2, 279-289; Jorgensen et al., Plant Mol. Biol., 1996, 31,957-973).

Cosuppression has since been found to occur in many species of plants, fungi, and has been particularly well characterized in *Neurospora crassa,* where it is known as "quelling" (Cogoni et al., Genes Dev., 2000, 10, 638-643; Guru, Nature, 2000, 404, 804-808).

The first evidence that dsRNA could lead to gene silencing in animals came from work in the nematode, *C. elegans.* In 1995, researchers Guo and Kemphues were attempting to use antisense RNA to shut down expression of the par-1 gene in order to assess its function. As expected, injection of the antisense RNA disrupted expression of par-1, but quizzically, injection of the sense-strand control also disrupted expression (Guo et al., Cell, 1995, 81, 611-620). This result was a puzzle until Fire et al. injected dsRNA (a mixture of both sense and antisense strands) into *C. elegans.* This injection resulted in much more efficient silencing than injection of either the sense or the antisense strands alone. Injection of just a few molecules of dsRNA per cell was sufficient to completely silence the homologous gene's expression. Furthermore, injection of dsRNA into the gut of the worm caused gene silencing not only throughout the worm, but also in first generation offspring (Fire et al., Nature, 1998, 391, 806-811).

The potency of this phenomenon led Timmons and Fire to explore the limits of the dsRNA effects by feeding nematodes bacteria that had been engineered to express dsRNA homologous to the *C. elegans* unc-22 gene. Surprisingly, these worms developed an unc-22 null-like phenotype (Timmons et al., Nature, 1998, 395, 854; Timmons et al., Gene, 2001, 263, 103-112). Further work showed that soaking worms in dsRNA was also able to induce silencing (Tabara et al., Science, 1998, 282, 430-431). PCT publication WO 01/48183 discloses methods of inhibiting expression of a target gene in a nematode worm involving feeding to the worm a food organism which is capable of producing a double-stranded RNA structure having a nucleotide sequence substantially identical to a portion of the target gene following ingestion of the food organism by the nematode, or by introducing a DNA capable of producing the double-stranded RNA structure.

The posttranscriptional gene silencing defined in *C. elegans* resulting from exposure to double-stranded RNA (dsRNA) has since been designated as RNA interference (RNAi). This term has come to generalize all forms of gene silencing involving dsRNA leading to the sequence-specific reduction of endogenous targeted mRNA levels; unlike co-suppression, in which transgenic DNA leads to silencing of both the transgene and the endogenous gene.

Introduction of exogenous double-stranded RNA (dsRNA) into *C. elegans* has been shown to specifically and potently disrupt the activity of genes containing homologous sequences. Montgomery et al. suggests that the primary interference effects of dsRNA are post-transcriptional; this conclusion being derived from examination of the primary DNA sequence after dsRNA-mediated interference a finding of no evidence of alterations followed by studies involving alteration of an upstream operon having no effect on the activity of its downstream gene. These results argue against an effect on initiation or elongation of transcription. Finally they observed by *in situ* hybridization, that dsRNA-mediated interference produced a substantial, although not complete, reduction in accumulation of nascent transcripts in the nucleus, while cytoplasmic accumulation of transcripts was virtually eliminated. These results indicate that the endogenous mRNA is the primary target for interference and suggest a mechanism that degrades the targeted mRNA before translation can occur. It was also found that this mechanism is not dependent on the SMG system, an mRNA surveillance system in *C. elegans* responsible for targeting and destroying aberrant messages. The authors further suggest a model of how dsRNA might function as a catalytic mechanism to target homologous mRNAs for degradation. (Montgomery et al., Proc. Natl. Acad. Sci. USA, 1998, 95, 15502-15507).

The development of a cell-free system from syncytial blastoderm Drosophila embryos that recapitulates many of the features of RNAi has been reported. The interference observed in this reaction is sequence specific, is promoted by dsRNA but not single-stranded RNA, functions by specific mRNA degradation, and requires a minimum length of dsRNA. Furthermore, preincubation of dsRNA potentiates its activity demonstrating that RNAi can be mediated by sequence-specific processes in soluble reactions (Tuschl et al., Genes Dev., 1999, 13, 3191-3197).

In subsequent experiments, Tuschl et al, using the Drosophila in vitro system, demonstrated that 21- and 22-nt RNA fragments are the sequence-specific mediators of RNAi. These fragments, which they termed short interfering RNAs (siRNAs) were shown to be generated by an RNase III-like processing reaction from long dsRNA. They also showed that chemically synthesized siRNA duplexes with overhanging 3' ends mediate efficient target RNA cleavage in the Drosophila lysate, and that the cleavage site is located near the center of the region spanned by the guiding siRNA. In addition, they suggest that the direction of dsRNA processing determines whether sense or antisense target RNA can be cleaved by the siRNA-protein complex (Elbashir et al., Genes Dev., 2001, 15, 188-200). Further characterization of the suppression of expression of endogenous and heterologous genes caused by the 21-23 nucleotide siRNAs have been investigated in several mammalian cell lines, including human embryonic kidney (293) and HeLa cells (Elbashir et al., Nature, 2001, 411, 494-498).

Tijsterman et al. have shown that, in fact, single-stranded RNA oligomers of antisense polarity can be potent inducers of gene silencing. As is the case for co-suppression, they showed that antisense RNAs act independently of the RNAi genes rde-1 and rde-4 but require the mutator/RNAi gene mut-7 and a putative DEAD box RNA helicase, mut-14. According to the authors, their data favor the hypothesis that gene silencing is accomplished by RNA primer extension using the mRNA as template, leading to dsRNA that is subsequently degraded suggesting that single-stranded RNA oligomers are ultimately responsible for the RNAi phenomenon (Tijsterman et al., Science, 2002, 295, 694-697).

Several other publications have described the structural requirements for the dsRNA trigger required for RNAi activity. Recent reports have indicated that ideal dsRNA sequences are 21nt in length containing 2 nt 3'-end overhangs (Elbashir et al, EMBO (2001), 20, 6817-6887, Sabine Brantl, Biochimica et Biophysica Acta, 2002, 1575, 15-25.) In this system, substitution of the 4 nucleosides from the 3'-end with 2'-deoxynucleosides has been demonstrated to not affect activity. On the other hand, substitution with 2'-deoxynucleosides or 2'-OMe-nucleosides throughout the sequence (sense or antisense) was shown to be deleterious to RNAi activity.

Investigation of the structural requirements for RNA silencing in *C. elegans* has demonstrated modification of the internucleotide linkage (phosphorothioate) to not interfere with activity (Parrish et al., Molecular Cell, 2000, 6, 1077-1087.) It was also shown by Parrish et al., that chemical modification like 2'-amino or 5'-iodouridine are well tolerated in the sense strand but not the antisense strand of the dsRNA suggesting differing roles for the 2 strands in RNAi. Base modification such as guanine to inosine (where one hydrogen bond is lost) has been demonstrated to decrease RNAi activity independently of the position of the modification (sense or antisense). Same "position independent" loss of activity has been observed following the introduction of mismatches in the dsRNA trigger. Some types of modifications, for example introduction of sterically demanding bases such as 5-iodoU, have been shown to be deleterious to RNAi activity when positioned in the antisense strand, whereas modifications positioned in the sense strand were shown to be less detrimental to RNAi activity. As was the case for the 21 nt dsRNA sequences, RNA-DNA heteroduplexes did not serve as triggers for RNAi. However, dsRNA containing 2'-F-2'-deoxynucleosides appeared to be efficient in triggering RNAi response independent of the position (sense or antisense) of the 2'-F-2'-deoxynucleosides.

In one experiment the reduction of gene expression was studied using electroporated dsRNA and a 25mer morpholino in post implantation mouse embryos (Mellitzer et al., Mehanisms of Development, 2002, 118, 57-63). The morpholino oligomer did show activity but was not as effective as the dsRNA.

A number of PCT applications have been published that relate to the RNAi phenomenon. These include: PCT publication WO 00/44895; PCT publication WO 00/49035; PCT publication WO 00/63364; PCT publication WO 01/36641; PCT publication WO 01/36646; PCT publication WO 99/32619; PCT publication WO 00/44914; PCT publication WO 01/29058; and PCT publication WO 01/75164.

U.S. patents 5,898,031 and 6,107,094 describe certain oligonucleotide having RNA like properties. When hybridized with RNA, these olibonucleotides serve as substrates for a dsRNase enzyme with resultant cleavage of the RNA by the enzyme.

In another published paper (Martinez et al., Cell, 2002, 110, 563-574) it was shown that double stranded as well as single stranded siRNA resides in the RNA-induced silencing complex (RISC) together with elF2C1 and elf2C2 (human GERp950 Argonaute proteins. The activity of 5'-phosphorylated single stranded siRNA was comparable to the double stranded siRNA in the system studied. In a related study, the inclusion of a 5'-phosphate moiety was shown to enhance activity of siRNA's in vivo in Drosophila embryos (Boutla, et al., Curr. Biol., 2001, 11, 1776-1780). In another study, it was reported that the 5'-phosphate was required for siRNA function in human HeLa cells (Schwarz et al., Molecular Cell, 2002, 10, 537-548).

A wide variety of chemical modifications have been made to siRNA compositions to try to enhance properties including stability and potency relative to the unmodified compositions. Much of the early work looked at modification of one strand while keeping the other strand unmodified. More recent work has focused on modification of both strands.

One group is working on modifying both strands of siRNA duplexes such that each strand has an alternating pattern wherein each nucleoside or a block of modified nucleosides is alternating with unmodified β-D-ribonucleosides. The chemical modification used in the modified portion is 2'-OCH₃ modified nucleosides (see European publication EP 1389637 A1, published on February 18, 2004 and PCT publication WO2004015107 published on February 19, 2004).

Another group has prepared a number of siRNA constructs with modifications in both strands (see PCT publication WO03/070918 published on August 28, 2003). The constructs disclosed generally have modified nucleosides dispersed in a pattern that is dictated by which strand is being modified and further by the positioning of the purines and pyrimidines in that strand. In general the purines are 2'-OCH₃ or 2'-H and pyrimidines are 2'-F in the antisense strand and the purines are 2'-H and the pyrimidines are 2'-OCH₃ or 2'-F in the sense strand. According to the definitions used in the present application these constructs would appear to be positionally modified as there is no set motif to the substitution pattern and positionally modified can describe a random substitution pattern.

Certain nucleoside compounds having bicyclic sugar moieties are known as locked nucleic acids or LNA (Koshkin et al., Tetrahedron 1998, 54, 3607-3630). These compounds are also referred toxin the literature as bicyclic nucleotide analogs (Imanishi et al., International Patent Application WO 98/39352), but this term is also applicable to a genus of compounds that includes other analogs in addition to LNAs. Such modified nucleosides mimic the 3'-endo sugar conformation of native ribonucleosides with the advantage of having enhanced binding affinity and increased resistance to nucleases.

One group recently reported that the incorporation of bicyclic nucleosides, each having a 4'-CH₂-O-2' bridge (LNA) into siRNA duplexes dramatically improved the half life in serum via enhanced nuclease resistance and also increased the duplex stability due to the increased affinity. This effect is seen with a minimum number of LNA's located as specific positions within the siRNA duplex. The placement of LNA's at the 5'-end of the sense strand was shown to reduce the loading of this strand which reduces off target effects (see Elmen et al., Nucleic Acids Res., 2005, 33(1), 439-447).

Some LNAs have a 2'-hydroxyl group linked to the 4' carbon atom of the sugar ring thereby forming a bicyclic sugar moiety. The linkage may be a methylene (-CH₂-)ₙ group bridging the 2' oxygen atom and the 4' carbon atom wherein n is 1 or 2 (Singh et al., Chem. Commun., 1998, 4, 455-456; Kaneko et al., U.S. Patent Application Publication No.: US 2002/0147332, also see Japanese Patent Application HEI-11-33863, February 12, 1999).

U. S. Patent Application Publication No. 2002/0068708 discloses a number of nucleosides having a variety of bicyclic sugar moieties with the various bridges creating the bicyclic sugar having a variety of configurations and chemical composition.

Braash et al., Biochemistry 2003, 42, 7967-7975 report improved thermal stability of LNA modified siRNA without compromising the efficiency of the siRNA. Grunweller, et. al., Nucleic Acid Research, 2003, 31, 3185-3193 discloses the potency of certain LNA gapmers and siRNAs.

One group has identified a 9 base sequence within an siRNA duplex that elicits a sequence-specific TLR7-dependent immune response in plasmacytoid dendritic cells. The immunostimulation was reduced by incorporating 4 bicyclic nucleosides, each having a 4'-CH₂-O-2' bridge (LNA) at the 3'-end of the sense strand. They also made 5' and both 3' and 5' versions of sense and antisense for incorporation into siRNA duplexes where one strand had the modified nucleosides and the other strand was unmodified (see Hornung et al., 2005, 11(3)1, 263-270).

One group of researchers used expression profiling to perform a genome wide analysis of the efficacy and specificity of siRNA induced silencing of two genes involved in signal transduction (insulin-like growth factor receptor (IGF1R) and mitogen-activated protein kinase 1 (MAPK14 or p38α). A unique expression profile was produced for each of the 8 siRNAs targeted to MAPK14 and 16 siRNA's targeted to IGF1R indicating that off target effects were highly dependent on the particular sequence. These expression patterns were reproducable for each individual siRNA. The group determined that off target effects were caused by both the antisense strand and the sense strand of siRNA duplexes. There is a need for siRNA's that are designed to preferentially load only the antisense strand thereby reducing the off target effects caused by the sense strand also being loaded into the RISC.

A number of published applications that are commonly assigned with the present application disclose double strand compositions wherein one or both of the strands comprise a particular motif. The motifs include hemimer motifs, blockmer motifs, gapped motifs, fully modified motifs, positionally modified motifs and alternating motifs (see published PCT applications: WO 2004/044133 published May 27, 2004, 3'-endo motifs; WO 2004/113496 published December 29, 2004, 3'-endo motifs; WO 2004/044136 published May 27, 2004, alternating motifs; WO 2004/044140 published May 27, 2004, 2'-modified motifs; WO 2004/043977 published May 27, 2004, 2'-F motifs; WO 2004/043978 published May 27, 2004, 2'-OCH₃ motifs; WO 2004/041889 published May 21, 2004, polycyclic sugar motifs; WO 2004/043979 published May 27, 2004, sugar surrogate motifs; and WO 2004/044138 published May 27, 2004, chimeric motifs; also see published US Application US20050080246 published April 14, 2005 and WO 2005/121372).

Like the RNAse H pathway, the RNA interference pathway of antisense modulation of gene expression is an effective means for modulating the levels of specific gene products and may therefore prove to be uniquely useful in a number of therapeutic, diagnostic, and research applications involving gene silencing. The present invention therefore further relates to compositions useful for modulating gene expression pathways, including those relying on an antisense mechanism of action such as RNA interference and dsRNA enzymes as well as non-antisense mechanisms. One having skill in the art, once armed with this disclosure will be able, without undue experimentation, to identify additional compositions for these uses.

### Summary of the Invention

The present invention provides compositions having first and second oligomeric compounds wherein the first oligomeric compound comprises a positional/full motif that either symmetric or asymmetric and the second oligomeric compound comprises a continuous sequence of nucleosides that define a motif selected from an alternating motif, as defined in the appended claims. The motifs derive from the positioning of sugar modified nucleosides relative to other modified or unmodified nucleosides in a strand and are independent of the sequence, type of nucleobases (purine, pyrimidine or other) or type of internucleoside linkage. Each of the first and second oligomeric compounds can be further modified with one or more groups selected from a 3'-capping group, a 5'-capping group, a 5'-phosphate moiety, a linked conjugate group, a non hybridizing 3'-overhang region and a non hybridizing 3'-overhang region that is linked to a conjugate group.

The first oligomeric compound, the antisense strand, comprises a hybridizing region that is fully complementary to the second oligomeric compound and also to a nucleic acid target. The hybridizing region of the first olgomeric compound comprises a continuous sequence of linked nucleosides that define a positional/full motif having the formula:

5'-(Ni-L)₂-(Nₘ-L)₃-(Nₙ-L)₂-(Nₘ-L)₂-(Nₙ-L)₂-(Nₘ-L)₅-(Nₙ-L)₃-3' wherein, each Nᵢ is, independently, a 4'-thio modified nucleoside or a 2'-F modified nucleoside; each Nₘ is a 2'-F modified nucleoside; each Nₙ is a 2'-OCH₃ modified nucleoside and each L is an internucleoside linkage. The second oligomeric compound, the sense strand, comprises a continuous sequence of nucleosides that define an alternating motif comprising 2'-O(CH₂)₂OCH₃. Each of the first and second oligomeric compounds can be further modified with one or more groups selected from a 3'-capping group, a 5'-capping group, a 5'-phosphate moiety, a linked conjugate group, a non hybridizing 3'-overhang region and a non hybridizing 3'-overhang region that is linked to a conjugate group.

The present invention also provides an in vitro method of inhibiting gene expression comprising contacting one or more cell or a tissue with a composition of the present invention.

The present invention provides compositions comprising first and second oligomeric compounds wherein each oligomeric compound independently has a hybridizing region of 19 nucleosides wherein each of the nucleosides of the second oligomeric compound are complementary to and hybridize to the first oligomeric compound. The second oliogmeric compound is fully complementary to the first oliogmeric compound. In certain embodiements, modified nucleosides (e.g. abasic, acyclic et al.,) or non nucleosides are also contemplated at one or more positions within one or both hybridizing regions.

The first oligomeric compound optionally further includes a 5'-phosphate moiety and a non hybridizing 3'-overhang region comprising from 1 to about 2 2'-modified nucleosides. In a preferred embodiment at least one of these non hybridizing 2'-modified nucleosides has increased nuclease resistance relative to a β-D-2'-deoxyribonucleoside being located at the same position. In a more preferred embodiment all of the 2'-modified nucleosides located in the non hybridizing 3'-overhang regions have increased nuclease resistance relative to a β-D-2'-deoxyribonucleoside being located at the same positions. Such 2'-modified nucleosides can be further modified such as for example 4'-S and/or 5'-modified. 2'-Modified nucleosides are meant to include 2'-substituted nucleosides as well as bicyclic nucleosides (wherein one point of attachment for the second ring is the 2'-position such as for example 4'-(CH₂)ₙ-O-2' where n is 1 or 2, In one embodiment the non hybridizing 3'-overhang region comprises 2'-modified nucleosides. In another embodiment the second oligomeric compound has a 5'-hydroxyl or 5'-protected hydroxyl group. In a preferred embodiment the first oligomeric compound includes a 5'-posphate group and a 3'-overhang region and the second oligomeric compound includes a 3'-overhang region and or a 3'-linked conjugate group.

The second oligomeric compound opitonally includes a 3'-overhang region comprising from 1 to about 2 2'-modified nucleosides, a linked conjugate group or both wherein the conjugate group is linked to the 3'-overhang region. In a preferred embodiment at least one of these non hybridizing 2'-modified nucleosides, when present, has increased nuclease resistance relative to a β-D-2'-deoxyribo-nucleoside being located at the same position. In a more preferred embodiment all of the non hybridizing 2'-modified nucleosides have increased nuclease resistance relative to a β-D-2'-deoxyribo-nucleoside being located at the same positions. Such 2'-modified nucleosides can be further modified such as for example 4'-S and/or 5'-modified. 2'-Modified nucleosides are meant to include 2'-substituted nucleosides as well as bicyclic nucleosides (wherein one point of attachment for the second ring is the 2'-position such as for example 4'-(CH₂)ₙ-O-2' where n is 1 or 2. In one embodiment the non hybridizing 3'-overhang region comprises 2'-modified nucleosides. In another embodiment the second oligomeric compound does not comprise a non hybridizing 3'-overhang region.

The second oligomeric compound optionally comprises a linked conjugate group. A large number of conjugate groups are known to the art skilled that would be amenable to the present invention. Attachment of conjugate groups is also well known in the art and any and all such linkages are envisioned by the present invention. In one embodiment the second oligomeric compound comprises a non hybridizing region, a phosphorothioate group, a linker and a conjugate group. In another embodiment the optional non hybridizing region is omitted. One representative formula for a linked conjugate is shown below for illustration and is not meant to be limiting:

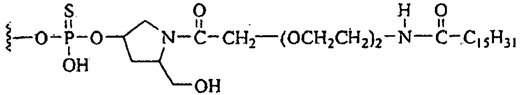

The formula includes a C₁₆ lipophilic conjugate attached via a pyrrolidinyl linker to a phosphororthioate group. The phosphorothioate group can be attached directly to the 3'-end of the second oligomeric compound organ be attached to the 3'-terminal non hybridizing nucleoside.

The second oligomeric compound (sense strand) comprises a continuous sequence of nucleosides that define an alternating motif as defined in the appended claims. The sense strand is useful in, for example, influencing the preferential loading of the opposite strand into the RISC (or cleavage) complex. In particular, the present invention provides oligomeric compounds that comprise chemical modifications in the sense strand to drive loading of the antisense strand (first oligomeric compound) into the RISC (or cleavage) complex. Such modifications can be used to increase potency of duplex constructs that have been modified to enhance stability. Examples of chemical 2'-sugar modifications that are especially efficient in motifs that enhance the loading of the antisense strand relative to the sense strand include, but are not limited to, F, MOE (2'-O(CH₂)₂OCH₃), 2'-O-methyl, -ethyl, -propyl, -O-(CH₂)₂-O-N(CH₃)₂, -O-CH₂-C(=O)-N(H)(CH₂)₂N(CH₃)₂, -O-(CH₂)₂-O-(CH₂)₂-N(CH₃)₂, -N-methylacetamide and 4'-(CH₂)₂-O-2' (n is 1 or 2). Such modifications can be used alone or in combination with sugar modifed nucleosides including but not limited to 2'-H, 2'-OH, 2'-F and others. Such modifications can be distributed throughout the strand to define an alternating, positional, positional/full, full or blockmer motif or placed at one or both of the 5' and/or 3' ends to make a gapmer or hemimer motif on the sense strand. The compositions are useful for targeting selected nucleic acid molecules and modulating the expression of one or more genes. In use, the compositions of the present invention hybridize to a portion of a target RNA resulting in loss of normal function of the target RNA.

### Detailed Description

Various compositions comprising the motif combinations of the present invention have been shown to have enhanced properties. The properties that can be enhanced include, but are not limited, to modulation of pharmacokinetic properties through modification of protein binding, protein off-rate, absorption and clearance; modulation of nuclease stability as well as chemical stability; modulation of the binding affinity and specificity of the oligomer (affinity and specificity for enzymes as well as for complementary sequences); and increasing efficacy of RNA cleavage.

The compositions ofthe present invention are useful for, for example, modulating gene expression. For example, a targeted cell, group of cells, a tissue or an animal is contacted with a composition ofthe invention to effect reduction of mRNA that can directly inhibit gene expression. In another embodiment, the reduction of mRNA can indirectly upregulate a non-targeted gene through a pathway that relates the targeted gene to a non-targeted gene. Numerous methods and models for the regulation of genes using compositions of the invention are illustrated in the art and in the example section below.

The compositions of the invention modulate gene expression by hybridizing to a nucleic acid target resulting in loss of its normal function. As used herein, the term "target nucleic acid" or "nucleic acid target" is used for convenience to encompass any nucleic acid capable of being targeted including without limitation DNA, RNA (including pre-mRNA and mRNA or portions thereof) transcribed from such DNA, and also cDNA derived from such RNA. In some embodiments, the target nucleic acid is a messenger RNA. In another embodiment, the degradation ofthe targeted messenger RNA is facilitated by an activated RISC complex that is formed with compositions of the invention. In another embodiment, the degradation of the targeted messenger RNA is facilitated by a nuclease such as RNaseH.

As used in the present invention the term "gapped motif" is meant to include a contiguous sequence of nucleosides that are divided into 3 regions, an internal region flanked by two external regions. The regions are differentiated from each other at least by having different sugar groups that comprise the nucleosides. The types of nucleosides that are used to differentiate the regions of a gapped oligomeric compound include β-D-ribonucleosides, 2'-modified nucleosides, 4'-thio modified nucleosides, 4'-thio-2'-modified nucleosides, and bicyclic sugar modified nucleosides. Each region is uniformly modified e.g. the sugar groups are identical. The internal region or the gap generally comprises β-D-ribonucleosides but can be a sequence of sugar modified nucleosides. The nucleosides located in the gap of a gapped oligomeric compound have different sugar groups than both of the wings.

Gapped oligomeric compounds are further defined as being either "symmetric" or "asymmetric". A gapmer having the same uniform sugar modification in each of the wings is termer a symmetric gapped oligomeric compound. A gapmer having different uniform modifications in each wing is termed an asymmetric gapped oligomeric compound. Gapped oligomeric compounds such as these can have for example both wings comprising 4'-thio modified nucleosides (symmetric gapmer) and a gap comprising β-D-ribonucleosides or modified nucleosides other than 4'-thio modified nucleosides. Asymmetric gapped oligomeric compounds for example can have one wing comprising 2'-OCH₃ modified nucleosides and the other wing comprising 4'-thio modified nucleosides with the internal region (gap) comprising β-D-ribonucleosides or sugar modified nucleosides that are other than 4'-thio or 2'-OCH₃ modified nucleosides.

Gapped oligomeric compounds as used in the present invention include wings that independently have from 1 to about 6 nucleosides. Suitable wings comprise from 1 to about 4 nucleosides and can comprise wings comprising from 1 to about 3 nucleosides. The number of nucleosides in each wing can be the same or different. The present invention therefore includes gapped oligomeric compounds wherein each wing independently comprises 1, 2, 3, 4, 5, or 6 sugar modified nucleosides.

As used in the present invention the term "alternating motif" is meant to include a contiguous sequence of nucleosides comprising two different nucleosides that alternate for essentially the entire sequence of the oligomeric compound. The pattern of alternation can be described by the formula: 5'-A(-L-B-L-A)ₙ(-L-B)ₙₙ-3' where A and B are nucleosides differentiated by having at least different sugar groups, each L is an internucleoside linking group, nn is 0 or 1 and n is from about 7 to about 11. This permits alternating oligomeric compounds from about 17 to about 24 nucleosides in length. This length range is not meant to be limiting as longer and shorter oligomeric compounds are also amenable to the present invention. This formula also allows for even and odd lengths for alternating oligomeric compounds wherein the 3' and 5'-terminal nucleosides are the same (odd) or different (even).

The "A" and "B" nucleosides comprising alternating oligomeric compounds of the present invention are differentiated from each other by having at least different sugar moieties. Each of the A and B nucleosides is selected from β-D-ribonucleosides, 2'-modified nucleosides, 4'-thio modified nucleosides, 4'-thio-2'-modified nucleosides, and bicyclic sugar modified nucleosides. The alternating motif includes the alternation of nucleosides having different sugar groups but is independent from the nucleobase sequence and the internucleoside linkages. The internucleoside linkage can vary at each or selected locations or can be uniform or alternating throughout the oligomeric compound.

Alternating oligomeric compounds of the present invention can be designed to function as the sense or the antisense strand. Alternating 2'-OCH₃/2'-F modified oligomeric compounds have been used as the antisense strand and have shown good activity with a variety of sense strands. One antisense oligomeric compound comprising an alternating motif is a 19mer wherein the A's are 2'-OCH₃ modified nucleosides and the B's are 2'-F modified nucleosides (nn is 0 and n is 9). The resulting alternating oligomeric compound will have a register wherein the 3'and 5'-ends are both 2'-OCH₃ modified nucleosides.

Alternating oligomeric compounds have been designed to function as the sense strand also. The chemistry or register is generally different than for the oligomeric compounds designed for the antisense strand. When a alternating 2'-F/2'-OCH₃ modified 19mer was paired with the antisense strand in the previous paragraph the preferred orientation was determined to be an offset register wherein both the 3' and 5'-ends of the sense strand were 2'-F modified nucleosides. In a matched register the sugar modifications match between hybridized nucleosides so all the terminal ends of a 19mer would have the same sugar modification. Another alternating motif that has been tested and works in the sense strand is β-D-ribonucleosides alternating with 2'-MOE modified nucleosides.

As used in the present invention the term "fully modified motif' or "full motif' is meant to include a contiguous sequence of sugar modified nucleosides wherein essentially each nucleoside is modified to have the same sugar modification. Suitable sugar modified nucleosides for fully modified strands of the invention include 2'-F, 4'-thio and 2'-OCH₃ with 2'-OCH₃ particularly suitable. In one aspect the 3' and 5'-terminal nucleosides are unmodified.

As used in the present disclosurethe term "hemimer motif" is meant to include a sequence of nucleosides that have uniform sugar moieties (identical sugars, modified or unmodified) and wherein one of the 5'-end or the 3'-end has a sequence of from 2 to 12 nucleosides that are sugar modified nucleosides that are different from the other nucleosides in the hemimer modified oligomeric compound. An example of a typical hemimer is an oligomeric compound comprising β-D-ribonucleosides that have a sequence of sugar modified nucleosides at one of the termini. In one embodiment the hemimer motif includes a sequence of β-D-ribonucleosides having from 2-12 sugar modified nucleosides located at one of the termini. In another embodiment the hemimer motif includes a sequence of β-D-ribonucleosides having from 2-6 sugar modified nucleosides located at one of the termini with from 2-4 sugar modified nucleosides being preferred.

As used in the present disclosure the term "blockmer motif' is meant to include a sequence of nucleosides that have uniform sugars (identical sugars, modified or unmodified) that is internally interrupted by a block of sugar modified nucleosides that are uniformly modified and wherein the modification is different from the other nucleosides. More generally, oligomeric compounds having a blockmer motif comprise a sequence of β-D-ribonucleosides having one internal block of from 2 to about 6, or from 2 to about 4 sugar modified nucleosides. The internal block region can be at any position within the oligomeric compound as long as it is not at one of the termini which would then make it a hemimer. The base sequence and internucleoside linkages can vary at any position within a blockmer motif.

As used in the present disclosure the term "positionally modified motif' is meant to include a sequence of β-D-ribonucleosides wherein the sequence is interrupted by two or more regions comprising from 1 to about 4 sugar modified nucleosides. The positionally modified motif includes internal regions of sugar modified nucleoside and can also include one or both termini. Each particular sugar modification within a region of sugar modified nucleosides is variable with uniform modification desired. The sugar modified regions can have the same sugar modification or can vary such that one region may have a different sugar modification than another region. Positionally modified strands comprise at least two sugar modified regions and at least three when both the 3' and 5'-termini comprise sugar modified regions. Positionally modified oligomeric compounds are distinguished from gapped motifs, hemimer motifs, blockmer motifs and alternating motifs because the pattern of regional substitution defined by any positional motif is not defined by these other motifs. Positionally modified motifs are not determined by the nucleobase sequence or the locations or types of internucleoside linkages. The term positionally modified oligomeric compound includes many different specific substitution patterns. A number of these substitution patterns have been prepared and tested in compositions.

As used in the present disclosure the term "positional/full motif' is meant to include a sequence of uniformly sugar modified nucleosides wherein the sequence is interrupted by two or more regions comprising from 1 to about 4 sugar modified nucleosides or 2'-deoxyribonucleosides. The positional/full motif is identical to the positional motif except that the β-D-ribonucleosides are replaced with sugar modified nucleosides.

The terms symmetric and asymmetric as applied to a positional or a positional/full motif refer to the two or more regions of sugar modified nucleosides. When each sugar modified nucleoside in the the two or more regions of sugar modified nucleosides have the same sugar modification the positional motif is a symmetric positional or symmetric positional/full motif. When one or more of the sugar modified nucleosides in the two or more regions of sugar modified nucleosides have different sugar modifications then the positional motif is an asymmetric positional or an asymmetric positional/full motif.

A list of substitution patterns corresponding to positionally modified oligomeric compounds illustrated in the examples are shown below. This list is meant to be instructive and not limiting.

| **ISIS No:** | **Length** | **Substitution pattern 5'-3'** | **Modified positions** |
|---|---|---|---|
| 345838 | 19mer | 5-1-5-1-2-1-2-2 | 6, 12, 15 and 18-19 |
| 352506 | 19mer | 5-2-2-2-5-3 | 7-8, 10-11, 17-19 |
| 352505 | 19mer | 4-1-2-1-2-1-2-1-2-3 | 5, 8, 11, 14, 17-19 |
| xxxxxx | 19mer | 4-1-6-1-4-3 | 5, 12, 17-19 |
| xxxxxx | 19mer | 4-2-4-2-5-2 | 5-6, 11-12, 18-19 |
| 345839 | 19mer | 4-2-2-2-6-3 | 5-6, 9-10, 17-19 |
| xxxxxx | 19mer | 3-1-4-1-4-1-3-1-1 | 4, 9, 14, 18 |
| 353539 | 19mer | 3-5-1-2-1-4-3 * | 1-3, 9, 12 |
| 355715 | 19mer | 3-1-4-1-8-1-1 | 4, 9, 18 |
| xxxxxx | 19mer | 3-1-5-1-7-1-1 | 4, 10, 18 |
| 384760 | 19mer | 2-7-2-5-3 * | 1-2, 10-11 and 17-19 |
| 371315 | 19mer | 3-6-2-5-3 | 1-3, 10-11, 17-19 |
| 353538 | 19mer | 2-1-5-1-2-1-4-3 | 3, 9, 12, 17-19 |
| xxxxxx | 19mer | 2-1-4-1-4-1-4-1-1 | 3, 8, 13, 18 |
| 336674 | 20mer | 15-1-1-3 | 16, 18-20 |
| 355712 | 20mer | 4-1-2-1-2-1-2-1-2-3 * | 5,8, 11, 14 |
| 347348 | 20mer | 3-2-1-2-1-2-1-2-1-2-3 | 1-3, 6, 9, 12, 15, 18-20 |
| 348467 | 20mer | 3-2-1-2-1-2-1-2-1-5 | 1-3, 6, 9, 12, 15 |
| 357278 | 20mer | 3-1-4-1-4-1-3-1-1 | 4,9, 14, 18 |
| xxxxxx | 20mer | 3-1-1-10-1-1-3 | 1-3, 5, 16, 18-20 |
| xxxxxx | 20mer | 3-1-6-1-7-1-1 | 4, 11, 19 |
| 357276 | 20mer | 3-1-3-1-7-1-4 | 4, 8, 16 |
| xxxxxx | 20mer | 3-1-5-2-5-1-3 | 4, 11, 17 |
| 357275 | 20mer | 3-1-5-1-8-1-1 | 4, 10, 19 |
| 373424 | 20mer | 3-6-2-5-3 | 1-3, 11-12, 18-20 |
| 357277 | 20mer | 2-1-5-1-5-1-4-2 | 3,9,15,20-21 |
| 345712 | 20mer | 2-2-5-2-5-2-2 | 3-4, 10-11, 17-18 |

| | | | |
|---|---|---|---|
| * indicates that more than one type of sugar modified nucleosides were used in the sugar modified regions. | | | |

Certain sense strands amenable to the present invention includes but is not limited to the following:

| **SEQ ID NO:/ISIS NO:** | **5'-3'** |
|---|---|
| 64/398239† | A_{f}AₘG_{f}UₘA_{f}AₘG_{f}GₘA_{f}CₘC_{f}AₘG_{f}AₘG_{f}AₘC_{f}AₘA_{f}UₑUₑ |
| 64/398240 | AAₑGUₑAAₑGGₑACₑCAₑGAₑGAₑCAₑAUₑUₑ |
| 64/398241 | _{d}AA_{ed}GU_{ed}AA_{ed}GG_{ed}AC_{ed}CA_{ed}GA_{ed}GA_{ed}CA_{ed}AUₑUₑ |
| 64/398242 | A_{f}AₑC_{f}UₑA_{f}AₑG_{f}GₑA_{f}CₑC_{f}AₑG_{f}AₑG_{f}AₑC_{f}AₑA_{f}UₑUₑ |
| 64/398243 | A_{f}AₑG_{f}UₑA_{f}AₑG_{f}GₑA_{f}CₑC_{f}AₑG_{f}AₑG_{f}AₑC_{f}AₑA_{f}UₑUₑ |
| 64/398244† | A_{f}AₚG_{f}UₚA_{f}AₚG_{f}GₚA_{f}CₚC_{f}AₚG_{f}AₚG_{f}AₚC_{f}AₚA_{f}UₑUₑ |
| 64/398245† | _{d}AA_{pd}GU_{pd}AA_{pd}GG_{pd}AC_{pd}CA_{pd}GA_{pd}GA_{pd}CA_{pd}AUₑUₑ |
| 64/398246† | A_{f}AₘG_{f}UₘA_{f}AₘG_{f}GₘA_{f}CₘC_{f}AₘG_{f}AₘG_{f}AₘC_{f}AₘA_{f}UₑUₑ |
| 64/398247 | A_{f}AₑG_{f}UₑA_{f}AₑG_{f}GₑA_{f}CₑC_{f}AₑG_{f}AₑG_{f}AₑG_{f}AₑA_{f}UₑUₑ |
| 64/398248 | _{d}AA_{ed}GU_{ed}AA_{ed}GG_{ed}AC_{ed}CA_{ed}GA_{ed}GA_{ed}CA_{ed}AUₑUₑ |
| 64/398249† | A_{f}AₘG_{f}UₘA_{f}AₘG_{f}GₘA_{f}CₘC_{f}AₘG_{f}AₘG_{f}AₘC_{f}AₘA_{f}UₑUₑ-L-C₁₆ |
| 64/398250 | _{d}AA_{ed}GU_{ed}AA_{ed}GG_{ed}AC_{ed}CA_{ed}GA_{ed}GA_{ed}CA_{ed}AUₑUₑ-L-C₁₆ |
| 64/398251 | AₑAₑGₑUAAGGACCAGAGACₑAₑAₑUₑUₑ |
| 64/398252 | AₑAₑG_{ed}U_{d}A_{d}A_{d}G_{d}G_{d}A_{d}C_{d}C_{d}A_{d}G_{d}A_{d}G_{d}ACₑAₑAₑUₑUₑ |
| 64/398253 | AₑAₑGₑU_{f}A_{f}A_{f}G_{f}G_{f}A_{f}C_{f}C_{f}A_{f}G_{f}A_{f}G_{f}AₑAₑAₑUₑUₑ |
| 64/398254 | AₑAₑGₑU_{f}A_{f}A_{f}G_{f}G_{f}A_{f}C_{f}C_{f}A_{f}G_{f}A_{f}G_{f}AₑA_{f}AₑUₑUₑ |
| 64/398255 | AₑAₑG_{ed}U_{d}A_{d}A_{d}G_{d}GAₑCₑC_{ed}A_{d}G_{d}A_{d}G_{d}ACₑAₑAₑUₑ. |

| | |
|---|---|
| † = disclosed for information only. | |

All non-annotated nucleosides are β-D-ribonucleosides linked by phosphodiester internucleoside linkages. Phosphorothioate internucleoside linkages are indicated by underlining. Modified nucleosides are indicated by a subscripted letter following the capital letter indicating the nucleoside. In particular, subscript "f" indicates 2'-fluoro; subscript "m" indicates 2'-O-methyl; subscript "1" indicates LNA; subscript "e" indicates 2'-O-methoxyethyl (MOE); subscript "p" indicates 2'-O-propyl; and subscript "t" indicates 4'-thio. For example Uₘ is a modified uridine having a 2'-OCH₃ group. A "d" preceding a nucleoside indicates a deoxynucleoside such as dT which is deoxythymidine. Some of the strands have a 5'-phosphate group designated as "P-". Bolded and italicized "C" indicates a 5-methyl C ribonucleoside. Where noted next to the ISIS number of a compound, "as" designates the antisense strand, and "s" designates the sense strand of the duplex, with respect to the target sequence.

The specific sequences here and throughout the speicification are provided only as examples. Thus, while certain motifs are exemplified using certain sequences, it is to be understood that other sequences could easily be prepared using the same motif of modifications. Accordingly, unless otherwise indicated, for the purposes of defining a motif, each nucleoside in the sequences herein may be substituted with "N" which indicates any base.

The term "sugar modified nucleosides" as used in the present invention is intended to include all manner of sugar modifications known in the art. The sugar modified nucleosides can have any heterocyclic base moiety and internucleoside linkage and may include further groups independent from the sugar modification. One group of preferred sugar modified nucleosides includes 2'-modified nucleosides, 4'-thio modified nucleosides, 4'-thio-2'-modified nucleoside, and bicyclic sugar modified nucleosides.

The term "2'-modified nucleoside" as used in the present invention is intended to include all manner of nucleosides having a 2'-substituent group that is other than H and OH. Suitable 2'-substituent groups for 2'-modified nucleosides of the invention include, but are not limited to: halo, allyl, amino, azido, amino, SH, CN, OCN, CF₃, OCF₃, O-, S-, or N(Rₘ)-alkyl; O-, S-, or N(Rₘ)-alkenyl; O-, S- or N(Rₘ)-alkynyl; O-alkylenyl-O-alkyl, alkynyl, alkaryl, aralkyl, O-alkaryl, O-aralkyl, O(CH₂)₂SCH₃, O-(CH₂)₂-O-N(Rₘ)(Rₙ) or O-CH₂-C(=O)-N(Rₘ)(Rₙ), where each Rₘ and Rₙ is, independently, H, an amino protecting group or substituted or unsubstituted C₁-C₁₀ alkyl. These 2'-substituent groups can be further substituted with substituent groups selected from hydroxyl, amino, alkoxy, carboxy, benzyl, phenyl, nitro (NO₂), thiol, thioalkoxy (S-alkyl), halogen, alkyl, aryl, alkenyl and alkynyl where each Rₘ is, independently, H, an amino protecting group or substituted or unsubstituted C₁-C₁₀ alkyl.

A list of 2'-substituent groups includes F, -NH₂, N₃, OCF₃, O-CH₃, O(CH₂)₃NH₂), CH₂-CH=CH₂, -O-CH₂-CH=CH₂, OCH₂CH₂OCH₃, 2'-O(CH₂)₂SCH₃, O-(CH₂)₂-O-N(Rₘ)(Rₙ), -O(CH₂)₂O(CH₂)₂N(CH₃)₂, and N-substituted acetamide (O-CH₂-C(=O)-N(Rₘ)(Rₙ) where each Rₘ and Rₙ is, independently, H, an amino protecting group or substituted or unsubstituted C₁-C₁₀ alkyl. Another list of 2'-substituent groups includes F, OCF₃, O-CH₃, OCH₂CH₂OCH₃, 2'-O(CH₂)₂SCH₃, O-(CH₂)₂-O-N(Rₘ)(Rₙ), -O(CH₂)₂O(CH₂)₂N(CH₃)₂, and N-substituted acetamides (O-CH₂-C(=O)-N(Rₘ)(Rₙ) where each Rₘ and Rₙ is, independently, H, an amino protecting group or substituted or unsubstituted C₁-C₁₀ alkyl.

Also amenable to the present invention is the manipulation of the stereochemistry of the basic furanose ring system which can be prepared in a number of different configurations. The attachment of the heterocyclic base to the 1'-position can result in the α-anomer (down) or the β-anomer (up). The β-anomer is the anomer found in native DNA and RNA but both forms can be used to prepare oligomeric compounds. A further manipulation can be achieved through the substitution the native form of the furanose with the enantiomeric form e.g. replacement of a native D-furanose with its mirror image enantiomer, the L-furanose. Another way to manipulate the furanose ring system is to prepare stereoisomers such as for example substitution at the 2'-position to give either the ribofuranose (down) or the arabinofuranose (up) or substitution at the 3'-position to give the xylofuranose or by altering the 2', and the 3'-position simultaneously to give a xylofuranose. The use of stereoisomers of the same substituent can give rise to completely different conformational geometry such as for example 2'-F which is 3'-endo in the ribo configuration and 2'-endo in the arabino configuration. The use of different anomeric and stereoisomeric sugars in oligomeric compounds is known in the art and amenable to the present invention.

The term "4'-thio modified nucleoside" is intended to include β-D-ribonucleosides having the 4'-O replaced with 4'-S. The term "4'-thio-2'-modified nucleoside" is intended to include 4'-thio modified nucleosides having the 2'-OH replaced with a 2'-substituent group. The preparation of 4'-thio modified nucleosides is disclosed in publications such as for example U.S. Patent 5,639,837 issued June 17, 1997 and PCT publication WO 2005/027962 published on March 31, 2005. The preparation of 4'-thio-2'-modified nucleosides and their incorporation into oligonucleotides is disclosed in the PCT publication WO 2005/027962 published on March 31, 2005. The 4'-thio-2'-modified nucleosides can be prepared with the same 2'-substituent groups previously mentioned with 2'-OCH₃, 2'-O-(CH₂)₂-OCH₃ and 2'-F are suitable groups.

Nucleosides can be modified at the 5'-position with substituent groups. Racemic as well as both R and S isomers can be prepared. Various 5'-modified nucleosides have been prepared and reported in the patent literature as well as in scientific literature, see for example: Mikhailov et al.., Nucleosides and Nucleotides: 1991, 10, 393-343; Saha et al., J. Org. Chem., 1995, 60, 788-789; Beigleman el al., Nucleosides and Nucleotides, 1995, 14, 901-905; Wang, et al., Bioorganic & Medicinal Chemistry Letters, 1999, 9, 885-890; and PCT Internation Application WO94/22890 published October 13, 1994,.

The term "bicyclic sugar modified nucleoside" is intended to include nucleosides having a second ring formed from the bridging of 2 atoms of the ribose ring. Such bicyclic sugar modified nucleosides can incorporate a number of different bridging groups that form the second ring and can be formed from different ring carbon atoms on the furanose ring. Bicyclic sugar modified nucleosides wherein the bridge links the 4' and the 2'-carbons and has the formula 4'-(CH₂)ₙ-O-2' wherein n is 1 or 2 are suitable. The synthesis of bicyclic sugar modified nucleosides is disclosed in US patents 6,268,490, 6,794,499 and published U.S. application 20020147332.

The synthesis and preparation of the bicyclic sugar modified nucleosides wherein the bridge is 4'-(CH₂-O-2' having nucleobases selected from adenine, cytosine, guanine, 5-methyl-cytosine, thymine and uracil, along with their oligomerization, and nucleic acid recognition properties have been described (Koshkin et al.,, Tetrahedron, 1998, 54, 3607-3630 and WO 98/39352 and WO 99/14226). The L isomer of this bicyclic sugar modified nucleoside has also been prepared (Frieden et al., Nucleic Acids Research, 2003, 21, 6365-6372). The 4'-CH₂-S-2' analog has also been prepared (Kumar et al., Bioorg. Med. Chem. Lett., 1998, 8, 2219-2222), and 2'-amino-LNA (Singh et al.. J. Org. Chem., 1998, 63, 10035-10039).

Oligomeric compounds of the present invention can also include one or more terminal phosphate moieties. Terminal phosphate moieties can be located at any terminal nucleoside but are suitable at 5'-terminal nucleosides with the 5'-terminal nucleoside of the antisense strand are also suitable. In one aspect, the terminal phosphate is unmodified having the formula -O-P(=O)(OH)OH. In another aspect, the terminal phosphate is modified such that one or more of the O and OH groups are replaced with H, O, S, N(R) or alkyl where R is H, an amino protecting group or unsubstituted or substituted alkyl.

The term "alkyl," as used herein, refers to a saturated straight or branched-hydrocarbon radical containing up to twenty four carbon atoms. Examples of alkyl groups include, but are not limited to, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms with from 1 to about 6 carbon atoms are also suitable. Alkyl groups as used herein may optionally include one or more further substituent groups.

The term "alkenyl," as used herein, refers to a straight or branched hydrocarbon chain radical containing up to twenty four carbon atoms having at least one carbon-carbon double bond. Examples of alkenyl groups include, but are not limited to, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 two about 6 carbon atoms are also suitable. Alkenyl groups as used herein may optionally include one or more further substituent groups.

The term "alkynyl," as used herein, refers to a straight or branched hydrocarbon radical containing up to twenty four carbon atoms and having at least one carbon-carbon triple bond. Examples of alkynyl groups include, but are not limited to, ethynyl, 1-propynyl, 1-butynyl, and the like. Alkynyl groups typically include from 2 to about 24 carbon atoms, more typically from 2 to about 12 carbon atoms with from 2 to about 6, carbon atoms are also suitable. Alkynyl groups as used herein may optionally include one or more further substituent groups.

The term "aliphatic," as used herein, refers to a straight or branched hydrocarbon radical containing up to twenty four carbon atoms wherein the saturation between any two carbon atoms is a single, double or triple bond. An aliphatic group can contain from 1 to about 24 carbon atoms, more typically from 1 to about 12 carbon atoms with from 1 to about 6 carbon atoms being desired. The straight or branched chain of an aliphatic group may be interrupted with one or more heteroatoms that include nitrogen, oxygen, sulfur and phosphorus. Such aliphatic groups interrupted by heteroatoms include without limitation polyalkoxys, such as polyalkylene glycols, polyamines, and polyimines, for example. Aliphatic groups as used herein may optionally include further substituent groups.

The term "alkoxy," as used herein, refers to a radical formed between an alkyl group and an oxygen atom wherein the oxygen atom is used to attach the alkoxy group to a parent molecule. Examples of alkoxy groups include, but are not limited to, methoxy, ethoxy, propoxy, isopropoxy, *n*-butoxy, sec-butoxy, *tert*-butoxy, n-pentoxy, neopentoxy, n-hexoxy and the like. Alkoxy groups as used herein may optionally include further substituent groups.

The terms "halo" and "halogen," as used herein, refer to an atom selected from fluorine, chlorine, bromine and iodine.

The terms "aryl" and "aromatic," as used herein, refer to a mono- or polycyclic carbocyclic ring system radical having one or more aromatic rings. Examples of aryl groups include, but not limited to, phenyl, naphthyl, tetrahydronaphthyl, indanyl, idenyl and the like. Aryl groups as used herein may optionally include further substituent groups.

The term "heterocyclic," as used herein, refers to a radical mono-, or poly-cyclic ring system that includes at least one heteroatom and is unsaturated, partially saturated or fully saturated, thereby including heteroaryl groups. Heterocyclic is also meant to include fused ring systems wherein one or more of the fused rings contain no heteroatoms. A heterocyclic group typically includes at least one atom selected from sulfur, nitrogen or oxygen. Examples of heterocyclic groups include, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, quinoxalinyl, pyridazinonyl, tetrahydrofuryl and the like. Heterocyclic groups as used herein may optionally include further substituent groups.

The terms "substituent and substituent group," as used herein, are meant to include groups that are typically added to other groups or parent compounds to enhance desired properties or give desired effects. Substituent groups can be protected or unprotected and can be added to one available site or to many available sites in a parent compound. Substituent groups may also be further substituted with other substituent groups and may be attached directly or via a linking group such as an alkyl or hydrocarbyl group to the parent compound. Such substituent groups include without limitation, halogen, hydroxyl, alkyl, alkenyl, alkynyl, acyl (-C(O)Rₐ), carboxyl (-C(O)O-Rₐ), aliphatic, alicyclic, alkoxy, substituted oxo (-O-Rₐ), aryl, aralkyl, heterocyclic, heteroaryl, heteroarylalkyl, amino (-NR_{b}R_{c}), imino(=NR_{b}), amido (-C(O)NR_{b}R_{c} or -N(R_{b})C(O)Rₐ), azido (-N₃), nitro (-NO₂), cyano (-CN), carbamido (-OC(O)NR_{b}R_{c} or-N(R_{b})C(O)ORₐ), ureido (-N(R_{b})C(O)NR_{b}R_{c}), thioureido (-N(R_{b})C(S)NR_{b}R_{c}), guanidinyl (-N(R_{b})C(=NR_{b})NR_{b}R_{c}), amidinyl (-C(=NR_{b})NR_{b}R_{c} or -N(R_{b})C(NR_{b})Rₐ), thiol (-SR_{b}), sulfinyl (-S(O)R_{b}), sulfonyl (-S(O)₂R_{b}) and sulfonamidyl (-S(O)₂NR_{b}R_{c} or -N(R_{b})S(O)₂R_{b}). Wherein each Rₐ, R_{b} and R_{c} is a further substituent group which can be without limitation alkyl, alkenyl, alkynyl, aliphatic, alkoxy, acyl, aryl, aralkyl, heteroaryl, alicyclic, heterocyclic and heteroarylalkyl.

The term "protecting group," as used herein, refers to a labile chemical moiety which is known in the art to protect reactive groups including without limitation, hydroxyl, amino and thiol groups, against undesired reactions during synthetic procedures. Protecting groups are typically used selectively and/or orthogonally to protect sites during reactions at other reactive sites and can then be removed to leave the unprotected group as is or available for further reactions. Protecting groups as known in the art are described generally in Greene and Wuts, Protective Groups in Organic Synthesis, 3rd edition, John Wiley & Sons, New York (1999).

Examples of hydroxyl protecting groups include, but are not limited to, benzyloxycarbonyl, 4-nitrobenzyloxycarbonyl, 4-bromobenzyloxycarbonyl, 4-methoxybenzyloxycarbonyl, methoxycarbonyl, tert-butoxycarbonyl (BOC), isopropoxycarbonyl, diphenylmethoxycarbonyl, 2,2,2-trichloroethoxycarbonyl, 2-(trimethylsilyl)ethoxycarbonyl, 2-furfuryloxycarbonyl, allyloxycarbonyl (Alloc), acetyl (Ac), formyl, chloroacetyl, trifluoroacetyl, methoxyacetyl, phenoxyacetyl, benzoyl (Bz), methyl, t-butyl, 2,2,2-trichloroethyl, 2-trimethylsilyl ethyl, 1,1-dimethyl-2-propenyl, 3-methyl-3-butenyl, allyl, benzyl (Bn), para-methoxybenzyldiphenylmethyl, triphenylmethyl (trityl), 4,4'-dimethoxytriphenylmethyl (DMT), substituted or unsubstituted 9-(9-phenyl)xanthenyl (pixyl), tetrahydrofuryl, methoxymethyl, methylthiomethyl, benzyloxymethyl, 2,2,2-trichloroethoxymethyl, 2-(trimethylsilyl)ethoxymethyl, methanesulfonyl, para-toluenesulfonyl, trimethylsilyl, triethylsilyl, triisopropylsilyl, and the like. Suitable hydroxyl protecting groups for the present invention are DMT and substituted or unsubstituted pixyl.

Examples of amino protecting groups include, but are not limited to, *t*-butoxycarbonyl (BOC), 9-fluorenylmethoxycarbonyl (Fmoc), benzyloxycarbonyl, and the like.
Examples of thiol protecting groups include, but are not limited to, triphenylmethyl (Trt), benzyl (Bn), and the like.

The synthesized oligomeric compounds can be separated from a reaction mixture and further purified by a method such as column chromatography, high pressure liquid chromatography, precipitation, or recrystallization. Further methods of synthesizing the compounds of the formulae herein will be evident to those of ordinary skill in the art. Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T. W. Greene and P. G. M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995), and subsequent editions thereof.

The compounds described herein contain one or more asymmetric centers and thus give rise to enantiomers, diastereomers, and other stereoisomeric forms that may be defined, in terms of absolute stereochemistry, as (R)- or (S)-, or as (D)- or (L)- for amino acids. The present invention is meant to include all such possible isomers, as well as their racemic and optically pure forms. Optical isomers may be prepared from their respective optically active precursors by the procedures described above, or by resolving the racemic mixtures. The resolution can be carried out in the presence of a resolving agent, by chromatography or by repeated crystallization or by some combination of these techniques which are known to those skilled in the art. Further details regarding resolutions can be found in Jacques, et al., Enantiomers, Racemates, and Resolutions (John Wiley & Sons, 1981). When the compounds described herein contain olefinic double bonds, other unsaturation, or other centers of geometric asymmetry, and unless specified otherwise, it is intended that the compounds include both E and Z geometric isomers or cis- and trans-isomers. Likewise, all tautomeric forms are also intended to be included. The configuration of any carbon-carbon double bond appearing herein is selected for convenience only and is not intended to designate a particular configuration unless the text so states; thus a carbon-carbon double bond or carbon-heteroatom double bond depicted arbitrarily herein as trans may be cis, trans, or a mixture of the two in any proportion.

The term "nucleoside," as used herein, refers to a base-sugar combination. The base portion of the nucleoside is normally a heterocyclic base moiety. The two most common classes of such heterocyclic bases are purines and pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. The term nucleoside is intended to include both modified and unmodified nucleosides. Within the oligonucleotide structure, the phosphate groups are commonly referred to as forming the backbone of the oligomeric compound. In forming oligonucleotides, the phosphate groups covalently link adjacent nucleosides to one another to form a linear polymeric compound. The normal internucleoside linkage of RNA and DNA is a 3' to 5' phosphodiester linkage.

In the context of this invention, the term "oligonucleoside" refers to a sequence of nucleosides that are joined by internucleoside linkages that do not have phosphorus atoms. Internucleoside linkages of this type are further described in the "modified internucleoside linkage" section below.

The term "oligonucleotide," as used herein, refers to an oligomer or polymer of ribonucleic acid (RNA) or deoxyribonucleic acid (DNA) composed of naturally occurring nucleobases, sugars and phosphodiester internucleoside linkages.

The terms "oligomer" and "oligomeric compound," as used herein, refer to a plurality of naturally occurring and/or non-naturally occurring nucleosides, joined together with internucleoside linking groups in a specific sequence. At least some of the oligomeric compounds can be capable of hybridizing a region of a target nucleic acid. Included in the terms "oligomer" and "oligomeric compound" are oligonucleotides, oligonucleotide analogs, oligonucleotide mimetics, oligonucleosides and chimeric combinations of these: As such the term oligomeric compound is broader than the term "oligonucleotide," including all oligomers having all manner of modifications including but not limited to those known in the art. Oligomeric compounds are typically structurally distinguishable from, yet functionally interchangeable with, naturally-occurring or synthetic wild-type oligonucleotides. Thus, oligomeric compounds include all such structures that function effectively to mimic the structure and/or function of a desired RNA or DNA strand, for example, by hybridizing to a target. Such non-naturally occurring oligonucleotides are often desired over the naturally occurring forms because they often have enhanced properties, such as for example, enhanced cellular uptake, enhanced affinity for nucleic acid target and increased stability in the presence of nucleases.

Oligomeric compounds can include compositions comprising double-stranded constructs such as, for example, two oligomeric compounds forming a double stranded hybridized construct or a single strand with sufficient self complementarity to allow for hybridization and formation of a fully or partially double-stranded compound. In one embodiment of the invention, double-stranded oligomeric compounds encompass short interfering RNAs (siRNAs). As used herein, the term "siRNA" is defined as a double-stranded construct comprising a first and second strand and having a central complementary portion between the first and second strands and terminal portions that are optionally complementary between the first and second strands or with a target nucleic acid. Each strand in the complex may have a length or from about 12 to about 24 nucleosides and may further comprise a central complementary portion having one of these defined lengths. Each strand may further comprise a terminal unhybridized portion having from 1 to about 6 nucleobases in length. The siRNAs may also have no terminal portions (overhangs) which is referred to as being blunt ended. The two strands of an siRNA can be linked internally leaving free 3' or 5' termini or can be linked to form a continuous hairpin structure or loop. The hairpin structure may contain an overhang on either the 5' or 3' terminus producing an extension of single-stranded character.

In one embodiment of the invention, compositions comprising double-stranded constructs are canonical siRNAs. As used herein, the term "canonical siRNA" is defined as a double-stranded oligomeric compound having a first strand and a second strand each strand being 21 nucleobases in length with the strands being complementary over 19 nucleobases and having on each 3' termini of each strand a deoxy thymidine dimer (dTdT) which in the double-stranded compound acts as a 3' overhang. In another aspect compositions comprise double-stranded constructs having overhangs may be of varying lengths with overhangs of varying lengths and may include compositions wherein only one strand has an overhang.

Further modifications can be made to the double-stranded compositions including modification of selected nucleobase positions (e.g. 5-methyl cytosine), sugar positions or to one of the internucleoside linkages. Alternatively, the two strands can be linked via a non-nucleic acid moiety or linker group. When formed from only one strand, dsRNA can take the form of a self-complementary hairpin-type molecule that doubles back on itself to form a duplex. Thus, the dsRNAs can be fully or partially double-stranded. When formed from two strands, or a single strand that takes the form of a self-complementary hairpin-type molecule doubled back on itself to form a duplex, the two strands (or duplex-forming regions of a single strand) are complementary RNA strands that base pair in Watson-Crick fashion.

In another embodiment, the oligomeric compounds of the invention are 19 to 30 nucleobases in length, or up to 30 nucleobases in length. One having ordinary skill in the art will appreciate that this embodies oligomeric compounds of 19, 20, 21, 22, 23, 24, 25, 26, 27, 28, 29, or 30 nucleobases in length, or any range therewithin.

In another embodiment, the oligomeric compounds of the invention are 19 to 23 nucleobases in length, or up to 23 nucleobases in length. One having ordinary skill in the art will appreciate that this embodies oligomeric compounds of 19, 20, 21, 22 or 23 nucleobases in length, or any range therewithin.

In another embodiment, the oligomeric compounds of the invention are 19 to 23 nucleobases in length, or up to 21 nucleobases in length. One having ordinary skill in the art will appreciate that this embodies oligomeric compounds of 19, 20, 21, 22 or 23 nucleobases in length, or any range therewithin.

In another embodiment, the oligomeric compounds of the invention are 19 to 21 nucleobases in length, or up to 21 nucleobases in length. One having ordinary skill in the art will appreciate that this embodies oligomeric compounds of 19, 20 or 21 nucleobases in length, or any range therewithin.

As used herein the term "heterocyclic base moiety" refers to nucleobases and modified or substitute nucleobases used to form nucleosides of the invention. The term "heterocyclic base moiety" includes unmodified nucleobases such as the native purine bases adenine (A) and guanine (G), and the pyrimidine bases thymine (T), cytosine (C) and uracil (U). The term is also intended to include all manner of modified or substitute nucleobases including but not limited to synthetic and natural nucleobases such as xanthine, hypoxanthine, 2-aminopyridine and 2-pyridone, 5-methylcytosine (5-me-C), 5-hydroxymethylenyl cytosine, 2-amino and 2-fluoroadenine, 2-propyl and other alkyl derivatives of adenine and guanine, 2-thio cytosine, uracil, thymine, 3-deaza guanine and adenine, 4-thiouracil, 5-uracil (pseudouracil), 5-propynyl (-C≡C-CH₃) uracil and cytosine and other alkynyl derivatives of pyrimidine bases, 5-halo particularly 5-bromo, 5-trifluoromethyl and other 5-substituted uracils and cytosines, 6-methyl and other alkyl derivatives of adenine and guanine, 6-azo uracil, cytosine and thymine, 7-methyl adenine and guanine, 7-deaza adenine and guanine, 8-halo, 8-amino, 8-aza, 8-thio, 8-thioalkyl, 8-hydroxyl and other 8-substituted adenines and guanines, universal bases, hydrophobic bases, promiscuous bases, size-expanded bases, and fluorinated bases as defined herein. Further modified nucleobases include tricyclic pyrimidines such as phenoxazine cytidine(1H-pyrimido[5,4-b][1,4]benzoxazin-2(3H)-one) and phenothiazine cytidine (1H-pyrimido[5,4-b] [1,4]benzothiazin-2(3H)-one).

Further nucleobases (and nucleosides comprising the nucleobases) include those disclosed in US Patent No. 3,687,808, those disclosed in The Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613, those disclosed in Limbach et al., Nucleic Acids Research, 1994, 22(12), 2183-2196, and those disclosed by Sanghvi, Y.S., Chapter 15, Antisense Research and Applications, pages 289-302, Crooke, S.T. and Lebleu, B. , ed., CRC Press, 1993.

Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyl-adenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278) and are especially useful when combined with 2'-O-methoxyethyl (2'-MOE) sugar modifications.

Representative U.S. patents that teach the preparation of certain of the above noted modified nucleobases as well as other modified nucleobases include, but are not limited to, the above noted U.S. 3,687,808, as well as U.S.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941, and 5,750,692.

The term "universal base" as used herein, refers to a moiety that may be substituted for any base. The universal base need not contribute to hybridization, but should not significantly detract from hybridization and typically refers to a monomer in a first sequence that can pair with a naturally occuring base, i.e A, C, G, T or U at a corresponding position in a second sequence of a duplex in which one or more of the following is true: (1) there is essentially no pairing (hybridization) between the two; or (2) the pairing between them occurs non-discriminant with the universal base hybridizing one or more of the the naturally occurring bases and without significant destabilization of the duplex. Exemplary universal bases include, without limitation, inosine, 5-nitroindole and 4-nitrobenzimidazole. For further examples and descriptions of universal bases see Survey and summary: the applications of universal DNA base analogs. Loakes, Nucleic Acids Research, 2001, 29, 12, 2437-2447.

The term "promiscuous base" as used herein, refers to a monomer in a first sequence that can pair with a naturally occuring base, i.e A, C, G, T or U at a corresponding position in a second sequence of a duplex in which the promiscuous base can pair non-discriminantly with more than one of the naturally occurring bases, i.e. A, C, G, T, U. Non-limiting examples of promiscuous bases are 6H,8H-3,4-dihydropyrimido[4,5-c][1,2]oxazin-7-one and N⁶-methoxy-2,6-diaminopurine, shown below. For further information, see Polymerase recognition of synthetic oligodeoxyribonucleotides incorporating degenerate pyrimidine and purine bases. Hill, et al., Proc. Natl. Acad. Sci., 1998, 95, 4258-4263.

Examples of G-clamps include substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one) and pyridoindole cytidine (H-pyrido[3',2':4,5]pyrrolo[2,3-d]pyrimidin-2-one).

Representative cytosine analogs that make 3 hydrogen bonds with a guanosine in a second oligonucleotide include 1,3-diazaphenoxazirie-2-one (Kurchavov et al., Nucleosides and Nucleotides, 1997, 16, 1837-1846), 1,3-diazaphenothiazine-2-one (Lin et al., J. Am. Chem. Soc. 1995, 117, 3873-3874) and 6,7,8,9-tetrafluoro-1,3-diazaphenoxazine-2-one (Wang et al., Tetrahedron Lett. 1998, 39, 8385-8388). When incorporated into oligonucleotides these base modifications hybridized with complementary guanine (the latter also hybridized with adenine) and enhanced helical thermal stability by extended stacking interactions (see U.S. Serial Number 10/013,295).

Oligomeric compounds of the invention may also contain one or more substituted sugar moieties such as the 2'-modified sugars discussed. A more comprehensive but not limiting list of sugar substituent groups includes: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly suitable are O((CH₂)ₙO)ₘCH₃, O(CH₂)ₙOCH₃, O(CH₂)ₙNH₂, O(CH₂)ₙCH₃, O(CH₂)ₙONH₂, and O(CH₂)ₙON-((CH₂)ₙCH₃)₂, where n and m are from 1 to about 10. Some oligonucleotides comprise a sugar substituent group selected from: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties.

One modification includes 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504) i.e., an alkoxyalkoxy group. One modification includes 2'-dimethylaminooxyethoxy, i.e., a O(CH₂)₂ON(CH₃)₂ group, also known as 2'-DMAOE, as described in examples hereinbelow, and 2'-dimethylaminoethoxyethoxy (also known in the art as 2'-O-dimethyl-amino-ethoxy-ethyl or 2'-DMAEOE), i.e., 2'-O-CH₂-O-CH₂-N(CH₃)₂.

Other sugar substituent groups include methoxy (-O-CH₃), aminopropoxy (-OCH₂CH₂CH₂NH₂), allyl (-CH₂-CH=CH₂), -O-allyl (-O-CH₂-CH=CH₂) and fluoro (F). 2'-Sugar substituent groups may be in the arabino (up) position or ribo (down) position. One 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar. Representative U.S. patents that teach the preparation of such modified sugar structures include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920.

Representative sugar substituent groups include groups of formula Iₐ or IIₐ: 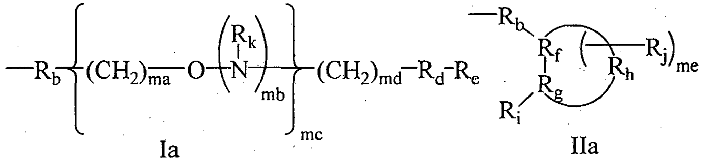 wherein:
R_{b} is O, S or NH;
R_{d} is a single bond, O, S or C(=O);
Rₑ is C₁-C₁₀ alkyl, N(Rₖ)(Rₘ), N(Rₖ)(Rₙ), N=C(Rₚ)(R_{q}), N=C(Rₚ)(Rᵣ) or has formula IIIa; 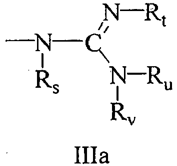
Rₚ and Rq are each independently hydrogen or C₁-C₁₀alkyl;
Rᵣ is -Rₓ-R_{y};
each Rₛ, Rₜ, Rᵤ and Rᵥ is, independently, hydrogen, C(O)R_{w}, substituted or unsubstituted C₁-C₁₀ alkyl, substituted or unsubstituted C₂-C₁₀ alkenyl, substituted or unsubstituted C₂-C₁₀ alkynyl, alkylsulfonyl, arylsulfonyl, a chemical functional group or a conjugate group, wherein the substituent groups are selected from hydroxyl, amino, alkoxy, carboxy, benzyl, phenyl, nitro, thiol, thioalkoxy, halogen, alkyl, aryl, alkenyl and alkynyl;
or optionally, Rᵤ and Rᵥ, together form a phthalimido moiety with the nitrogen atom to which they are attached;
each R_{w} is, independently, substituted or unsubstituted C₁-C₁₀alkyl, trifluoromethyl, cyanoethyloxy, methoxy, ethoxy, t-butoxy, allyloxy, 9-fluorenylmethoxy, 2-(trimethylsilyl)-ethoxy, 2,2,2-trichloroethoxy, benzyloxy, butyryl, iso-butyryl, phenyl or aryl;
Rₖ is hydrogen, a nitrogen protecting group or -Rₓ-R_{y};
Rₚ is hydrogen, a nitrogen protecting group or -Rₓ-R_{y};
Rₓ is a bond or a linking moiety;
R_{y} is a chemical functional group, a conjugate group or a solid support medium;
each Rₘ and Rₙ is, independently, H, a nitrogen protecting group, substituted or unsubstituted C₁-C₁₀ alkyl, substituted or unsubstituted C₂-C₁₀ alkenyl, substituted or unsubstituted C₂-C₁₀ alkynyl, wherein the substituent groups are selected from hydroxyl, ainino, alkoxy, carboxy, benzyl, phenyl, nitro, thiol, thioalkoxy, halogen, alkyl, aryl, alkenyl, alkynyl; NH₃⁺, N(Rᵤ)(Rᵥ), guanidino and acyl where the acyl is an acid amide or an ester;
or Rₘ and Rₙ, together, are a nitrogen protecting group, are joined in a ring structure that optionally includes an additional heteroatom selected from N and O or are a chemical functional group;
Rᵢ is OR_{z}, SR_{z}, or N(R_{z})₂;
each R_{z} is, independently, H, C₁-C₈ alkyl, C₁-C₈ haloalkyl, C(=NH)N(H)Rᵤ, C(=O)N(H)Rᵤ or OC(=O)N(H)Rᵤ;
R_{f}, R_{g} and Rₕ comprise a ring system having from about 4 to about 7 carbon atoms or having from about 3 to about 6 carbon atoms and 1 or 2 heteroatoms wherein the heteroatoms are selected from oxygen, nitrogen and sulfur and wherein the ring system is aliphatic, unsaturated aliphatic, aromatic, or saturated or unsaturated heterocyclic;
Rⱼ is alkyl or haloalkyl having 1 to about 10 carbon atoms, alkenyl having 2 to about 10 carbon atoms, alkynyl having 2 to about 10 carbon atoms, aryl having 6 to about 14 carbon atoms, N(Rₖ)(Rₘ) ORₖ, halo, SRₖ or CN;
mₐ is 1 to about 10;
each mb is, independently, 0 or 1;
m_{c} is 0 or an integer from 1 to 10;
md is an integer from 1 to 10;
me is from 0, 1 or 2; and
provided that when mc is 0, md is greater than 1.

Representative substituents groups of Formula I are disclosed in U.S. Serial No. 09/130,973, filed August 7, 1998, entitled "Capped 2'-Oxyethoxy Oligonucleotides."

Representative cyclic substituent groups of Formula II are disclosed in U.S. Serial No. 09/123,108, filed July 27, 1998, entitled "RNA Targeted 2'-Oligomeric compounds that are Conformationally Preorganized".

Particular sugar substituent groups include O((CH₂)ₙO)ₘCH₃, O(CH₂)ₙOCH₃, O(CH₂)ₙNH₂, O(CH₂)ₙCH₃ O(CH₂)ₙONH₂, and O(CH₂)ₙON((CH₂)ₙCH₃))₂, where n and m are from 1 to about 10.

Representative guanidino substituent groups that are shown in formula III and IV are disclosed in U.S. Serial No. 09/349,040, entitled "Functionalized Oligomers", filed July 7, 1999.

Representative acetamido substituent groups are disclosed in U.S. Patent 6,147,200.

Representative dimethylaminoethyloxyethyl substituent groups are disclosed in International Patent Application PCT/US99/17895, entitled "2'-O-Dimethylaminoethyloxyethyl-Oligomeric compounds", filed August 6, 1999.

The terms "modified internucleoside linkage" and "modified backbone," or simply "modified linkage" as used herein, refer to modifications or replacement of the naturally occurring phosphodiester internucleoside linkage connecting two adjacent nucleosides within an oligomeric compound. Such modified linkages include those that have a phosphorus atom and those that do not have a phosphorus atom.

Internucleoside linkages containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Oligonucleotides having inverted polarity can comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included. Representative U.S. patents that teach the preparation of the above phosphorus-containing linkages include, but are not limited to, U.S.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050.

In the *C. elegans* system, modification of the internucleotide linkage (phosphorothioate in place of phosphodiester) did not significantly interfere with RNAi activity, indicating that oligomeric compounds of the invention can have one or more modified internucleoside linkages, and retain activity. Indeed, such modified internucleoside linkages are often desired over the naturally occurring phosphodiester linkage because of advantageous properties they can impart such as, for example, enhanced cellular uptake, enhanced affinity for nucleic acid target and increased stability in the presence of nucleases.

Another phosphorus containing modified internucleoside linkage is the phosphonomonoester (see U.S. Patents 5,874,553 and 6,127,346). Phosphonomonoester nucleic acids have useful physical, biological and pharmacological properties in the areas of inhibiting gene expression (antisense oligonucleotides, ribozymes, sense oligonucleotides and triplex-forming oligonucleotides), as probes for the detection of nucleic acids and as auxiliaries for use in molecular biology.

As previously defined an oligonucleoside refers to a sequence of nucleosides that are joined by internucleoside linkages that do not have phosphorus atoms. Non-phosphorus containing internucleoside linkages include short chain alkyl, cycloalkyl, mixed heteroatom alkyl, mixed heteroatom cycloalkyl, one or more short chain heteroatomic and one or more short chain heterocyclic. These internucleoside linkages include but are not limited to siloxane, sulfide, sulfoxide, sulfone, acetyl, formacetyl, thioformacetyl, methylene formacetyl, thioformacetyl, alkeneyl, sulfamate; methyleneimino, methylenehydrazino, sulfonate, sulfonamide, amide and others having mixed N, O, S and CH₂ component parts. Representative U.S. patents that teach the preparation of the above oligonucleosides include, but are not limited to, U.S.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

Some additional examples of modified internucleoside linkages that do not contain a phosphorus atom therein include, -CH₂-NH-O-CH₂-, -CH₂-N(CH₃)-O-CH₂- (known as a methylene (methylimino) or MMI backbone), -CH₂-O-N(CH₃)-CH₂-, -CH₂-N(CH₃)-N(CH₃)-CH₂- and -O-N(CH₃)-CH₂-CH₂- (wherein the native phosphodiester internucleotide linkage is represented as -O-P(=O)(OH)-O-CH₂-). The MMI type and amide internucleoside linkages are disclosed in the below referenced U.S. patents 5,489,677 and 5,602,240, respectively.

Another modification that can enhance the properties of an oligomeric compound or can be used to track the oligomeric compound or its metabolites is the attachment of one or more moieties or conjugates. Properties that are typically enhanced include without limitation activity, cellular distribution and cellular uptake. In one embodiment, such modified oligomeric compounds are prepared by covalently attaching conjugate groups to functional groups available on an oligomeric compound such as hydroxyl or amino functional groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve properties including but not limited to oligomer uptake, enhance oligomer resistance to degradation, and/or strengthen sequence-specific hybridization with RNA. Groups that enhance the pharmacokinetic properties, in the context of this invention, include groups that improve properties including but not limited to oligomer uptake, distribution, metabolism and excretion. Representative conjugate groups are disclosed in International Patent Application PCT/US92/09196.

Conjugate groups include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

The oligomeric compounds of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in U.S. Patent Application 09/334,130.

Representative U.S. patents that teach the preparation of such oligonucleotide conjugates include, but are not limited to, U.S.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

Linking groups or bifunctional linking moieties such as those known in the art are amenable to the present invention. Linking groups are useful for attachment of chemical functional groups, conjugate groups, reporter groups and other groups to selective sites in a parent compound such as for example an oligomeric compound. Linking moieties are also useful for covalently binding one or more groups to a support medium for oliogmer synthesis. Generelly the linking moieties used for oligomer sythesis covalently bind the ultimate 3'-nucleoside (and thus the nascent oligonucleotide) to the solid support medium (or other medium) during synthesis, but which is cleaved under conditions orthogonal to the conditions under which the 5'-protecting group, and if applicable any 2'-protecting group, are removed.

In general a bifunctional linking moiety comprises a hydrocarbyl moiety having two functional groups. One of the functional groups is selected to bind to a parent molecule or compound of interest and the other is selected to bind essentially any selected group such as a chemical functional group or a conjugate group. In some embodiments, the linker comprises a chain structure or an oligomer of repeating units such as ethylene glyol or amino acid units. In other embodiments, the linker comprises a chain structure or an oligomer of repeating units such as ethylene glyol or amino acid units in combination with a alicyclic or heterocyclic ring system. Examples of functional groups that are routinely used in bifunctional linking moieties include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like, alone or in combination with a heterocyclcic ring system.

Further linking moieties include, but are not limited to, C₁-C₁₂ alkylene (e.g. methylene, ethylene (e.g. ethyl-1,2-ene), propylene (e.g. propyl-1,2-ene, propyl-1,3-ene), butylene, (e.g. butyl-1,4-ene, 2-methylpropyl-1,3-ene), pentylene, hexylene, heptylene, octylene, decylene, dodecylene), etc. Exemplary cycloalkylene groups include C₃-C₁₂ cycloalkylene groups, such as cyclopropylene, cyclobutylene, cyclopentanyl-1,3-ene, cyclohexyl-1,4-ene, etc. Exemplary arylene linking moietys include, but are not limited to, mono- or bicyclic arylene groups having from 6 to about 14 carbon atoms, e.g. phenyl-1,2-ene, naphthyl-1,6-ene, napthyl-2,7-ene, anthracenyl, etc. Exemplary heterocyclyl groups within the scope of the invention include mono- or bicyclic aryl groups having from about 4 to about 12 carbon atoms and about 1 to about 4 hetero atoms, such as N, O and S, where the cyclic moieties may be partially dehydrogenated. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

Oligomeric compounds used in the compositions of the present invention can also be modified to have one or more stabilizing groups that are generally attached to one or both termini of oligomeric compounds to enhance properties such as for example nuclease stability. Included in stabilizing groups are cap structures. The terms "cap structure" or "terminal cap moiety," as used herein, refer to chemical modifications, which can be attached to one or both of the termini of an oligomeric compound. These terminal modifications protect the oligomeric compounds having terminal nucleic acid moieties from exonuclease degradation, and can help in delivery and/or localization within a cell. The cap can be present at the 5'-terminus (5'-cap) or at the 3'-terminus (3'-cap) or can be present on both termini. In non-limiting examples, the 5'-cap includes inverted abasic residue (moiety), 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl riucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (for more details see Wincott et al., International PCT publication No. WO 97/26270).

Particularly suitable 3'-cap structures of the present invention include, for example 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Tyer, 1993, Tetrahedron 49, 1925 and Published U.S. Patent Application Publication No. US 2005/0020525 published on January 27, 2005). Further 3' and 5'-stabilizing groups that can be used to cap one or both ends of an oligomeric compound to impart nuclease stability include those disclosed in WO 03/004602.

Oligomerization of modified and unmodified nucleosides is performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA:Protein Interactions, Ed. Smith (1998), 1-36. Gallo et al., Tetrahedron (2001), 57, 5707-5713) synthesis as appropriate. In addition specific protocols for the synthesis of oligomeric compounds of the invention are illustrated in the examples below.

Support bound oligonucleotide synthesis relies on sequential addition of nucleotides to one end of a growing chain. Typically, a first nucleoside (having protecting groups on any exocyclic amine functionalities present) is attached to an appropriate glass bead support and nucleotides bearing the appropriate activated phosphite moiety, i.e. an "activated phosphorous group" (typically nucleotide phosphoramidites, also bearing appropriate protecting groups) are added stepwise to elongate the growing oligonucleotide. Additional methods for solid-phase synthesis may be found in Caruthers U.S. Patents Nos. 4,415,732; 4,458,066; 4,500,707; 4,668,777; 4,973,679; and 5,132,418; and Koster U.S. Patents Nos. 4,725,677 and Re. 34,069.

Oligonucleotides are generally prepared either in solution or on a support medium, e.g. a solid support medium. In general a first synthon (e.g. a monomer, such as a nucleoside) is first attached to a support medium, and the oligonucleotide is then synthesized by sequentially coupling monomers to the support-bound synthon. This iterative elongation eventually results in a final oligomeric compound or other polymer such as a polypeptide. Suitable support medium can be soluble or insoluble, or may possess variable solubility in different solvents to allow the growing support bound polymer to be either in or out of solution as desired. Traditional support medium such as solid support media are for the most part insoluble and are routinely placed in reaction vessels while reagents and solvents react with and/or wash the growing chain until the oligomer has reached the target length, after which it is cleaved from the support and, if necessary further worked up to produce the final polymeric compound. More recent approaches have introduced soluble supports including soluble polymer supports to allow precipitating and dissolving the iteratively synthesized product at desired points in the synthesis (Gravert et al., Chem. Rev., 1997, 97, 489-510).

The term support medium is intended to include all forms of support known to one of ordinary skill in the art for the synthesis of oligomeric compounds and related compounds such as peptides. Some representative support medium that are amenable to the methods of the present invention include but are not limited to the following: controlled pore glass (CPG); oxalyl-controlled pore glass (see, e.g., Alul, et al., Nucleic Acids Research 1991, 19, 1527); silica-containing particles, such as porous glass beads and silica gel such as that formed by the reaction of trichloro-[3-(4-chloromethyl)phenyl]propylsilane and porous glass beads (see Parr and Grohmann, Angew. Chem. Internal. Ed. 1972, 11, 314, sold under the trademark "PORASIL E" by Waters Associates, Framingham, Mass., USA); the mono ester of 1,4-dihydroxymethyl-enlybenzene and silica (see Bayer and Jung, Tetrahedron Lett., 1970, 4503, sold under the trademark "BIOPAK" by Waters Associates); TENTAGEL (see, e.g., Wright, et al., Tetrahedron Letters 1993, 34, 3373); cross-linked styrene/divinylbenzene copolymer beaded matrix or POROS, a copolymer of polystyrene/divinylbenzene (available from Perceptive Biosystems); soluble support medium, polyethylene glycol PEGs (see Bonora et al., Organic Process Research & Development, 2000, 4, 225-231).

Certain heteroaryl groups that may be mentioned as being within the scope of the invention include: pyrrolidinyl, piperidinyl (e.g. 2,5-piperidinyl, 3,5-piperidinyl), piperazinyl, tetrahydrothiophenyl, tetrahydrofuranyl, tetrahydro quinolinyl, tetrahydro isoquinolinyl, tetrahydroquinazolinyl, tetrahydroquinoxalinyl, etc. Exemplary heteroarylene groups include mono- or bicyclic aryl groups having from about 4 to about 12 carbon atoms and about 1 to about 4 hetero atoms, such as N, O and S. Certain heteroaryl groups that may be mentioned as being within the scope of the invention include: pyridylene (e.g. pyridyl-2,5-ene, pyridyl-3,5-ene), pyrimidinyl, thiophenyl, furanyl, quinolinyl, isoquinolinyl, quinazolinyl, quinoxalinyl, etc.

Commercially available equipment routinely used for the support medium based synthesis of oligomeric compounds and related compounds is sold by several vendors including, for example, Applied Biosystems (Foster City, CA). Any other means for such synthesis known in the art may additionally or alternatively be employed. Suitable solid phase techniques, including automated synthesis techniques, are described in F. Eckstein (ed.), Oligonucleotides and Analogues, a Practical Approach, Oxford University Press, New York (1991).

Although a lot of research has focused on the synthesis of oligoribonucleotides the main RNA synthesis strategies that are presently being used commercially include 5'-O-DMT-2'-O-t-butyldimethylsilyl (TBDMS), 5'-O-DMT-2'-O-[1(2-fluorophenyl)-4-methoxypiperidin-4-yl] (FPMP), 2'-O-[(triisopropylsilyl)oxy]methyl (2'-O-CH₂-o-Si(iPr)₃ (TOM), and the 5'-O-silyl ether-2'-ACE (5'-O-bis(trimethylsiloxy)cyclododecyloxysilyl ether (DOD)-2'-O-bis(2-acetoxyethoxy)methyl (ACE). A current list of some of the major companies currently offering RNA products include Pierce Nucleic Acid Technologies, Dharmacon Research Inc., Ameri Biotechnologies Inc., and Integrated DNA Technologies, Inc. One company, Princeton Separations, is marketing an RNA synthesis activator advertised to reduce coupling times especially with TOM and TBDMS chemistries. Such an activator would also be amenable to the present invention. The primary groups being used for commercial RNA synthesis are:
TBDMS = 5'-O-DMT-2'-O-t-butyldimethylsilyl;
TOM = 2'-O-[(triisopropylsilyl)oxy]methyl;
DOD/ACE = 5'-O-bis(trimethylsiloxy)cyclododecyloxysilylether-2'-O-bis(2-acetoxyethoxy)methyl;
FPMP = 5'-O-DMT-2'-O-[1(2-fluorophenyl)-4-methoxypiperidin-4-yl].

All of the aforementioned RNA synthesis strategies are amenable to the present invention. Strategies that would be a hybrid of the above e.g. using a 5'-protecting group from one strategy with a 2'-O-protecting from another strategy is also amenable to the present invention.

The terms "antisense" or "(antisense inhibition" as used herein refer to the hybridization of an oligomeric compound or a portion thereof with a selected target nucleic acid. Multiple antisense mechanisms exist by which oligomeric compounds can be used to modulate gene expression in mammalian cells. Such antisense inhibition is typically based upon hydrogen bonding-based hybridization of complementary strands or segments such that at least one strand or segment is cleaved, degraded, or otherwise rendered inoperable. In this regard, it is presently suitable to target specific nucleic acid molecules and their functions for such antisense inhibition.

The functions of DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA.

A commonly exploited antisense mechanism is RNase H-dependent degradation of a targeted RNA. RNase H is a ubiquitously expressed endonuclease that recognizes antisense DNA-RNA heteroduplexes, hydrolyzing the RNA strand. A further antisense mechanism involves the utilization of enzymes that catalyze the cleavage of RNA-RNA duplexes. These reactions are catalyzed by a class of RNAse enzymes including but not limited to RNAse III and RNAse L. The antisense mechanism known as RNA interference (RNAi) is operative on RNA-RNA hybrids and the like. Both RNase H-based antisense (usually using single-stranded compounds) and RNA interference (usually using double-stranded compounds known as siRNAs) are antisense mechanisms, typically resulting in loss of target RNA function.

Optimized siRNA and RNase H-dependent oligomeric compounds behave similarly in terms of potency, maximal effects, specificity and duration of action, and efficiency. Moreover it has been shown that in general, activity of dsRNA constructs correlated with the activity of RNase H-dependent single-stranded antisense oligomeric compounds targeted to the same site. One major exception is that RNase H-dependent antisense oligomeric compounds were generally active against target sites in pre-mRNA whereas siRNAs were not.

These data suggest that, in general, sites on the target RNA that were not active with RNase H-dependent oligonucleotides were similarly not good sites for siRNA. Conversely, a significant degree of correlation between active RNase H oligomeric compounds and siRNA was found, suggesting that if a site is available for hybridization to an RNase H oligomeric compound, then it is also available for hybridization and cleavage by the siRNA complex. Consequetly, once suitable target sites have been determined by either antisense approach, these sites can be used to design constructs that operate by the alternative antisense mechanism (Vickers et al., J. Biol. Chem., 2003, 278, 7108). Moreover, once a site has been demonstrated as active for either an RNAi or an RNAse H oligomeric compound, a single-stranded RNAi oligomeric compound (ssRNAi or asRNA) can be designed.

The oligomeric compounds and methods of the present invention are also useful in the study, characterization, validation and modulation of small non-coding RNAs. These include, but are not limited to, microRNAs (miRNA), small nuclear RNAs (snRNA), small nucleolar RNAs (snoRNA), small temporal RNAs (stRNA) and tiny non-coding RNAs (tncRNA) or their precursors or processed transcripts or their association with other cellular components.

Small non-coding RNAs have been shown to function in various developmental and regulatory pathways in a wide range of organisms, including plants, nematodes and mammals. MicroRNAs are small non-coding RNAs that are processed from larger precursors by enzymatic cleavage and inhibit translation of mRNAs. stRNAs, while processed from precursors much like miRNAs, have been shown to be involved in developmental timing regulation. Other non-coding small RNAs are involved in events as diverse as cellular splicing of transcripts, translation, transport, and chromosome organization.

As modulators of small non-coding RNA function, the oligomeric compounds of the present invention find utility in the control and manipulation of cellular functions or processes such as regulation of splicing, chromosome packaging or methylation, control of developmental timing events, increase or decrease of target RNA expression levels depending on the timing of delivery into the specific biological pathway and translational or transcriptional control. In addition, the oligomeric compounds of the present invention can be modified in order to optimize their effects in certain cellular compartments, such as the cytoplasm, nucleus, nucleolus or mitochondria.

The compounds of the present invention can further be used to identify components of regulatory pathways of RNA processing or metabolism as well as in screening assays or devices.

Targeting an oligomeric compound to a particular nucleic acid molecule, in the context of this invention, can be a multistep process. The process usually begins with the identification of a target nucleic acid whose function is to be modulated. The terms "target nucleic acid" and "nucleic acid target", as used herein, refer to any nucleic acid capable of being targeted including without limitation DNA (a cellular gene), RNA (including pre-mRNA and mRNA or portions thereof) transcribed from such DNA, and also cDNA derived from such RNA. In one embodiment the modulation of expression of a selected gene is associated with a particular disorder or disease state. In another embodiment the target nucleic acid is a nucleic acid molecule from an infectious agent.

The targeting process usually also includes determination of at least one target region, segment, or site within the target nucleic acid for the antisense interaction to occur such that the desired effect, e.g., modulation of expression, will result. Within the context of the present invention as it is applied to a nucleic acid target, the term "region" is defined as a portion of the target nucleic acid having at least one identifiable structure, function, or characteristic. Within regions of target nucleic acids are segments. "Segments" are defined as smaller or sub-portions of regions within a target nucleic acid. "Sites," as used in the present invention, are defined as positions within a target nucleic acid. The terms region, segment, and site can also be used to describe an oligomeric compound of the invention such as for example a gapped oligomeric compound having 3 separate regions or segments.

Since, as is known in the art, the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function *in vivo*. Thus, the terms "translation initiation codon" and "start codon" can encompass many codon sequences, even though the initiator amino acid in each instance is typically methionine (in eukaryotes) or formylmethionine (in prokaryotes). It is also known in the art that eukaryotic and prokaryotic genes may have two or more alternative start codons, any one of which may be preferentially utilized for translation initiation in a particular cell type or tissue, or under a particular set of conditions. In the context of the invention, "start codon" and "translation initiation codon" refer to the codon or codons that are used *in vivo* to initiate translation of an mRNA transcribed from a gene encoding a nucleic acid target, regardless of the sequence(s) of such codons. It is also known in the art that a translation termination codon (or "stop codon") of a gene may have one of three sequences, i.e., 5'-UAA, 5'-UAG and 5'-UGA (the corresponding DNA sequences are 5'-TAA, 5'-TAG and 5'-TGA, respectively).

The terms "start codon region" and "translation initiation codon region" refer to a portion of such an mRNA or gene that encompasses from about 25 to about 50 contiguous nucleotides in either direction (i.e., 5' or 3') from a translation initiation codon. Similarly, the terms "stop codon region" and "translation termination codon region" refer to a portion of such an mRNA or gene that encompasses from about 25 to about 50 contiguous nucleotides in either direction (i.e., 5' or 3') from a translation termination codon. Consequently, the "start codon region" (or "translation initiation codon region") and the "stop codon region" (or "translation termination codon region") are all regions which may be targeted effectively with the antisense oligomeric compounds of the present invention.

The open reading frame (ORF) or "coding region," which is known in the art to refer to the region between the translation initiation codon and the translation termination codon, is also a region which may be targeted effectively. Within the context of the present invention, one region is the intragenic region encompassing the translation initiation or termination codon of the open reading frame (ORF) of a gene.

Other target regions include the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene), and the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap site. It is also suitable to target the 5' cap region.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. Targeting splice sites, i.e., intron-exon junctions or exon-intron junctions, may also be particularly useful in situations where aberrant splicing is implicated in disease, or where an overproduction of a particular splice product is implicated in disease. Aberrant fusion junctions due to rearrangements or deletions are also suitable target sites. mRNA transcripts produced via the process of splicing of two (or more) mRNAs from different gene sources are known as "fusion transcripts". It is also known that introns can be effectively targeted using antisense oligomeric compounds targeted to, for example, DNA or pre-mRNA.

It is also known in the art that alternative RNA transcripts can be produced from the same genomic region of DNA. These alternative transcripts are generally known as "variants". More specifically, "pre-mRNA variants" are transcripts produced from the same genomic DNA that differ from other transcripts produced from the same genomic DNA in either their start or stop position and contain both intronic and exonic sequences.

Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

It is also known in the art that variants can be produced through the use of alternative signals to start or stop transcription and that pre-mRNAs and mRNAs can possess more that one start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. Within the context of the invention, the types of variants described herein are also suitable target nucleic acids.

The locations on the target nucleic acid to which the antisense oligomeric compounds hybridize are hereinbelow referred to as "suitable target segments." As used herein the term "suitable target segment" is defined as at least an 8-nucleobase portion of a target region to which an active antisense oligomeric compound is targeted. While not wishing to be bound by theory, it is presently believed that these target segments represent portions of the target nucleic acid which are accessible for hybridization.

Exemplary antisense oligomeric compounds include oligomeric compounds that comprise at least the 8 consecutive nucleobases from the 5'-terminus of a targeted nucleic acid e.g. a cellular gene or mRNA transcribed from the gene (the remaining nucleobases being a consecutive stretch of the same oligonucleotide beginning immediately upstream of the 5'-terminus of the antisense oligomeric compound which is specifically hybridizable to the target nucleic acid and continuing until the oligonucleotide contains from about 8 to about 80 nucleobases). Similarly, antisense oligomeric compounds are represented by oligonucleotide sequences that comprise at least the 8 consecutive nucleobases from the 3'-terminus of one of the illustrative antisense oligomeric compounds (the remaining nucleobases being a consecutive stretch of the same oligonucleotide beginning immediately downstream of the 3'-terminus of the antisense oligomeric compound which is specifically hybridizable to the target nucleic acid and continuing until the oligonucleotide contains from about 8 to about 80 nucleobases). One having skill in the art armed with the antisense oligomeric compounds illustrated herein will be able, without undue experimentation, to identify further antisense oligomeric compounds.

Once one or more target regions, segments or sites have been identified, antisense oligomeric compounds are chosen which are sufficiently complementary to the target, i.e., hybridize sufficiently well and with sufficient specificity, to give the desired effect.

In accordance with one embodiment of the present invention, a series of nucleic acid duplexes comprising the antisense oligomeric compounds of the present invention and their complements can be designed for a specific target or targets. The ends of the strands may be modified by the addition of one or more natural or modified nucleobases to form an overhang. The sense strand of the duplex is then designed and synthesized as the complement of the antisense strand and may also contain modifications or additions to either terminus. For example, in one embodiment, both strands of the duplex would be complementary over the central nucleobases, each having overhangs at one or both termini.

RNA strands of the duplex can be synthesized by methods disclosed herein or purchased from various RNA synthesis companies such as for example Dharmacon Research Inc., (Lafayette, CO). Once synthesized, the complementary strands are annealed. The single strands are aliquoted and diluted to a concentration of 50 µM. Once diluted, 30 µL of each strand is combined with 15 µL of a 5X solution of annealing buffer. The final concentration of the buffer is 100 mM potassium acetate, 30 mM HEPES-KOH pH 7.4, and 2mM magnesium acetate. The final volume is 75 µL. This solution is incubated for 1 minute at 90°C and then centrifuged for 15 seconds. The tube is allowed to sit for 1 hour at 37°C at which time the dsRNA duplexes are used in experimentation. The final concentration of the dsRNA compound is 20 µM. This solution can be stored frozen (-20°C) and freeze-thawed up to 5 times.

Once prepared, the desired synthetic duplexs are evaluated for their ability to modulate target expression. When cells reach 80% confluency, they are treated with synthetic duplexs comprising at least one oligomeric compound of the invention. For cells grown in 96-well plates, wells are washed once with 200 µL OPTI-MEM-1 reduced-serum medium (Gibco BRL) and then treated with 130 µL of OPTI-MEM-1 containing 12 µg/mL LIPOFECTIN (Gibco BRL) and the desired dsRNA compound at a final concentration of 200 nM. After 5 hours of treatment, the medium is replaced with fresh medium. Cells are harvested 16 hours after treatment, at which time RNA is isolated and target reduction measured by RT-PCR.

In a further embodiment, the "suitable target segments" identified herein may be employed in a screen for additional oligomeric compounds that modulate the expression of a target. "Modulators" are those oligomeric compounds that decrease or increase the expression of a nucleic acid molecule encoding a target and which comprise at least an 8-nucleobase portion which is complementary to a suitable target segment. The screening method comprises the steps of contacting a suitable target segment of a nucleic acid molecule encoding a target with one or more candidate modulators, and selecting for one or more candidate modulators which decrease or increase the expression of a nucleic acid molecule encoding a target. Once it is shown that the candidate modulator or modulators are capable of modulating (e.g. either decreasing or increasing) the expression of a nucleic acid molecule encoding a target, the modulator may then be employed in further investigative studies of the function of a target, or for use as a research, diagnostic, or therapeutic agent in accordance with the present invention.

The suitable target segments of the present invention may also be combined with their respective complementary antisense oligomeric compounds of the present invention to form stabilized double stranded (duplexed) oligonucleotides.

In the context of this invention, "hybridization" means hydrogen bonding, which may be Watson-Crick, Hoogsteen or reversed Hoogsteen hydrogen bonding, between the heterocyclic base moieties of complementary nucleosides. For example, adenine and thymine are complementary nucleobases which pair through the formation of hydrogen bonds. "Complementary," as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense oligomeric compound need not be 100% complementary to that of its target nucleic acid to be specifically hybridizable. An antisense oligomeric compound is specifically hybridizable when binding of the compound to the target DNA or RNA molecule interferes with the normal function of the target DNA or RNA to cause a complete or partial loss of function, and there is a sufficient degree of complementarity to avoid non-specific binding of the antisense oligomeric compound to non-target sequences under conditions in which specific binding is desired, i.e., under physiological conditions in the case of therapeutic treatment, or under conditions in which *in vitro* or *in vivo* assays are performed. Moreover, an oligonucleotide may hybridize over one or more segments such that intervening or adjacent segments are not involved in the hybridization event (e.g., a loop structure, mismatch or hairpin structure).

The oligomeric compounds of the present invention comprise at least 70%, at least 75%, at least 80%, at least 85%, at least 90%, at least 95%, at least 99%, or 100% sequence complementarity to a target region within the target nucleic acid sequence to which they are targeted. For example, an antisense oligomeric compound in which 18 of 20 nucleobases of the antisense oligomeric compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense oligomeric compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention.

Percent complementarity of an antisense oligomeric compound with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656). Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math., 1981, 2, 482-489). In some embodiments, homology, sequence identity or complementarity, between the oligomeric compound and the target is about 70%, about 75%, about 80%, about 85%, about 90%, about 92%, about 94%, about 95%, about 96%, about 97%, about 98%, about 99%, or 100%.

In some embodiments, "suitable target segments" may be employed in a screen for additional oligomeric compounds that modulate the expression of a selected protein. "Modulators" are those oligomeric compounds that decrease or increase the expression of a nucleic acid molecule encoding a protein and which comprise at least an 8-nucleobase portion which is complementary to a suitable target segment. The screening method comprises the steps of contacting a suitable target segment of a nucleic acid molecule encoding a protein with one or more candidate modulators, and selecting for one or more candidate modulators which decrease or increase the expression of a nucleic acid molecule encoding a protein. Once it is shown that the candidate modulator or modulators are capable of modulating (e.g. either decreasing or increasing) the expression of a nucleic acid molecule encoding a peptide, the modulator may then be employed in further investigative studies of the function of the peptide, or for use as a research, diagnostic, or therapeutic agent in accordance with the present invention.

The suitable target segments of the present disclosure may also be combined with their respective complementary antisense oligomeric compounds of the present disclosure to form stabilized double stranded (duplexed) oligonucleotides. Such double stranded oligonucleotide moieties have been shown in the art to modulate target expression and regulate translation as well as RNA processsing via an antisense mechanism. Moreover, the double stranded moieties may be subject to chemical modifications (Fire et al., Nature, 1998, 391, 806-811; Timmons and Fire, Nature 1998, 395, 854; Timmons et al., Gene, 2001, 263, 103-112; Tabara et al., Science, 1998, 282, 430-431; Montgomery et al., Proc. Natl. Acad. Sci. USA, 1998, 95, 15502-15507; Tuschl et al., Genes Dev., 1999, 13, 3191-3197; Elbashir et al., Nature, 2001, 411, 494-498; Elbashir et al., Genes Dev. 2001, 15, 188-200). For example, such double stranded moieties have been shown to inhibit the target by the classical hybridization of antisense strand of the duplex to the target, thereby triggering enzymatic degradation of the target (Tijsterman et al., Science, 2002, 295, 694-697). The oligomeric compounds of the present invention can also be applied in the areas of drug discovery and target validation. The present disclosure comprehends the use of the oligomeric compounds and targets identified herein in drug discovery efforts to elucidate relationships that exist between proteins and a disease state, phenotype, or condition. These methods include detecting or modulating a target peptide comprising contacting a sample, tissue, cell, or organism with the oligomeric compounds of the present invention, measuring the nucleic acid or protein level of the target and/or a related phenotypic or chemical endpoint at some time after treatment, and optionally comparing the measured value to a non-treated sample or sample treated with a further oligomeric compound of the invention. These methods can also be performed in parallel or in combination with other experiments to determine the function of unknown genes for the process of target validation or to determine the validity of a particular gene product as a target for treatment or prevention of a particular disease, condition, or phenotype.

Effect of nucleoside modifications on RNAi activity can be evaluated according to existing literature (Elbashir et al., Nature, 2001, 411, 494-498; Nishikura et al., Cell, 2001, 107, 415-416; and Bass et al., Cell, 2000, 101, 235-238.)

The oligomeric compounds of the present invention can be utilized for diagnostics, therapeutics, prophylaxis and as research reagents and kits. Furthermore, antisense oligonucleotides, which are able to inhibit gene expression with exquisite specificity, are often used by those of ordinary skill to elucidate the function of particular genes or to distinguish between functions of various members of a biological pathway. For use in kits and diagnostics, the oligomeric compounds of the present invention, either alone or in combination with other oligomeric compounds or therapeutics, can be used as tools in differential and/or combinatorial analyses to elucidate expression patterns of a portion or the entire complement of genes expressed within cells and tissues.

As one nonlimiting example, expression patterns within cells or tissues treated with one or more antisense oligomeric compounds are compared to control cells or tissues not treated with antisense oligomeric compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds and or oligomeric compounds which affect expression patterns.

Examples of methods of gene expression analysis known in the art include DNA arrays or microarrays (Brazma and Vilo, FEBS Lett., 2000, 480, 17-24; Celis, et al., FEBS Lett., 2000, 480, 2-16), SAGE (serial analysis of gene expression)(Madden, et al., Drug Discov. Today, 2000, 5, 415-425), READS (restriction enzyme amplification of digested cDNAs) (Prashar and Weissman, Methods Enzymol., 1999, 303, 258-72), TOGA (total gene expression analysis) (Sutcliffe, et al., Proc. Natl. Acad. Sci. U. S. A., 2000, 97, 1976-81), protein arrays and proteomics (Celis, et al., FEBS Lett., 2000, 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., Anal. Biochem., 2000, 286, 91-98; Larson, et al., Cytometry, 2000, 41, 203-208), subtractive cloning, differential display (DD) (Jurecic and Belmont, Curr. Opin. Microbiol., 2000, 3, 316-21), comparative genomic hybridization (Carulli, et al., J. Cell Biochem. Suppl., 1998, 31, 286-96), FISH (fluorescent in situ hybridization) techniques (Going and Gusterson, Eur. J. Cancer, 1999, 35, 1895-904) and mass spectrometry methods (To, Comb. Chem. High Throughput Screen, 2000, 3, 235-41).

The oligomeric compounds of the invention are useful for research and diagnostics, in one aspect because they hybridize to nucleic acids encoding proteins. For example, oligonucleotides that are shown to hybridize with such efficiency and under such conditions as disclosed herein as to be effective protein inhibitors will also be effective primers or probes under conditions favoring gene amplification or detection, respectively. These primers and probes are useful in methods requiring the specific detection of nucleic acid molecules encoding proteins and in the amplification of the nucleic acid molecules for detection or for use in further studies. Hybridization of the antisense oligonucleotides, particularly the primers and probes, of the invention with a nucleic acid can be detected by means known in the art. Such means may include conjugation of an enzyme to the oligonucleotide, radiolabelling of the oligonucleotide or any other suitable detection means. Kits using such detection means for detecting the level of selected proteins in a sample may also be prepared.

The specificity and sensitivity of antisense is also harnessed by those of skill in the art for therapeutic uses. Antisense oligomeric compounds have been employed as therapeutic moieties in the treatment of disease states in animals, including humans. Antisense oligonucleotide drugs, including ribozymes, have been safely and effectively administered to humans and numerous clinical trials are presently underway. It is thus established that antisense oligomeric compounds can be useful therapeutic modalities that can be configured to be useful in treatment regimes for the treatment of cells, tissues and animals, especially humans.

As used herein, the term "patient" refers to a mammal that is afflicted with one or more disorders associated with expression or overexpression of one or more genes. It will be understood that the most suitable patient is a human. It is also understood that this invention relates specifically to the inhibition of mammalian expression or overexpression of one or more genes.

It is recognized that one skilled in the art may affect the disorders associated with expression or overexpression of a gene by treating a patient presently afflicted with the disorders with an effective amount of one or more oligomeric compounds or compositions of the present invention. Thus, the terms "treatment" and "treating" are intended to refer to all processes wherein there may be a slowing, interrupting, arresting, controlling, or stopping of the progression of the disorders described herein, but does not necessarily indicate a total elimination of all symptoms.

As used herein, the term "effective amount" or "therapeutically effective amount" of a compound of the present invention refers to an amount that is effective in treating or preventing the disorders described herein.

For therapeutics, a patient, such as a human, suspected of having a disease or disorder which can be treated by modulating the expression of a gene is treated by administering antisense oligomeric compounds of the invention. The compounds of the invention can be utilized in pharmaceutical compositions by adding an effective amount of an antisense oligomeric compound to a suitable pharmaceutically acceptable diluent or carrier. Use of the antisense oligomeric compounds of the invention may also be useful prophylactically, e.g., to prevent or delay infection, inflammation or tumor formation, for example. In some instances, the patient being treated has been identified as being in need of treatment or has been previously diagnosed as such.

The oligomeric compounds of the invention encompass any pharmaceutically acceptable salts, esters, or salts of such esters, or any other compound which, upon administration to an animal including a human, is capable of providing (directly or indirectly) the biologically active metabolite or residue thereof. Accordingly, for example, the disclosure is also drawn to prodrugs and pharmaceutically acceptable salts of the compounds of the invention, pharmaceutically acceptable salts of such prodrugs, and other bioequivalents. For oligonucleotides, examples of pharmaceutically acceptable salts and their uses are further described in U.S. Patent 6,287,860.

The compositions of the invention may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative U.S. patents that teach the preparation of such uptake, distribution and/or absorption-assisting formulations include, but are not limited to, U.S.: 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

The present invention also includes pharmaceutical compositions and formulations which include the compositions of the invention. The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration. Oligonucleotides with at least one 2'-O-methoxyethyl modification are believed to be particularly useful for oral administration. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Pharmaceutical compositions of the present invention include, but are not limited to, solutions, emulsions, foams and liposome-containing formulations. The pharmaceutical compositions and formulations of the present invention may comprise one or more penetration enhancers, carriers, excipients or other active or inactive ingredients.

One of skill in the art will recognize that formulations are routinely designed according to their intended use, i.e. route of administration.

Suitable formulations for topical administration include those in which the oligonucleotides of the invention are in admixture with a topical delivery agent such as lipids, liposomes, fatty acids, fatty acid esters, steroids, chelating agents and surfactants. Suitable lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA). Penetration enhancers and their uses are further described in U.S. Patent 6,287,860. Surfactants and their uses are further described in U.S. Patent 6,287,860.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Suitable oral formulations are those in which oligonucleotides of the invention are administered in conjunction with one or more penetration enhancers surfactants and chelators. Suitable surfactants include fatty acids and/or esters or salts thereof, bile acids and/or salts thereof. Suitable bile acids/salts and fatty acids and their uses are further described in U.S. Patent 6,287,860. Also suitable are combinations of penetration enhancers, for example, fatty acids/salts in combination with bile acids/salts. A particularly suitable combination is the sodium salt of lauric acid, capric acid and UDCA. Further penetration enhancers include polyoxyethylene-9-lauryl ether, polyoxyethylene-20-cetyl ether. Oligonucleotides of the invention may be delivered orally, in granular form including sprayed dried particles, or complexed to form micro or nanoparticles. Oligonucleotide complexing agents and their uses are further described in U.S. Patent 6,287,860. Oral formulations for oligonucleotides and their preparation are described in detail in U.S. applications 09/108,673 (filed July 1, 1998), 09/315,298 (filed May 20, 1999) and 10/071,822, filed February 8, 2002.

In another related embodiment, therapeutically effective combination therapies may comprise the use of two or more compositions of the invention wherein the multiple compositions are targeted to a single or multiple nucleic acid targets. Numerous examples of antisense oligomeric compounds are known in the art. Two or more combined compounds may be used together or sequentially.

The formulation of therapeutic compositions and their subsequent administration is believed to be within the skill of those in the art. Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models. In general, dosage is from 0.01 µg to 100 g per kg of body weight, and may be given once or more daily, weekly, monthly or yearly. Persons of ordinary skill in the art can easily estimate repetition rates for dosing based on measured residence times and concentrations of the drug in bodily fluids or tissues. Following successful treatment, it may be desirable to have the patient undergo maintenance therapy to prevent the recurrence of the disease state, wherein the oligonucleotide is administered in maintenance doses, ranging from 0.01 µg to 100 g per kg of body weight, once or more daily, weekly, monthly, or yearly. For double-stranded compounds, the dose must be calculated to account for the increased nucleic acid load of the second strand (as with compounds comprising two separate strands) or the additional nucleic acid length (as with self complementary single strands having double-stranded regions).

While the present invention has been described with specificity in accordance with certain of its embodiments, the following examples serve only to illustrate the invention and are not intended to limit the same. Compounds not included within the scope of the appended claims are provided for information only.

### Examples

### General

The sequences listed in the examples have been annotated to indicate where there are modified nucleosides or internucleoside linkages. All non-annotated nucleosides are β-D-ribonucleosides linked by phosphodiester internucleoside linkages. Phosphorothioate internucleoside linkages are indicated by underlining. Modified nucleosides are indicated by a subscripted letter following the capital letter indicating the nucleoside. In particular, subscript "f" indicates 2'-fluoro; subscript "m" indicates 2'-O-methyl; subscript "I" indicates LNA; subscript "e" indicates 2'-O-methoxyethyl (MOE); subscript "p" indicates 2'-O-propyl; and subscript "t" indicates 4'-thio. For example Uₘ is a modified uridine having a 2'-OCH₃ group. A "d" preceding a nucleoside indicates a deoxynucleoside such as dT which is deoxythymidine, while "T" indicates a thymidine base and a ribo sugar. Some of the strands have a 5'-phosphate group designated as "P-". Bolded and italicized "***C***" indicates a 5-methyl C ribonucleoside. Where noted next to the ISIS number of a compound, "as" designates the antisense strand, and "s" designates the sense strand of the duplex, with respect to the target sequence.

The specific sequences here and throughout the speicification are provided only as examples. Thus, while certain motifs are exemplified using certain sequences, it is to be understood that other sequences could easily be prepared using the same motif of modifications.

### Example 1: Synthesis of Nucleoside Phosphoramidites

The preparation of nucleoside phosphoramidites is performed following procedures that are extensively illustrated in the art such as but not limited to US Patent 6,426,220 and published PCT WO 02/36743.

### Example 2: Oligonucleotide and oligonucleoside synthesis

The oligomeric compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides are synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation was effected by utilizing a 10% w/v solution of 3,H-1,2-benzodithiole-3-one 1,1-dioxide in acetonitrile for the oxidation of the phosphite linkages. The thiation reaction step time was increased to 180 sec and preceded by the normal capping step. After cleavage from the CPG column and deblocking in concentrated ammonium hydroxide at 55°C (12-16 hr), the oligonucleotides were recovered by precipitating with >3 volumes of ethanol from a 1 M NH₄OAc solution. Phosphinate oligonucleotides are prepared as described in U.S. Patent 5,508,270.

Alkyl phosphonate oligonucleotides are prepared as described in U.S. Patent 4,469,863.

3'-Deoxy-3'-methylene phosphonate oligonucleotides are prepared as described in U.S. Patents 5,610,289 or 5,625,050.

Phosphoramidite oligonucleotides are prepared as described in U.S. Patent, 5,256,775 or U.S. Patent 5,366,878.

Alkylphosphonothioate oligonucleotides are prepared as described in published PCT applications PCT/US94/00902 and PCT/US93/06976 (published as WO 94/17093 and WO 94/02499, respectively).

3'-Deoxy-3'-amino phosphoramidate oligonucleotides are prepared as described in U.S. Patent 5,476,925.

Phosphotriester oligonucleotides are prepared as described in U.S. Patent 5,023,243.

Borano phosphate oligonucleotides are prepared as described in U.S. Patents 5,130,302 and 5,177,198.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone oligomeric compounds having, for instance, alternating MMI and P=O or P=S linkages are prepared as described in U.S. Patents 5,378,825, 5,386,023, 5,489,677, 5,602,240 and 5,610,289.

Formacetal and thioformacetal linked oligonucleosides are prepared as described in U.S. Patents 5,264,562 and 5,264,564.

Ethylene oxide linked oligonucleosides are prepared as described in U.S. Patent 5,223,618.

### Example 3: Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAc with >3 volumes of ethanol. Synthesized oligonucleotides were analyzed by electrospray mass spectroscopy (molecular weight determination) and by capillary gel electrophoresis and judged to be at least 70% full length material. The relative amounts of phosphorothioate and phosphodiester linkages obtained in the synthesis was determined by the ratio of correct molecular weight relative to the -16 amu product (+/-32 +/-48). For some studies oligonucleotides were purified by HPLC, as described by Chiang et al., J. Biol. Chem. 1991, 266, 18162-18171. Results obtained with HPLC-purified material were similar to those obtained with non-HPLC purified material.

### Example 4: Oligonucleotide Synthesis - 96 Well Plate Format

Oligonucleotides can be synthesized via solid phase P(III) phosphoramidite chemistry on an automated synthesizer capable of assembling 96 sequences simultaneously in a 96-well format. Phosphodiester internucleotide linkages are afforded by oxidation with aqueous iodine. Phosphorothioate unternucleotide linkages are generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites are purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides are cleaved from support and deprotected with concentrated NH₄OH at elevated temperature (55-60°C) for 12-16 hours and the released product then dried *in vacuo.* The dried product is then re-suspended in sterile water to afford a master plate from which all analytical and test plate samples are then diluted utilizing robotic pipettors.

### Example 5: Oligonucleotide Analysis using 96-Well Plate Format

The concentration of oligonucleotide in each well is assessed by dilution of samples and UV absorption spectroscopy. The full-length integrity of the individual products is evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition is confirmed by mass analysis of the oligomeric compounds utilizing electrospray-mass spectroscopy. All assay test plates are diluted from the master plate using single and multi-channel robotic pipettors. Plates are judged to be acceptable if at least 85% of the oligomeric compounds on the plate are at least 85% full length.

### Example 6: Cell culture and oligonucleotide treatment

The effect of oligomeric compounds on target nucleic acid expression can be tested in any of a variety of cell types provided that the target nucleic acid is present at measurable levels. This can be routinely determined using, for example, PCR or Northern blot analysis. Cell lines derived from multiple tissues and species can be obtained from American Type Culture Collection (ATCC, Manassas, VA).

The following cell types are provided for illustrative purposes, but other cell types can be routinely used, provided that the target is expressed in the cell type chosen. This can be readily determined by methods routine in the art, for example Northern blot analysis, ribonuclease protection assays or RT-PCR.

T-24 cells: The human transitional cell bladder carcinoma cell line T-24 is obtained from the American Type Culture Collection (ATCC) (Manassas, VA). T-24 cells are routinely cultured in complete McCoy's 5A basal media (Invitrogen Corporation, Carlsbad, CA) supplemented with 10% fetal calf serum (Invitrogen Corporation, Carlsbad, CA), penicillin 100 units per mL, and streptomycin 100 micrograms per mL (Invitrogen Corporation, Carlsbad, CA). Cells are routinely passaged by trypsinization and dilution when they reached 90% confluence. Cells are seeded into 96-well plates (Falcon-Primaria #353872) at a density of 7000 cells/well for uses including but not limited to oligomeric compound transfection experiments.

A549 cells: The human lung carcinoma cell line A549 was obtained from the American Type Culture Collection (Manassas, VA). A549 cells were routinely cultured in DMEM, high glucose (Invitrogen Life Technologies, Carlsbad, CA) supplemented with 10% fetal bovine serum, 100 units per ml penicillin, and 100 micrograms per ml streptomycin (Invitrogen Life Technologies, Carlsbad, CA). Cells were routinely passaged by trypsinization and dilution when they reached approximately 90% confluence. Cells were seeded into 96-well plates (Falcon-Primaria #3872) at a density of approximately 5000 cells/well for uses including but not limited to oligomeric compound transfection experiments.

b.END cells: The mouse brain endothelial cell line b.END was obtained from Dr. Werner Risau at the Max Plank Institute (Bad Nauheim, Germany). b.END cells were routinely cultured in DMEM, high glucose (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #353872, BD Biosciences, Bedford, MA) at a density of approximately 3000 cells/well for uses including but not limited to oligomeric compound transfection experiments.

HeLa cells: The human epitheloid carcinoma cell line HeLa was obtained from the American Tissue Type Culture Collection (Manassas, VA). HeLa cells were routinely cultured in DMEM, high glucose (Invitrogen Corporation, Carlsbad, CA) supplemented with 10% fetal bovine serum (Invitrogen Corporation, Carlsbad, CA). Cells were routinely passaged by trypsinization and dilution when they reached 90% confluence. Cells were seeded into 24-well plates (Falcon-Primaria #3846) at a density of 50,000 cells/well or in 96-well plates at a density of 5,000 cells/well for uses including but not limited to oligomeric compound transfection experiments.

MH-S cells: The mouse alveolar macrophage cell line was obtained from American Type Culture Collection (Manassas, VA). MH-S cells were cultured in RPMI Medium 1640 with L-glutamine(Invitrogen Life Technologies, Carlsbad, CA), supplemented with 10% fetal bovine serum, 1 mM sodium pyruvate and 10mM HEPES (all supplements from Invitrogen Life Technologies, Carlsbad, CA). Cells were routinely passaged by trypsinization and dilution when they reached 70-80% confluence. Cells were seeded into 96-well plates (Falcon-Primaria #353047, BD Biosciences, Bedford, MA) at a density of 6500 cells/well for uses including but not limited to oligomeric compound transfection experiments.

U-87 MG: The human glioblastoma U-87 MG cell line was obtained from the American Type Culture Collection (Manassas, VA). U-87 MG cells were cultured in DMEM (Invitrogen Life Technologies, Carlsbad, CA) supplemented with 10% fetal bovine serum (Invitrogen Life Technologies, Carlsbad, CA) and antibiotics. Cells were routinely passaged by trypsinization and dilution when they reached appropriate confluence. Cells were seeded into 96-well plates (Falcon-Primaria #3872) at a density of about 10,000 cells/well for for uses including but not limited to oligomeric compound transfection experiments.

Experiments involving treatment of cells with oligomeric compounds:
When cells reach appropriate confluency, they are treated with oligomeric compounds using a transfection method as described.

### LIPOFECTIN™

When cells reached 65-75% confluency, they were treated with oligonucleotide. Oligonucleotide was mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture was replaced with fresh culture medium. Cells were harvested 16-24 hours after oligonucleotide treatment.

Other suitable transfection reagents known in the art include, but are not limited to, CYTOFECTIN™, LIPOFECTAMINE™, OLIGOFECTAMINE™, and FUGENE™. Other suitable transfection methods known in the art include, but are not limited to, electroporation.

The concentration of oligonucleotide used varies from cell line to cell line. To determine the optimal oligonucleotide concentration for a particular cell line, the cells are treated with a positive control oligonucleotide at a range of concentrations. For human cells the positive control oligonucleotide is selected from either ISIS 13920 (TₑCₑCₑGTCATCGCTCₑCₑTₑCₑAₑGₑGₑ, SEQ ID NO: 1) which is targeted to human H-ras, or ISIS 18078, (GₑTₑGₑCₑGₑCGCGAGCCCGₑAₑAₑAₑTₑCₑ, SEQ ID NO: 2) which is targeted to human Jun-N-terminal kinase-2 (JNK2). Both controls are 2'-O-methoxyethyl gapmers with a phosphorothioate backbone. For mouse or rat cells the positive control oligonucleotide is ISIS 15770 (AₑTₑGₑCₑAₑTCTGCCCCCAₑGₑGₑAₑ, SEQ ID NO: 3), a 2'-O-methoxyethyl gapmer with a phosphorothioate backbone which is targeted to both mouse and rat c-raf. The concentration of positive control oligonucleotide that results in 80% inhibition of c-H-ras (for ISIS 13920), JNK2 (for ISIS 18078) or c-raf (for ISIS 15770) mRNA is then utilized as the screening concentration for new oligonucleotides in subsequent experiments for that cell line. If 80% inhibition is not achieved, the lowest concentration of positive control oligonucleotide that results in 60% inhibition of c-H-ras, JNK2 or c-raf mRNA is then utilized as the oligonucleotide screening concentration in subsequent experiments for that cell line. If 60% inhibition is not achieved, that particular cell line is deemed as unsuitable for oligonucleotide transfection experiments.

### Example 7: Analysis of oligonucleotide inhibition of a target expression

Antisense modulation of a target expression can be assayed in a variety of ways known in the art. For example, a target mRNA levels can be quantitated by, e.g., Northern blot analysis, competitive polymerase chain reaction (PCR), or real-time PCR. Real-time quantitative PCR is presently desired. RNA analysis can be performed on total cellular RNA or poly(A)+ mRNA. One method of RNA analysis is the use of total cellular RNA as described in other examples herein. Methods of RNA isolation are well known in the art. Northern blot analysis is also routine in the art. Real-time quantitative (PCR) can be conveniently accomplished using the commercially available ABI PRISM™ 7600, 7700, or 7900 Sequence Detection System, available from PE-Applied Biosystems, Foster City, CA and used according to manufacturer's instructions.

Protein levels of a target can be quantitated in a variety of ways well known in the art, such as immunoprecipitation, Western blot analysis (immunoblotting), enzyme-linked immunosorbent assay (ELISA) or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F.M. et al., Current Protocols in Molecular Biology, Volume 2, pp. 11.2.1-11.2.22, John Wiley & Sons, Inc., 1991.

### Example 8: Design of phenotypic assays and in vivo studies for the use of target inhibitors

### Phenotypic assays

Once target inhibitors have been identified by the methods disclosed herein, the oligomeric compounds are further investigated in one or more phenotypic assays, each having measurable endpoints predictive of efficacy in the treatment of a particular disease state or condition.

Phenotypic assays, kits and reagents for their use are well known to those skilled in the art and are herein used to investigate the role and/or association of a target in health and disease. Representative phenotypic assays, which can be purchased from any one of several commercial vendors, include those for determining cell viability, cytotoxicity, proliferation or cell survival (Molecular Probes, Eugene, OR; PerkinElmer, Boston, MA), protein-based assays including enzymatic assays (Panvera, LLC, Madison, WI; BD Biosciences, Franklin Lakes, NJ; Oncogene Research Products, San Diego, CA), cell regulation, signal transduction, inflammation, oxidative processes and apoptosis (Assay Designs Inc., Ann Arbor, MI), triglyceride accumulation (Sigma-Aldrich, St. Louis, MO), angiogenesis assays, tube formation assays, cytokine and hormone assays and metabolic assays (Chemicon International Inc., Temecula, CA; Amersham Biosciences, Piscataway, NJ).

In one non-limiting example, cells determined to be appropriate for a particular phenotypic assay (i.e., MCF-7 cells selected for breast cancer studies; adipocytes for obesity studies) are treated with a target inhibitors identified from the *in vitro* studies as well as control compounds at optimal concentrations which are determined by the methods described above. At the end of the treatment period, treated and untreated cells are analyzed by one or more methods specific for the assay to determine phenotypic outcomes and endpoints.

Phenotypic endpoints include changes in cell morphology over time or treatment dose as well as changes in levels of cellular components such as proteins, lipids, nucleic acids, hormones, saccharides or metals. Measurements of cellular status which include pH, stage of the cell cycle, intake or excretion of biological indicators by the cell, are also endpoints of interest.

Measurement of the expression of one or more of the genes of the cell after treatment is also used as an indicator of the efficacy or potency of the a target inhibitors. Hallmark genes, or those genes suspected to be associated with a specific disease state, condition, or phenotype, are measured in both treated and untreated cells.

### In vivo studies

The individual subjects of the *in vivo* studies described herein are warm-blooded vertebrate animals, which includes humans.

A clinical trial is subjected to rigorous controls to ensure that individuals are not unnecessarily put at risk and that they are fully informed about their role in the study.

To account for the psychological effects of receiving treatments, volunteers are randomly given placebo or a target inhibitor. Furthermore, to prevent the doctors from being biased in treatments, they are not informed as to whether the medication they are administering is a a target inhibitor or a placebo. Using this randomization approach, each volunteer has the same chance of being given either the new treatment or the placebo.

Volunteers receive either the a target inhibitor or placebo for eight week period with biological parameters associated with the indicated disease state or condition being measured at the beginning (baseline measurements before any treatment), end (after the final treatment), and at regular intervals during the study period. Such measurements include the levels of nucleic acid molecules encoding a target or a target protein levels in body fluids, tissues or organs compared to pre-treatment levels. Other measurements include, but are not limited to, indices of the disease state or condition being treated, body weight, blood pressure, serum titers of pharmacologic indicators of disease or toxicity as well as ADME (absorption, distribution, metabolism and excretion) measurements.

Information recorded for each patient includes age (years), gender, height (cm), family history of disease state or condition (yes/no), motivation rating (some/moderate/great) and number and type of previous treatment regimens for the indicated disease or condition.

Volunteers taking part in this study are healthy adults (age 18 to 65 years) and roughly an equal number of males and females participate in the study. Volunteers with certain characteristics are equally distributed for placebo and a target inhibitor treatment. In general, the volunteers treated with placebo have little or no response to treatment, whereas the volunteers treated with the target inhibitor show positive trends in their disease state or condition index at the conclusion of the study.

### Example 9 : RNA Isolation

### Poly(A)+ mRNA isolation

Poly(A)+ mRNA is isolated according to Miura et al., (Clin. Chem., 1996, 42, 1758-1764). Other methods for poly(A)+ mRNA isolation are routine in the art. Briefly, for cells grown on 96-well plates, growth medium was removed from the cells and each well was washed with 200 µL cold PBS. 60 µL lysis buffer (10 mM Tris-HCl, pH 7.6, 1 mM EDTA, 0.5 M NaCl, 0.5% NP-40, 20 mM vanadyl-ribonucleoside complex) was added to each well, the plate was gently agitated and then incubated at room temperature for five minutes. 55 µL of lysate was transferred to Oligo d(T) coated 96-well plates (AGCT Inc., Irvine CA). Plates were incubated for 60 minutes at room temperature, washed 3 times with 200 µL of wash buffer (10 mM Tris-HCl pH 7.6, 1 mM EDTA, 0.3 M NaCl). After the final wash, the plate was blotted on paper towels to remove excess wash buffer and then air-dried for 5 minutes. 60 µL of elution buffer (5 mM Tris-HCl pH 7.6), preheated to 70°C, was added to each well, the plate was incubated on a 90°C hot plate for 5 minutes, and the eluate was then transferred to a fresh 96-well plate.

Cells grown on 100 mm or other standard plates may be treated similarly, using appropriate volumes of all solutions.

### Total RNA Isolation

Total RNA is isolated using an RNEASY 96™ kit and buffers purchased from Qiagen Inc. (Valencia, CA) following the manufacturer's recommended procedures. Briefly, for cells grown on 96-well plates, growth medium is removed from the cells and each well is washed with 200 µL cold PBS. 150 µL Buffer RLT is added to each well and the plate vigorously agitated for 20 seconds. 150 µL of 70% ethanol is then added to each well and the contents mixed by pipetting three times up and down. The samples are then transferred to the RNEASY 96™ well plate attached to a QIAVAC™ manifold fitted with a waste collection tray and attached to a vacuum source. Vacuum is applied for 1 minute. 500 µL of Buffer RW1 is added to each well of the RNEASY 96™ plate and incubated for 15 minutes and the vacuum is again applied for 1 minute. An additional 500 µL of Buffer RW1 is added to each well of the RNEASY 96™ plate and the vacuum is applied for 2 minutes. 1 mL of Buffer RPE is then added to each well of the RNEASY 96™ plate and the vacuum applied for a period of 90 seconds. The Buffer RPE wash is then repeated and the vacuum is applied for an additional 3 minutes. The plate is then removed from the QIAVAC™ manifold and blotted dry on paper towels. The plate is then reattached to the QIAVAC™ manifold fitted with a collection tube rack containing 1.2 mL collection tubes. RNA is then eluted by pipetting 140 µL of RNAse free water into each well, incubating 1 minute, and then applying the vacuum for 3 minutes.

The repetitive pipetting and elution steps may be automated using a QIAGEN Bio-Robot 9604 (Qiagen, Inc., Valencia CA). Essentially, after lysing of the cells on the culture plate, the plate is transferred to the robot deck where the pipetting, DNase treatment and elution steps are carried out.

### Example 10: Design and screening of duplexed antisense compounds

In accordance with the present invention, a series of nucleic acid duplexes comprising the compounds of the present invention and their complements can be designed. The nucleobase sequence of the antisense strand of the duplex comprises at least a portion of an antisense oligonucleotide targeted to a target sequence as described herein. The ends of the strands may be modified by the addition of one or more natural or modified nucleobases to form an overhang. The sense strand of the dsRNA is then designed and synthesized as the complement of the antisense strand and may also contain modifications or additions to either terminus. For example, in one embodiment, both strands of the dsRNA duplex would be complementary over the central nucleobases, each having overhangs at one or both termini.

For example, a duplex comprising an antisense strand having the sequence CGAGAGGCGGACGGGACCG (SEQ ID NO: 20) and having a two-nucleobase overhang of deoxythymidine(dT) would have the following structure: In another embodiment, a duplex comprising an antisense strand having the same sequence CGAGAGGCGGACGGGACCG (SEQ ID NO: 20) may be prepared with blunt ends (no single stranded overhang) as shown:

RNA strands of the duplex can be synthesized by methods disclosed herein or purchased from Dharmacon Research Inc., (Lafayette, CO). Once synthesized, the complementary strands are annealed. The single strands are aliquoted and diluted to a concentration of 50 µM. Once diluted, 30 µL of each strand is combined with 15µL of a 5X solution of annealing buffer. The final concentration of the buffer is 100 mM potassium acetate, 30 mM HEPES-KOH pH 7.4, and 2mM magnesium acetate. The final volume is 75 µL. This solution is incubated for 1 minute at 90°C and then centrifuged for 15 seconds. The tube is allowed to sit for 1 hour at 37°C at which time the dsRNA duplexes are used in experimentation. The final concentration of the dsRNA duplex is 20 µM.

Once prepared, the duplexed compounds are evaluated for their ability to modulate target mRNA levels When cells reach 80% confluency, they are treated with duplexed compounds of the invention. For cells grown in 96-well plates, wells are washed once with 200 µL OPTI-MEM-1^{™} reduced-serum medium (Gibco BRL) and then treated with 130 µL of OPTI-MEM-1^{™} containing 5 µg/mL LIPOFECTAMINE 2000™ (Invitrogen Life Technologies, Carlsbad, CA) and the duplex antisense compound at the desired final concentration. After about 4 hours of treatment, the medium is replaced with fresh medium. Cells are harvested 16 hours after treatment, at which time RNA is isolated and target reduction measured by quantitative real-time PCR as described herein.

### Example 11: Real-time Quantitative PCR Analysis of target mRNA Levels

Quantitation of a target mRNA levels was accomplished by real-time quantitative PCR using the ABI PRISM™ 7600, 7700, or 7900 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time. As opposed to standard PCR in which amplification products are quantitated after the PCR is completed, products in real-time quantitative PCR are quantitated as they accumulate. This is accomplished by including in the PCR reaction an oligonucleotide probe that anneals specifically between the forward and reverse PCR primers, and contains two fluorescent dyes. A reporter dye (e.g., FAM or JOE, obtained from either PE-Applied Biosystems, Foster City, CA, Operon Technologies Inc., Alameda, CA or Integrated DNA Technologies Inc., Coralville, IA) is attached to the 5' end of the probe and a quencher dye (e.g., TAMRA, obtained from either PE-Applied Biosystems, Foster City, CA, Operon Technologies Inc., Alameda, CA or Integrated DNA Technologies Inc., Coralville, IA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated. With each cycle, additional reporter dye molecules are cleaved from their respective probes, and the fluorescence intensity is monitored at regular intervals by laser optics built into the ABI PRISM™ Sequence Detection System. In each assay, a series of parallel reactions containing serial dilutions of mRNA from untreated control samples generates a standard curve that is used to quantitate the percent inhibition after antisense oligonucleotide treatment of test samples.

Prior to quantitative PCR analysis, primer-probe sets specific to the target gene being measured are evaluated for their ability to be "multiplexed" with a GAPDH amplification reaction. In multiplexing, both the target gene and the internal standard gene GAPDH are amplified concurrently in a single sample. In this analysis, mRNA isolated from untreated cells is serially diluted. Each dilution is amplified in the presence of primer-probe sets specific for GAPDH only, target gene only ("single-plexing"), or both (multiplexing). Following PCR amplification, standard curves of GAPDH and target mRNA signal as a function of dilution are generated from both the single-plexed and multiplexed samples. If both the slope and correlation coefficient of the GAPDH and target signals generated from the multiplexed samples fall within 10% of their corresponding values generated from the single-plexed samples, the primer-probe set specific for that target is deemed multiplexable. Other methods of PCR are also known in the art.

RT and PCR reagents were obtained from Invitrogen Life Technologies (Carlsbad, CA). RT, real-time PCR was carried out by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR are normalized using either the expression level of GAPDH, a gene whose expression is constant, or by quantifying total RNA using RIBOGREEN^{™} (Molecular Probes, Inc. Eugene, OR). GAPDH expression is quantified by real time RT-PCR, by being run simultaneously with the target, multiplexing, or separately. Total RNA is quantified using RiboGreen^{™} RNA quantification reagent (Molecular Probes, Inc. Eugene, OR). Methods of RNA quantification by RIBOGREEN^{™} are taught in Jones, L.J., et al, (Analytical Biochemistry, 1998, 265, 368-374).

In this assay, 170 µL of RIBOGREEN^{™} working reagent (RIBOGREEN^{™} reagent diluted 1:350 in 10mM Tris-HCl, 1 mM EDTA, pH 7.5) is pipetted into a 96-well plate containing 30 µL purified, cellular RNA. The plate is read in a CytoFluor 4000 (PE Applied Biosystems) with excitation at 485nm and emission at 530nm.

### Example 12: Target-specific primers and probes

Probes and primers may be designed to hybridize to a target sequence, using published sequence information.

For example, for human PTEN, the following primer-probe set was designed using published sequence information (GENBANK™ accession number U92436.1, SEQ ID NO: 4).
Forward primer: AATGGCTAAGTGAAGATGACAATCAT (SEQ ID NO: 5)
Reverse primer: TGCACATATCATTACACCAGTTCGT (SEQ ID NO: 6)
And the PCR probe:
FAM-TTGCAGCAATTCACTGTAAAGCTGGAAAGG-TAMRA (SEQ ID NO: 7), where FAM is the fluorescent dye and TAMRA is the quencher dye.
For example, for human survivin, the following primer-probe set was designed using published sequence information (GENBANK™ accession number NM_001168.1, SEQ ID NO: 8).
Forward primer: CACCACTTCCAGGGTTTATTCC (SEQ ID NO: 9)
Reverse primer: TGATCTCCTTTCCTAAGACATTGCT (SEQ ID NO: 10)
And the PCR probe:
FAM-ACCAGCCTTCCTGTGGGCCCCT-TAMRA (SEQ ID NO: 11),
where FAM is the fluorescent dye and TAMRA is the quencher dye.
For example, for human eIF4E, the following primer-probe set was designed using published sequence information (GENBANK™ accession number M15353.1, SEQ ID NO: 12).
Forward primer: TGGCGACTGTCGAACCG (SEQ ID NO: 13)
Reverse primer: AGATTCCGTTTTCTCCTCTTCTGTAG (SEQ ID NO: 14)
And the PCR probe:
FAM-AAACCACCCCTACTCCTAATCCCCCG-TAMRA (SEQ ID NO: 15), where FAM is the fluorescent dye and TAMRA is the quencher dye.
For example, for mouse eIF4E, the following primer-probe set was designed using published sequence information (GENBANK™ accession number NM_007917.2, SEQ ID NO: 16).
Forward primer: AGGACGGTGGCTGATCACA (SEQ ID NO: 17)
Reverse primer: TCTCTAGCCAGAAGCGATCGA (SEQ ID NO: 18)
And the PCR probe:
FAM-TGAACAAGCAGCAGAGACGGAGTGA-TAMRA (SEQ ID NO: 19),
where FAM is the fluorescent dye and TAMRA is the quencher dye.

### Example 13: Northern blot analysis of a target mRNA levels

Eighteen hours after antisense treatment, cell monolayers were washed twice with cold PBS and lysed in 1 mL RNAZOL™ (TEL-TEST "B" Inc., Friendswood, TX). Total RNA was prepared following manufacturer's recommended protocols. Twenty micrograms of total RNA was fractionated by electrophoresis through 1.2% agarose gels containing 1.1% formaldehyde using a MOPS buffer system (AMRESCO, Inc. Solon, OH). RNA was transferred from the gel to HYBOND™-N+ nylon membranes (Amersham Pharmacia Biotech, Piscataway, NJ) by overnight capillary transfer using a Northern/Southern Transfer buffer system (TEL-TEST "B" Inc., Friendswood, TX). RNA transfer was confirmed by UV visualization. Membranes were fixed by UV cross-linking using a STRATALINKER™ UV Crosslinker 2400 (Stratagene, Inc, La Jolla, CA) and then probed using QUICKHYB™ hybridization solution (Stratagene, La Jolla, CA) using manufacturer's recommendations for stringent conditions.

To detect human a target, a human a target specific primer probe set is prepared by PCR. To normalize for variations in loading and transfer efficiency membranes are stripped and probed for human glyceraldehyde-3-phosphate dehydrogenase (GAPDH) RNA (Clontech, Palo Alto, CA).

Hybridized membranes were visualized and quantitated using a PHOSPHORIMAGER™ and IMAGEQUANT™ Software V3.3 (Molecular Dynamics, Sunnyvale, CA). Data was normalized to GAPDH levels in untreated controls.

### Example 14: Western blot analysis of target protein levels

Western blot analysis (immunoblot analysis) is carried out using standard methods. Cells are harvested 16-20 h after oligonucleotide treatment, washed once with PBS, suspended in Laemmli buffer (100 µl/well), boiled for 5 minutes and loaded on a 16% SDS-PAGE gel. Gels are run for 1.5 hours at 150 V, and transferred to membrane for western blotting. Appropriate primary antibody directed to a target is used, with a radiolabeled or fluorescently labeled secondary antibody directed against the primary antibody species. Bands are visualized using a PHOSPHORIMAGER™ (Molecular Dynamics, Sunnyvale CA).

### Example 15: In vitro assay of selected differentially modified siRNAs

Differentially modified siRNA duplexes designed to target human survivin using published sequence information were prepared and assayed as described below. The antisense strand was held constant as a 4'-thio gapped strand and 3 different sense strands were compared. The nucleosides are annotated as to chemical modification as per the legend at the beginning of the examples.

| **SEQ ID NO. /ISIS NO.** | **Composition (5' 3')** | **Features** |
|---|---|---|
| 24/353537 (as) | UₜUₜUₜGAAAAUGUUGAUCUₜCₜCₜ | 4'-S wings (3/13/3) |
| 25/352512 (s) | | 2'-OCH₃ full |
| 25/352513 (s) | | 2'-OCH₃ block (1/17/1) |
| 25/352514 (s) | | MOE alternating |

The differentially modified siRNA duplexes were assayed for their ability to inhibit target mRNA levels in HeLa cells. Culture methods used for HeLa cells are available from the ATCC and may be found, for example, at www (dot)atcc.org. For cells grown in 96-well plates, wells were washed once with 200 µL OPTI-MEM-1 reduced-serum medium and then treated with 130 µL of OPTI-MEM-1 containing 12 µg/mL LIPOFECTIN™ (Invitrogen Life Technologies, Carlsbad, CA) and the dsRNA at the desired concentrations. After about 5 hours of treatment, the medium was replaced with fresh medium. Cells were harvested 16 hours after treatment, at which time RNA was isolated and target reduction measured by RT-PCR as previously described. Dose-response data was used to determine the IC50 for each pair noted below (antisense:sense).

| **Construct** | **Assay/Species** | **Target IC50 (nM)** |
|---|---|---|
| 353537:352512 | Dose Response/Human | Survivin 0.60192 |
| 353537:352513 | Dose Response/Human | Survivin 0.71193 |
| 353537:352514 | Dose Response/Human | Survivin 0.48819. |

### Example 16: In vitro assay of differentially modified siRNAs having MOE modified sense and 4'-thio(4'-thio/2'-OCH₃) gapmer antisense strands

In accordance with the present invention, a series of oligomeric compounds were synthesized and tested for their ability to reduce target expression over a range of doses relative to an unmodified compound. The compounds tested were 19 nucleotides in length having phosphorothioate internucleoside linkages throughout.

HeLa cells were treated with the double stranded oligomeric compounds (siRNA constructs) shown below (antisense strand followed by the sense strand of the duplex) at concentrations of 0, 0.15, 1.5, 15, and 150 nM using methods described herein. The nucleosides are annotated as to chemical modification as per the legend at the beginning of the examples. Expression levels of human PTEN were determined by quantitative real-time PCR and normalized to RIBOGREEN™ as described in other examples herein. Resulting dose-response curves were used to determine the IC50 for each pair. Also shown is the effect of each duplex on target mRNA levels as a percentage of untreated control (%UTC).

| **SEQ ID NO. /ISIS NO.** | **Composition (5' to 3')** | **IC50** | **%UTC** |
|---|---|---|---|
| 26/359458 (as) | UUGUCUCUGGUCCUUACUU | 0.94 | 13 |
| 27/359467 (s) | AAGUAAGGACCAGAGACAA | | |
| 26/359458 (as) | UUGUCUCUGGUCCUUACUU | .055 | 13 |
| 27/359351 (s) | AₑAₑGₑUAAGGACCAGAGACₑAₑAₑ | | |
| 26/359347 (as) | UₜUₜGUCUCUGGUCCUUACUₜUₜ | 2.2 | 25 |
| 27/359551 (s) | AₑAₑGₑUAAGGACCAGAGACₑAₑAₑ | | |
| 26/359346 (as) | UₜUₜGUCUCUGGUCCUUACₘUₘUₘ | 0.18 | 11 |
| 27/359351 (s) | AₑAₑGUAAGGACCAGAGACₑAₑAₑ | | |
| 26/359345 (as) | UₜUₜGUCUCUGGUCCUUACUₜUₜ | 5.3 | 18 |
| 27/359467 (s) | AAGUAAGGACCAGAGACAA | | |
| 26/359346 (as) | UₜUₜGUCUCUGGUCCUUACₘUₘUₘ | 0.73 | 15 |
| 27/359467 (s) | AAGUAAGGACCAGAGACAA | | |
| 26/359345 (as) | UₜUₜGUCUCUGGUCCUUACUₜUₜ | 0.49 | 14 |
| 27/359467 (s) | AAₑGUₑAAₑGGₑACCAₑGAₑGAₑCAₑA | | |
| 26/359345 (as) | UₜUₜGUCUCUGGUCCUUACUₜUₜ | 0.55 | 15 |
| 27/359351 (s) | AₑAₑGₑUAAGGACCAGAGACₑAₑAₑ | | |

From these data it is evident that the activity of the double strand construct containing the 4'-thio gapmer RNA in the antisense strand paired with an RNA sense strand (359345_341401 having an IC50 of 5.3) can be improved by incorporating 2'MOE modifications into the sense strand on the terminal ends or in an alternating configuration with RNA. It is also evident that improvements in IC50 values can be obtained over the unmodified pure RNA construct (341391_341401; RNA in both strands with an IC50 value of 0.94) by using an alternating motif.

### Example 17: In vitro assay of selected differentially modified siRNAs

Selected siRNAs (shown below as antisense strand followed by the sense strand of the duplex) were prepared and evaluated in HeLa cells treated as described herein with varying doses of the selected siRNAs. The mRNA levels were quantitated using real-time PCR as described herein and were compared to untreated control levels (%UTC). The IC50's were calculated using the linear regression equation generated by plotting the normalized mRNA levels to the log of the concentrations used.

| **SEQ ID NO. /ISIS NO.** | **Composition (5' to 3')** | **IC50** | **%UTC** |
|---|---|---|---|
| 26/359346 (as) | UₜUₜGUCUCUGGUCCUUACₘUₘUₘ | 1.9 | 10 |
| 27/367287 (s) | AAGUₜAAGGACₜCₜAGAGACₜAA | | |
| 26/359345 (as) | UₜUₜGUCUCUGGUCCUUACUₜUₜ | 1.7 | 20 |
| 27/367287 (s) | AAGUₜAAGGACₜCₜAGAGACₜAA | | |
| 26/359345 (as) | UₜUₜGUCUCUGGUCCUUACUₜUₜ | 0.2 | 10 |
| 27/367288 (s) | AₜAₜGUAAGGACCAGAGACAₜAₜ | | |
| 26/359346 (as) | UₜUₜGUCUCUGGUCCUUACₘUₘUₘ | <0.1 | 10 |
| 27/367288 (s) | AₜAₜGUAAGGACCAGAGACAₜAₜ | | |
| 26/359345 (as) | UₜUₜGUCUCUGGUCCUUACUₜUₜ | 0.5 | 15 |
| 27/359351 (s) | AₑAₑGₑUAAGGACCAGAGACₑAₑAₑ | | |
| 26/359346 (as) | UₜUₜGUCUCUGGUCCUUACₘUₘUₘ | 0.2 | 11 |
| 27/359351 (s) | AₑAₑGₑUAAGGACCAGAGACₑAₑAₑ | | |
| 26/359995 (as) | | 0.4 | 17 |
| 27/359351 (s) | AₑAₑGₑUAAGGACCAGAGACₑAₑAₑ | | |
| 26/359345 (as) | UₜUₜGUCUCUGGUCCUUACUₜUₜ | 0.2 | 13 |
| 27/359996 (s) | | | |
| 26/359346 (as) | UₜUₜGUCUCUGGUCCUUACₘUₘUₘ | 0.2 | 13 |
| 27/359996 (s) | | | |
| 26/361203 (as) | UUGₘUCUCUₘGGUCCₘUUACUₘU | <0.1 | -- |
| 27/359351 (s) | AₑAeGₑUAAGGACCAGAGACₑAₑAₑ | | |
| 26/361209 (as) | UUGUₘCUCUGₘGUCCUₘUACUUₘ | 1.5 | -- |
| 27/359351 (s) | AₑAeGₑUAAGGACCAGAGACₑAₑAₑ | | |
| 26/361204 (as) | UUGUₑCUCUGGₑUCCUUACUₑU | 1.5 | -- |
| 27/359351 (s) | AₑAₑGₑUAAGGACCAGAGACₑAₑAₑ | | |
| 26/361205 (as) | UUGUCₑUCUGGUCₑCUUACₑUₑUₑ | 2.5 | -- |
| 27/359351 (s) | AₑAₑGₑUAAGGACCAGAGACₑAₑAₑ | | |
| 26/361206 (as) | UUGUCₑUₑCUGGUₑCₑCUUACUₑUₑ | -- | -- |
| 27/359351 (s) | AₑAₑGₑUAAGGACCAGAGACₑAₑAₑ | | |
| 26/361207 (as) | UUGUCUₑCₑUGGₑUₑCCUUACₑUₑUₑ | 10.1 | -- |
| 27/359351 (s) | AₑAₑGₑUAAGGACCAGAGACₑAₑAₑ | | |
| 26/341391 (as) | UUGUCUCUGGUCCUUACUU | 0.1 | -- |
| 27/341401 (s) | AAGUAAGGACCAGAGACAA | | |
| 26/359979 (as) | UUGUCₘUCUₘGGUₘCCUₘUACₘUₘUₘ | -- | -- |
| 27/359351 (s) | AₑAₑGₑUAAGGACCAGAGACₑAₑAₑ | | |
| 26/359980 (as) | UUGUCUₘCₘUGGₘUₘCCUUACₘUₘUₘ | 0.2 | -- |
| 27/359351 (s) | AₑAₑGₑUAAGGACCAGAGACₑAₑAₑ | | |
| 26/359980 (as) | UUGUCUₘCₘUGGₘUₘCCUUACₘUₘUₘ | 0.1 | -- |
| 27/361221 (s) | AₘAₘGₘUAAGGACCAGAGACₘAₘAₘ | | |

### Example 18: In vitro assay of modified siRNAs targeted to human survivin

In accordance with the present invention, a series of oligomeric compounds were synthesized and tested for their ability to reduce survivin expression over a range of doses. HeLa cells were treated with the double stranded oligomeric compounds (siRNA constructs) shown below (antisense strand followed by the sense strand of the duplex) at concentrations of 0.0006 nM, 0.084 nM, 0.16 nM, 0.8 nM, 4 nM, or 20 nM using methods described herein. The nucleosides are annotated as to chemical modification as per the legend at the beginning of the examples. Expression levels of human survivin were determined using real-time PCR methods as described herein. The effect of the 20 nM dose on survivin mRNA levels is shown below. Results are presented as a percentage of untreated control mRNA levels.

| **SEQ ID NO. /ISIS NO.** | **Composition (5' to 3')** | **%UTC** |
|---|---|---|
| 24/343867 (as) | UUUGAAAAUGUUGAUCUCC | 3 |
| 25/343868 (s) | GGAGAUCAACAUUUUCAAA | |
| 24/352506 (as) | UUUGAAₘAₘAUGₘUₘUGAUCUₘCₘCₘ | 2 |
| 25/371314 (s) | GₑGₑAₑGₑAₑUCAACAUUUUₑCₑAₑAₑAₑ | |
| 24/352506 (as) | UUUGAAₘAₘAUGₘUₘUGAUCUₘCₘCₘ | 3 |
| 25/371316 (s) | GₘGₘAₘGAUCAACAUUUUCAₘAₘAₘ | |
| 24/352506 (as) | UUUGAAₘAₘAUGₘUₘUGAUCUₘCₘCₘ | 2 |
| 25/371313 (s) | GₑGₑAₑGAUCAACAUUUUCAₑAₑAₑ | |
| 24/353537 (as) | UₜUₜUₜGAAAAUGUUGAUCUₜCₜCₜ | 5 |
| 25/371313 (s) | GₑGₑAₑGAUCAACAUUUUCAₑAₑAₑ | |
| 24/353537 (as) | UₜUₜUtGAAAAUGUUGAUCUₜCₜCₜ | 5 |
| 25/352514 (s) | GGₑAGₑAUₑCAₑACₑAUₑUUₑUCₑAAₑA | |
| 24/353537 (as) | UₜUₜUₜGAAAAUGUUGAUCUₜCₜCₜ | 6 |
| 25/371314 (s) | GₑGₑAₑGₑAₑUCAACAUUUUₑCₑAₑAₑAₑ | |
| 24/353537 (as) | UₜUₜUₜGAAAAUGUUGAUCUₜCₜCₜ | 5 |
| 25/371315 (s) | GₑGₑAₑGAUCAACₑAₑUUUUCAₑAₑAₑ | |
| 24/353537 (as) | UₜUₜUₜGAAAAUGUUGAUCUₜCₜCₜ | 5 |
| 25/371316 (s) | GₘGₘAₘGAUCAACAUUUUCAₘAₘAₘ | |
| 24/353540 (as) | UₘUₘUₘGAAAAUGUUGAUCUₜCₜCₜ | 3 |
| 25/371313 (s) | GₑGₑAₑGAUCAACAUUUUCAₑAₑAₑ | |
| 24/353540 (as) | UₘUₘUₘGAAAAUGUUGAUCUₜCₜCₜ | 2 |
| 25/352514 (s) | GGₑAG_{c}AUₑCAₑACₑAUₑUUₑUCₑAAₑA | |
| 24/353540 (as) | UₘUₘUₘGAAAAUGUUGAUCUₜCₜCₜ | 3 |
| 25/371314 (s) | GₑGₑAₑGₑAₑUCAACAUUUUₑCₑAₑAₑAₑ | |
| 24/353540 (as) | UₘUₘUₘGAAAAUGUUGAUCUₜCₜCₜ | 3 |
| 25/371315 (s) | GₑGₑAₑGAUCAACₑAₑUUUUCAₑAₑAₑ | |
| 24/353540 (as) | UₘUₘUₘGAAAAUGUUGAUCUₜCₜCₜ | 3 |
| 25/371316 (s) | GₘGₘAₘGAUCAACAUUUUCAₘAₘAₘ | |
| 24/368679 (as) | UₘU_{f}UₘG_{f}AₘA_{f}AₘA_{f}UₘG_{f}UₘU_{f}GₘA_{f}UₘC_{f}UₘC_{f}Cₘ | 2 |
| 25/371313 (s) | GₑGₑAₑGAUCAACAUUUUCAₑAₑAₑ | |
| 24/368679 (as) | UₘU_{f}UₘG_{f}AₘA_{f}AₘA_{f}UₘG_{f}UₘU_{f}GₘA_{f}UₘC_{f}UₘC_{f}Cₘ | 3 |
| 25/371314 (s) | GₑGₑAₑGₑAₑUCAACAUUUUₑCₑAₑAₑAₑ | |
| 24/368679 (as) | UₘU_{f}UₘG_{f}AₘA_{f}AₘA_{f}UₘG_{f}UmU_{f}GmA_{f}UₘC_{f}UₘC_{f}Cₘ | 3 |
| 25/371316 (s) | GₘGₘAₘGAUCAACAUUUUCAₘAₘAₘ | |
| 24/352506 (as) | UUUGAAₘAₘAUGₘUₘUGAUCUₘCₘCₘ | 12 |
| 25/352514 (s) | GGₑAGₑAUₑCAₑACₑAUₑUUₑUCₑAAₑA | |
| 24/368679 (as) | UₘUfUₘG_{f}AₘA_{f}AₘA_{f}UₘG_{f}UₘU_{f}GₘA_{f}UₘC_{f}UₘC_{f}Cₘ | 8 |
| 25/371315 (s) | GₑGₑAₑGAUCAACₑAₑUUUUCAₑAₑAₑ | |

### Example 19: In vitro assay of selected differentially modified siRNAs targeted to eIF4E

In accordance with the present invention, a series of oligomeric compounds were synthesized and tested for their ability to reduce eIF4E expression over a range of doses. The nucleosides are annotated as to chemical modification as per the legend at the beginning of the examples. HeLa cells were treated with the double stranded oligomeric compounds (siRNA constructs) shown below (antisense strand followed by the sense strand to which it was duplexed) at concentrations of 0.0006 nM, 0.032 nM, 0.16 nM, 0.8 nM, 4 nM, or 20 nM using methods described herein. Expression levels of human eIF4E were determined using real-time PCR methods as described herein. Resulting dose-response curves were used to determine the IC50 for each pair as shown below.

| **SEQ ID NO. /ISIS NO.** | **Composition (5' to 3')** | **IC50** |
|---|---|---|
| 30/371286 (as) | UUUAGCUCUAACAUUAACA | 0.440 |
| 31/371280 (s) | UGUUAAUGUUAGAGCUAAA | |
| 30/371287 (as) | UUUAGCₘUₘCUAₘAₘCAUUAAₘCₘAₘ | 0.356 |
| 31/371280 (s) | UGUUAAUGUUAGAGCUAAA | |
| 30/371287 (as) | UUUAGCₘUₘCUAₘAₘCAUUAAₘCₘAₘ | 2.520 |
| 31/371284 (s) | UₑGₑUₑUAAUGUUAGAGCUAₑAₑAₑ | |
| 32/371297 (as) | UUACUAGACAACUGGAUAU | 0.381 |
| 33/371291 (s) | AUAUCCAGUUGUCUAGUAA | |
| 32/371298 (as) | UUACUAₘGₘACAₘAₘCUGGAUₘAₘUₘ | 0.260 |
| 33/371291 (s) | AUAUCCAGUUGUCUAGUAA | |
| 32/371298 (as) | UUACUAₘGₘACAₘAₘCUGGAUₘAₘUₘ | 0.260 |
| 33/371295 (s) | AₑUₑAₑUCCAGUUGUCUAGUₑAₑAₑ | |
| 32/379960 (as) | UₘU_{f}AₘC_{f}UₘA_{f}GmA_{f}CₘA_{f}AₘC_{f}UₘG_{f}GₘA_{f}UₘA_{f}Uₘ | 0.260 |
| 33/371295 (s) | AₑUₑAₑUCCAGUUGUCUAGUₑAₑAₑ | |
| 34/371308 (as) | UUAAAAAGUGAGUAGUCAC | 0.126 |
| 35/371302 (s) | GUGACUACUCACUUUUUAA | |
| 34/371309 (as) | UUAAAAₘAₘGUGₘAₘGUAGUCₘAₘCₘ | 0.168 |
| 35/371302 (s) | GUGACUACUCACUUUUUAA | |
| 34/371309 (as) | UUAAAAₘAₘGUGmAₘGUAGUCₘAₘCₘ | 0.040 |
| 35/371306 (s) | GₑUₑGₑACUACUCACUUUUUₑAₑAₑ | |
| 34/371309 (as) | UUAAAAₘAₘGUGₘAₘGUAGUCₘAₘCₘ | 0.017 |
| 35/379964 (s) | GₘU_{f}GₘA_{f}CₘU_{f}AₘC_{f}UₘC_{f}AₘC_{f}UₘU_{f}UₘU_{f}UₘA_{f}Aₘ. | |

### Example 20: In vitro assay of selected differentially modified siRNAs targeted to eIF4E

In accordance with the present invention, a series of oligomeric compounds were synthesized and tested for their ability to reduce eIF4E expression over a range of doses. The nucleosides are annotated as to chemical modification as per the legend at the beginning of the examples. HeLa cells were treated with the double stranded oligomeric compounds (siRNA constructs) shown below at concentrations of 0.008 nM, 0.04 nM, 0.2 nM, 0.8 nM, 1.0 nM, or 5.0 nM using methods described herein. Expression levels of human eIF4E were determined using real-time PCR methods as described herein. Resulting dose-response curves were used to determine the IC50 for each pair as shown below.

| **SEQ ID NO. /ISIS NO.** | **Composition (5' to 3')** |
|---|---|
| 35/379964 (s) | G_{f}UₘG_{f}AₘC_{f}UₘA_{f}CₘU_{f}CₘA_{f}CₘU_{f}UₘU_{f}UₘU_{f}AₘA_{f} |
| 34/379965 (as) | P-UₘU_{f}AₘA_{f}AₘA_{f}AₘG_{f}UₘG_{f}AₘG_{f}UₘA_{f}GₘU_{f}CₘA_{f}Cₘ |
| 66/400539 (s) | G_{f}UₘG_{f}AₘC_{f}UₘA_{f}CₘU_{f}CₘA_{f}CₘU_{f}UₘU_{f}UₘU_{f}AₘA_{f}UₑUₑ |
| 67/400547 (as) | P-UₘU_{f}AₘA_{f}AₘA_{f}AₘG_{f}UₘG_{f}AₘG_{f}UₘA_{f}GₘU_{f}CₘA_{f}CₘUₑUₑ |
| 35/400540 (s) | G_{f}UₘG_{f}AₘC_{f}UₘA_{f}CₘU_{f}CₘA_{f}CₘU_{f}UₘU_{f}AₘA_{f}-L-C₁₆ |
| 67/400547 (as) | P-UₘU_{f}AₘA_{f}AₘA_{f}AₘG_{f}UₘG_{f}AₘG_{f}UₘA_{f}GₘU_{f}CₘA_{f}CₘUₑUₑ |
| 66/400733 (s) | _{d}GU_{ed}GA_{ed}CU_{ed}AC_{ed}UC_{ed}AC_{ed}UU_{ed}UU_{ed}UA_{ed}AUₑUₑ |
| 67/400547 (as) | P-UₘU_{f}AₘA_{f}AₘA_{f}AₘG_{f}UₘG_{f}AₘG_{f}UₘA_{f}GₘU_{f}CₘA_{f}CₘUₑUₑ |
| 35/400734 (s) | dGU_{ed}GA_{ed}CU_{ed}AC_{ed}UC_{ed}AC_{ed}UU_{ed}UU_{ed}UA_{ed}A-L-C₁₆ |
| 67/400547 (as) | P-UₘU_{f}AₘA_{f}AₘA_{f}AₘG_{f}UₘG_{f}AₘG_{f}UₘA_{f}GₘU_{f}CₘA_{f}CₘUₑUₑ |
| 66/400541 (s) | GUₑGAₑCUₑACₑUCₑACₑUUₑUUₑUAₑAUₑUₑ |
| 67/400545 (as) | P-UUAAAAₘAₘGUGₘAₘGUAGUCₘAₘCₘUₑUₑ |
| 66/400733 (s) | _{d}GU_{ed}GA_{ed}CU_{ed}AC_{ed}UC_{ed}AC_{ed}UU_{ed}UU_{ed}UA_{ed}AUₑUₑ |
| 67/400545 (as) | P-UUAAAAₘAₘGUGₘAₘGUAGUCₘAₘCₘUₑUₑ |
| 35/400734 (s) | _{d}GU_{ed}GA_{ed}CU_{ed}AC_{ed}UC_{ed}AC_{ed}UU_{ed}UU_{ed}UA_{ed}A-L-C₁₆ |
| 67/400545 (as) | P-UUAAAAₘAₘGUGₘAₘGUAGUCₘAₘCₘUₑUₑ |

Each C₁₆ cojugate is attached to a pyrrolidinyl linker (L) which in turn is attached to the 3'-end of the respective antisense strand by a phosphorothioate linkage. The overall formula is shown below:

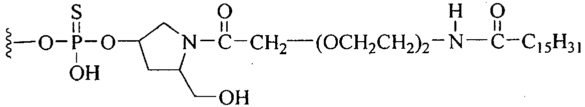

| **Composition (s-as)** | **IC50 (nM)** |
|---|---|
| 35/379964 - 34/379965 | 30 |
| 35/400539 - 34/400547 | 40 |
| 35/400540 - 34/400547 | 54 |
| 35/400733 - 34/400547 | 49 |
| 35/400734 - 34/400547 | 147 |
| 35/400541 - 34/400545 | 39 |
| 35/400733 - 34/400545 | 30 |
| 35/400734 - 34/400545 | 51. |

### Example 21: In vitro assay of selected differentially modified siRNAs in Survivin Xenograft Studies

The following siRNA compositions were prepared for Xenograft studies.

| **SEQ ID NO. /ISIS NO.** | **Composition (5' 3')** |
|---|---|
| 24/353714 (s) | G_{f}GₘA_{f}GₘA_{f}UₘC_{f}AₘA_{f}CₘA_{f}UₘU_{f}UₘU_{f}CₘA_{f}AₘA_{f} |
| 68/384774 (as) | P-UₘU_{f}UₘG_{f}AₘA_{f}AₘA_{f}UₘG_{f}UₘU_{f}GₘA_{f}UₘC_{f}UₘCₘU_{f}Uₘ |
| 24/388774 (s) | G_{f}GₘA_{f}GₘA_{f}UₘC_{f}AₘA_{f}CₘA_{f}UₘU_{f}UₘU_{f}CₘA_{f}AₘA_{f}-L-C₁₆ |
| 68/384774 (as) | P-UₘU_{f}UₘG_{f}AₘA_{f}AₘA_{f}UₘG_{f}UₘU_{f}GₘA_{f}UₘC_{f}UₘC_{f}CₘU_{f}Uₘ |
| 24/392935 (s) | G_{f}GₘA_{f}GₘA_{f}UₘC_{f}AₘA_{f}CₘA_{f}UₘU_{f}UₘU_{f}CₘA_{f}AₘA_{f}-L-Cholesterol |
| 68/384774 (as) | P-UₘU_{f}UₘG_{f}AₘA_{f}AₘA_{f}UmG_{f}UₘU_{f}GₘA_{f}UₘC_{f}UₘC_{f}CₘU_{f}Uₘ |
| 24/352514 (s) | GGₑAGₑAUₑCAₑACₑAUₑUUₑUCₑAAₑA |
| 68/352506 (as) | UUUGAAₘAₘAUGₘUₘUGAUCUCCₘUₘUₘ |
| 24/352514 (s) | GGₑAGₑAUₑCAₑACₑAUₑUUₑUCₑAAₑA |
| 68/392914 (as) | UUUGAAₘAₘAUGₘUₘUGAUCUCCₘUₘUₘ |
| 24/392936 (s) | GGₑAGₑAUₑCAₑACₑAUₑUUₑUCₑAAₑA-L-Cholesterol |
| 68/392914 (as) | UUUGAAₘAₘAUGₘUₘUGAUCUCCₘUₘUₘ |
| 24/392934 (s) | GGₑAGₑAUₑCAₑACₑAUₑUUₑUCₑAAₑA-L-C₁₆ |
| 68/392914 (as) | UUUGAAₘAₘAUGₘUₘUGAUCUCₘUₘUₘ |
| 24/355714 (s) | G_{f}GₘA_{f}GₘA_{f}UₘC_{f}AₘA_{f}CₘA_{f}UₘU_{f}UₘU_{f}CₘA_{f}AₘA_{f} |
| 68/384774 (as) | P-UₘU_{f}UₘG_{f}AₘA_{f}AₘA_{f}UₘG_{f}UₘU_{f}GₘA_{f}UₘC_{f}UₘC_{f}CₘU_{f}Uₘ |
| 24/391361 (s) | G_{f}GₘA_{f}GₘA_{f}UₘC_{f}AₘA_{f}CₘA_{f}UₘU_{f}UₘU_{f}CₘA_{f}AₘA_{f}-L-Octreotate |
| 68/384774 (as) | P-UₘUₑUₘG_{f}AₘA_{f}AₘA_{f}UₘG_{f}UₘU_{f}GₘA_{f}UₘC_{f}UₘCₘU_{f}Uₘ |
| 24/391362 (s) | G_{f}GₘA_{f}GₘA_{f}UₘC_{f}AₘA_{f}CₘA_{f}UₘUₘUₘU_{f}CₘA_{f}AₘA_{f}-L-Lyp-1-protein |
| 68/384774 (as) | P-UₘU_{f}UₘG_{f}AₘA_{f}AₘA_{f}UₘG_{f}UₘU_{f}GₘA_{f}UₘC_{f}UₘC_{f}CₘU_{f}Uₘ |

| **Composition (s-as)** | **IC₅₀ (nM)** |
|---|---|
| 24/391361 - 68/384774 | 27 |
| 24/391362 - 68/384774 | 23 |

The linker (L) used was the same as in example 19 above.

### Example 22: In vitro assay of selected differentially modified siRNAs targeted to eIF4E

In accordance with the present invention, a series of oligomeric compounds were synthesized and tested for their ability to reduce eIF4E expression over a range of doses. The nucleosides are annotated as to chemical modification as per the legend at the beginning of the examples. b.END cells were treated with the double stranded oligomeric compounds (siRNA constructs) shown below (antisense strand followed by the sense strand of the duplex) at concentrations of 0.0625 nM, 0.25 nM, 1 nM, or 4 nM using methods described herein. Expression levels of mouse eIF4E were determined using real-time PCR methods as described herein. Resulting dose-response curves were used to determine the IC50 for each pair as shown below.

| **SEQ ID NO. /ISIS NO.** | **Composition (5' to 3')** | **IC50** |
|---|---|---|
| 30/371286 (as) | UUUAGCUCUAACAUUAACA | 0.2055 |
| 31/371280 (s) | UGUUAAUGUUAGAGCUAAA | |
| 30/371287 (as) | UUUAGCₘUₘCUAₘAₘCAUUAAₘCₘAₘ | 0.238 |
| 31/371280 (s) | UGUUAAUGUUAGAGCUAAA | |
| 30/371287 (as) | UUUAGCₘUₘCUAₘAₘCAUUAAₘCₘAₘ | 9.496 |
| 31/371284 (s) | UₑGₑUₑUAAUGUUAGAGCUAₑAₑAₑ | |
| 30/371286 (as) | UUUAGCUCUAACAUUAACA | 1.193 |
| 31/371284 (s) | UₑGₑUₑUAAUGUUAGAGCUAₑAₑAₑ | |
| 32/371297 (as) | UUACUAGACAACUGGAUAU | 0.1859 |
| 33/371291 (s) | AUAUCCAGUUGUCUAGUAA | |
| 32/371298 (as) | UUACUAₘGₘACAₘAₘCUGGAUₘAₘUₘ | 0.1946 |
| 33/371291 (s) | AUAUCCAGUUGUCUAGUAA | |
| 32/371297 (as) | UUACUAGACAACUGGAUAU | 0.0936 |
| 33/371295 (s) | AₑUₑAₑUCCAGUUGUCUAGUₑAₑAₑ | |
| 32/371298 (as) | UUACUAₘGₘACAₘAₘCUGGAUₘAₘUₘ | 0.1151 |
| 33/371295 (s) | AₑUₑAₑUCCAGUUGUCUAGUₑAₑAₑ | |
| 34/371308 (as) | UUAAAAAGUGAGUAGUCAC | 0.2926 |
| 35/371302 (s) | GUGACUACUCACUUUUUAA | |
| 34/371309 (as) | UUAAAAₘAₘGUGₘAₘGUAGUCₘAₘCₘ | 0.1626 |
| 35/371302 (s) | GUGACUACUCACUUUUUAA | |
| 34/371308 (as) | UUAAAAAGUGAGUAGUCAC | 0.0632 |
| 35/371306 (s) | GₑUₑGₑACUACUCACUUUUUₑAₑAₑ | |
| 34/371309 (as) | UUAAAAₘAₘGUGₘAₘGUAGUCₘAₘCₘ | 0.0061 |
| 35/371306 (s) | GₑUₑGₑACUACUCACUUUUUₑAₑAₑ. | |

### Example 23: Blockmer walk of 5 2'-O-methy modified nucleosides in the antisense strand of siRNAs assayed for PTEN mRNA levels against untreated control

The antisense (AS) strands listed below were designed to target human PTEN, and each was duplexed with the same sense strand (ISIS 271790, shown below). The duplexes were tested for their ability to reduce PTEN expression over a range of doses to determine the relative positional effect of the 5 modifications using methods described herein. The nucleosides are annotated as to chemical modification as per the legend at the beginning of the examples. Expression levels of PTEN were determined using real-time PCR methods as described herein, and were compared to levels determined for untreated controls.

| **SEQ ID NO:/ISIS NO** | **Sequence 5'-3'** |
|---|---|
| 36/271790 (S) | CAAAUCCAGAGGCUAGCAGdTdT |
| 37/271071(AS) | CₘUₘGₘCₘUₘAGCCUCUGGAUUUGdTdT |
| 37/271072(AS) | CUₘGₘCₘUₘAₘGCCUCUGGAUUUGdTdT |
| 37/271073(AS) | CUGₘCₘUₘAₘGₘCCUCUGGAUUUGdTdT |
| 37/271074(AS) | CUGCₘUₘAₘGₘCₘCUCUGGAUUUGdTdT |
| 37/271075(AS) | CUGCUₘAₘGₘCₘCₘUCUGGAUUUGdTdT |

The siRNAs having 2'-O-methyl groups at least 2 positions removed from the siRNAs having 5, 2'-O-methyl groups at least 2 positions removed from the 5'-end of the antisense strand reduced PTEN mRNA levels to from 25 to 35% of untreated control. The remaining 2 constructs increased PTEN mRNA levels above untreated control.

### Example 24: Solid block of 2'-O-methyl modified nucleosides in the antisense strand of siRNAs assayed for PTEN mRNA levels against untreated control

The antisense (AS) strands listed below were designed to target human PTEN, and each was duplexed with the same sense strand 271790. The duplexes were tested for their ability to reduce PTEN expression over a range of doses to determine the relative effect of adding either 9 or 14, 2'-O-methyl modified nucleosides at the 3'-end of the resulting siRNAs. The nucleosides are annotated as to chemical modification as per the legend at the beginning of the examples. Expression levels of PTEN were determined using real-time PCR methods as described herein, and were compared to levels determined for untreated controls.

| **SEQ ID NO:/ISIS NO** | **Sequence 5'-3'** |
|---|---|
| 36/271790 (S) | CAAAUCCAGAGGCUAGCAGdTdT |
| 38/271079(AS) | CUGCUAGCCUCUGₘGₘAₘUₘUₘUₘGₘUₘUₘ |
| 38/271081(AS) | CUGCUAGCₘCₘUₘCₘUₘGₘGₘAₘUₘUₘUₘGₘUₘUₘ |

The siRNA having 9, 2'-O-methyl nucleosides reduced PTEN mRNA levels to about 40% of untreated control whereas the construct having 14, 2'-O-methyl nucleosides only reduced PTEN mRNA levels to about 98% of control.

### Example 25: 2'-O-methy blockmers (siRNA vs asRNA)

A series of blockmers were prepared as single strand antisense RNAs (asRNAs). The antisense (AS) strands listed below were designed to target PTEN, and each was also assayed as part of a duplex with the same sense strand (ISIS 308746, shown below) for their ability to reduce PTEN expression levels. T24 cells were treated with the single stranded or double stranded oligomeric compounds created with the antisense compounds shown below using methods described herein. The nucleosides are annotated as to chemical modification as per the legend at the beginning of the examples. Expression levels of human PTEN were determined using real-time PCR methods as described herein, and were compared to levels determined for untreated controls.

| **SEQ ID NO:/ISIS NO** | **Sequence 5'-3'** |
|---|---|
| 39/308746 (S) | AAGUAAGGACCAGAGACAAA |
| 40/303912 (AS) | P-UUUGUCUCUGGUCCUUACUU |
| 40/316449 (AS) | P-UUUGUCUCUGGUCCUUACₘUₘUₘ |
| 40/335223 (AS) | P-UUUGUCUCUGGUCCUₘUₘAₘCUU |
| 40/335224 (AS) | P-UUUGUCUCUGGUₘCₘCₘUUACUU |
| 40/335225 (AS) | P-UUUGUCUCUₘGₘGₘUCCUUACUU |
| 40/335226 (AS) | P-UUUGUCₘUₘCₘUGGUCCUUACUU |
| 40/335227 (AS) | P-UUUₘGₘUₘCUCUGGUCCUUACUU |
| 40/335228 (AS) | P-UₘUₘUₘGUCUCUGGUCCUUACUU |

All of the asRNAs and siRNAs showed activity with the asRNAs having better activity than the corresponding duplex in each case. A clear dose response was seen for all of the siRNA constructs (20, 40, 80 and 150 nm doses). A dose-responsive effect was also observed for the asRNAs for 50, 100 and 200 nm doses. In general the siRNAs were more active in this system at lower doses than the asRNAs and at the 150 nm dose were able to reduce PTEN mRNA levels to from 15 to 40% of untreated control. The duplex containing unmodified 303912 reduced PTEN mRNA levels to about 19% of the untreated control.

### Example 26: siRNA hemimer constructs

Three siRNA hemimer constructs were prepared and were tested for their ability to reduce PTEN expression levels. The hemimer constructs had 7, 2'-O-ethyl nucleosides at the 3'-end. The hemimer was put in the sense strand only, the antisense strand only and in both strands to compare the effects. Cells were treated with the double stranded oligomeric compounds (siRNA constructs) shown below (antisense strand followed by the sense strand of the duplex) using methods described herein. The nucleosides are annotated as to chemical modification as per the legend at the beginning of the examples. Expression levels of PTEN were determined using real-time PCR methods as described herein, and were compared to levels determined for untreated controls.

| **SEQ ID NO:/ISIS NO** | **Constructs (overhangs) 5'-3'** |
|---|---|
| 38/XXXXX (AS) | CUGCUAGCCUCUGGAₘUₘUₘUₘGₘUₘUₘ |
| 41/271068 (S) | CAAAUCCAGAGGCUAₘGₘCₘAₘGₘUₘUₘ |
| 38/XXXXX (AS) | CUGCUAGCCUCUGGAUUUGUU |
| 41/271068 (S) | CAAAUCCAGAGGCUAₘGₘCₘAₘGₘUₘUₘ |
| 38/XXXXX (AS) | CUGCUAGCCUCUGGAₘUₘUₘUₘGₘUₘUₘ |
| 41/XXXXX (S) | CAAAUCCAGAGGCUAGCAGUU |

The construct having the 7, 2'-O-methyl nucleosides only in the antisense strand reduced PTEN mRNA levels to about 23% of untreated control. The construct having the 7, 2'-O-methyl nucleosides in both strands reduced the PTEN mRNA levels to about 25% of untreated control. When the 7, 2'-O-methyl nucleosides were only in the sense strand, PTEN mRNA levels were reduced to about 31 % of untreated control.

### Example 27: Representative siRNAs prepared having 2'O-Me gapmers

The following antisense strands of selected siRNA duplexes targeting PTEN are hybridized to their complementary full phosphodiester sense strands. Activity is measured using methods described herein. The nucleosides are annotated as to chemical modification as per the legend at the beginning of the examples.

| **SEQ ID NO:** | **Sequence (5'-3')** |
|---|---|
| 42/300852 | CUGCₘUₘAₘGₘCCUCUGGAUUₘUₘGₘAₘ |
| 42/300853 | P-CUGCₘUₘAₘGₘCCUCUGGAUUₘUₘGₘAₘ |
| 42/300854 | CₘUₘGₘCₘUAGCCUCUGGAUUₘUₘGₘAₘ |
| 42/300855 | P-CUGCUₘAₘGₘCCUCUGGAUUₘUₘGₘAₘ |
| 42/300856 | CₘUₘAₘGₘCCUCUGGAUUₘUₘGₘAₘ |
| 42/300858 | CUGCₘUₘAₘGₘCCUCUGGAUUₘUₘGₘAₘ |
| 42/300859 | P-CUGCₘUₘAₘGₘCCUCUGGAUUₘUₘGₘAₘ |
| 42/300860 | CₘUₘAₘGₘCCUCUGGAUUₘUₘGₘAₘ |
| 43/303913 | GₘUₘCₘUₘCUGGUCCUUAₘCₘUₘUₘ |
| 44/303915 | UₘUₘUₘUₘGUCUCUGGUCₘUₘUₘ |
| 45/303917 | CₘUₘGₘGₘUCCUUACUUCₘCₘCₘCₘ |
| 40/308743 | P-UₘUₘUₘGUCUCUGGUCCUUACₘUₘUₘ |
| 47/308744 | P-UₘCₘUₘCₘUₘGGUCCUUACUUₘCₘCₘCₘCₘ |
| 40/328795 | P-UUUGₘUₘCₘUₘCUGGUCCUUAₘCₘUₘUₘ. |

### Example 28: Representative siRNAs prepared having 2'-F modified nucleosides and various structural motifs

The following antisense strands of siRNAs targeting PTEN were tested as single strands alone or were hybridized to their complementary full phosphodiester sense strand and were tested in duplex. The nucleosides are annotated as to chemical modification as per the legend at the beginning of the examples. Bolded and italicized "C" indicates a 5-methyl C ribonucleoside.

| **SEQ ID NO/ISIS NO** | | **Sequences 5'-3'** |
|---|---|---|
| 40/319022 | AS | U_{f}U_{f}U_{f}C_{f}U_{f}C_{f}U_{f}C_{f}U_{f}G_{f}G_{f}U_{f}C_{f}C_{f}U_{f}U_{f}A_{f}C_{f}U_{f}U_{f} |
| 40/333749 | AS | UUUGUCUCUGGUCCU_{f}U_{f}A_{f}CUU |
| 40/333750 | AS | UUUGUCUCUGGU_{f}C_{f}C_{f}UUACUU |
| 40/333751 | AS | UUUGUCUCUGGU_{f}C_{f}C_{f}UUACUU |
| 40/333752 | AS | UUUGUC_{f}U_{f}C_{f}UGGUCCUUACUU |
| 40/333753 | AS | UUU_{f}G_{f}U_{f}CUCUGGUCCUUACUU |
| 40/333754 | AS | U_{f}U_{f}U_{f}GUCUCUGGUCCUUACUU |
| 40/333756 | AS | UUUGUCUCUGGUCCUUAC_{f}U_{f}U_{f} |
| 40/334253 | AS | UUUGUCUCU_{f}G_{f}G_{f}UCCUUACUU |
| 40/334254 | AS | UUUGUCUCUGGUCCUU_{f}A_{f}C_{f}U_{f}U_{f} |
| 40/334255 | AS | UUU_{f}G_{f}U_{f} CUCUGGUCCUUACUU |
| 40/334256 | AS | UUU_{f}G_{f}U_{f}CUCUGGU_{f}C_{f}C_{f}UUACUU |
| 40/334257 | AS | U_{f}U_{f}U_{f}GUCUCUGGUCCUUACUU |
| 40/317466 | AS | U_{f}U_{f}U_{f}GUCUCUGGUCCUUAC_{f}U_{f}U |
| 40/317468 | AS | U_{f}U_{f}U_{f}GUCUCUGGUCCUUAC_{f}U_{f}U |
| 40/317502 | AS | U_{f}U_{f}U_{f}GU_{f}C_{f}U_{f}CUGGUCC_{f}U_{f}U_{f}AC_{f}U_{f}U |

Cells were treated with the indicated concentrations of single or double stranded oligomeric compounds shown above using methods described herein. Expression levels of PTEN were determined using real-time PCR methods as described herein, and were compared to levels determined for untreated controls.

| | **% untreated control mRNA** | |
|---|---|---|
| **Construct** | **100 nM asRNA** | **100 nM siRNA** |
| 303912 | 35 | 18 |
| 317466 | -- | 28 |
| 317408 | -- | 18 |
| 317502 | -- | 21 |
| 334254 | -- | 33 |
| 333756 | 42 | 19 |
| 334257 | 34 | 23 |
| 334255 | 44 | 21 |
| 333752 | 42 | 18 |
| 334253 | 38 | 15 |
| 333750 | 43 | 21 |
| 333749 | 34 | 21 |

Additional siRNAs having 2'-F modified nucleosides are listed below.

| | | |
|---|---|---|
| 37/279471 | AS | ***C***_{f}U_{f}G_{f}***C***_{f}U_{f}A_{f}G_{f}***C***_{f}***C***_{f}U***_{f}C***_{f}U_{f}G_{f}G_{f}A_{f}U_{f}U_{f}U_{f}G_{f}dTdT |
| 36/279467 | S | C_{f}A_{f}A_{f}A_{f}U_{f}***C***_{f} ***C***_{f}A_{f}C_{f}A_{f}G_{f}G_{f}C_{f}U_{f}A_{f}G_{f}***C***_{f}A_{f}G_{f}dTdT |
| 40/319018 | AS | U_{f}U_{f}U_{f}GₜU_{f}C_{f}U_{f}C_{f}U_{f}G_{f}G_{f}U_{f}C_{f}C_{f}U_{f}U_{f}A_{f}C_{f}U_{f}U_{f} |
| 39/319019 | S | A_{f}A_{f}G_{f}U_{f}A_{f}A_{f}G_{f}G_{f}A_{f}C_{f}C_{f}A_{f}G_{f}A_{f}G_{f}A_{f}C_{f}A_{f}A_{f}A_{f} |

### Example 29: Representative siRNAs prepared with fully modified antisense strands (2'-F and 2'-OMe)

siRNA constructs targeting PTEN are prepared wherein the following sense and antisense strands are hybridized. The nucleosides are annotated as to chemical modification as per the legend at the beginning of the examples.

| **SEQ ID NO/ISIS NO** | | **Sequences 5'-3'** |
|---|---|---|
| 48/283546 | (as) | C_{f}U_{f}GₘC_{f}U_{f}AₘGₘC_{f}C_{f}U_{f}C_{f}U_{f}GₘGₘAₘU_{f}U_{f}U_{f}GₘUₘdT |
| 40/336240 | (s) | UUUGUCUC_{f}U_{f}GGU_{f}C_{f}CUUACₘUₘUₘ |

### Example 30: Representative siRNAs prepared having 2'-MOE modified nucleosides were assayed for PTEN mRNA levels against untreated control

siRNA constructs targeting PTEN were prepared wherein the following antisense strands were hybridized to the complementary full phosphodiester sense strand.

The following antisense strands of siRNAs were hybridized to the complementary full phosphodiester sense strand. The nucleosides are annotated as to chemical modification as per the legend at the beginning of the examples. Linkages are phosphorothioate. Cells were treated with the duplexes using methods described herein. Results obtained using 100nM duplex are presented as a percentage of untreated control PTEN mRNA levels.

| **SEQ ID NO. /ISIS NO.** | | **Composition (5' to 3')** | **PTEN mRNA level (%UTC) 100 nM** |
|---|---|---|---|
| 49/xxxxx | (as) | UUCAUUCCUGGUCUCUGUUU | -- |
| 49/xxxxx | (as) | UₑUₑCₑAUUCCUGGUCUCUGUUU | 50 |
| 49/xxxxx | (as) | UUCAₑUₑUₑCCUGGUCUCUGUUU | -- |
| 49/xxxxx | (as) | UUCAUUCₑCₑUₑGGUCUCUGUUU | 43 |
| 49/xxxxx | (as) | UUCAUUCCUGₑGₑUₑCUCUGUUU | 42 |
| 49/xxxxx | (as) | UUCAUUCCUGGUCₑUₑCₑUGUUU | 47 |
| 49/xxxxx | (as) | UUCAUUCCUGGUCUCUₑGₑU_{E}UU | 63 |
| 49/xxxxx | (as) | UUCAUUCCUGGUCUCUGUₑUₑUₑ | 106 |

### Example 31: 4'-Thio and 2'-OCH₃ chimeric oligomeric compounds

The double-stranded constructs shown below were prepared (antisense strand followed by the sense strand of the duplex). The "P" following the designation for antisense (as) indicates that the target is PTEN and the "S" indicates that the target is Survivin. The nucleosides are annotated as to chemical modification as per the legend at the beginning of the examples.

| **SEQ ID NO. /ISIS NO.** | | **Composition (5' to 3')** |
|---|---|---|
| 40/308743 | (as-P) | UₘUₘUₘGUCUCUGGUCCUUACₘUₘUₘ |
| 39/308746 | (s) | AAGUAAGGACCAGAGACAAA |
| 24/353537 | (as-S) | UₜUₜUₜGAAAAUGUUGAUCUₜCₜCₜ |
| 25/343868 | (s-S) | GGAGAUCAACAUUUUCAAA |
| 24/353537 | (as-S) | UₜUₜUₜGAAAAUGUUGAUCUₜCₜCₜ |
| 25/352512 | (s) | GₘGₘAₘGₘAₘUₘCₘAₘAₘCₘAₘUₘUₘUₘUₘCₘAₘAₘAₘ |
| 24/353537 | (as-S) | UₜUₜUₜGAAAAUGUUGAUCUₜCₜCₜ |
| 25/352513 | (s) | GGₘAₘGₘAₘUₘCₘAₘAₘCₘAₘUₘUₘUₘUₘCₘAₘAₘA |
| 24/353537 | (as-S) | U_{f}UₜUₜGAAAAUGUUGAUCUₜCₜCₜ |
| 25/352514 | (s) | GGₑAGₑAUₑCAₑACₑAUₑUUₑUCₑAAₑA |

The constructs designed to the targets indicated were tested in accordance with the assays described herein. The duplexed oligomeric compounds were evaluated in HeLa cells (American Type Culture Collection, Manassas VA). Culture methods used for HeLa cells are available from the ATCC and may be found, for example, at http://www.atcc.org. For cells grown in 96-well plates, wells were washed once with 200 µL OPTI-MEM-1 reduced-serum medium and then treated with 130 µL of OPTI-MEM-1 containing 12 µg/mL LIPOFECTIN™ (Invitrogen Life Technologies, Carlsbad, CA) and the dsRNA at the desired concentration. After about 5 hours of treatment, the medium was replaced with fresh medium. Cells were harvested 16 hours after dsRNA treatment, at which time RNA was isolated and target reduction measures by quantitative real-time PCR as described in previous examples. Resulting dose-response data was used to determine the IC50 for each construct.

| **Construct** | **Assay/Species** | **Target IC50 (nM)** |
|---|---|---|
| 308743:308746 | Dose Response/Human | PTEN 0.0275 |
| 353537:343868 | Dose Response/Human | Survivin 0.067284 |
| 353537:343868 | Dose Response/Human | Survivin 0.17776 |
| 353537:343868 | Dose Response/Human | Survivin 0.598 |
| 353537:343868 | Dose Response/Human | Survivin 4.23 |
| 353537:352512 | Dose Response/Human | Survivin 0.60192 |
| 353537:352513 | Dose Response/Human | Survivin 0.71193 |
| 353537:352514 | Dose Response/Human | Survivin 0.48819 |

### Example 32: Selected siRNA constructs prepared and tested against eIF4E and Survivin targets

Selected siRNA constructs were prepared and tested for their ability to lower targeted RNA as measured by quantitative real-time PCR. The duplexes are shown below (antisense strand followed by the sense strand of the duplex). The nucleosides are annotated as to chemical modification as per the legend at the beginning of the examples.

| **SEQ ID NO. /ISIS NO.** | **Composition (5' to 3') Targeted to eIF4E** |
|---|---|
| 50/349894 (as) | U_{f}G_{f}U_{f}C_{f}A_{f}UAUUCCUGGAUₘCₘCₘUₘUₘ |
| 51/338935 (s) | AAGGAUCCAGGAAUAUGACA |
| 52/349895 (as) | U_{f}C_{f}C_{f}U_{f}G_{f}GAUCCUUCACCₘAmAₘUₘGₘ |
| 53/338939 (s) | CAUUGGUGAAGGAUCCAGGA |
| 54/349896 (as) | U_{f}C_{f}U_{f}U_{f}A_{f}UCACCUUUAGCₘUₘCₘUₘAₘ |
| 55/338943 (s) | UAGAGCUAAAGGUGAUAAGA |
| 56/349897 (as) | A_{f}U_{f}A_{f}C_{f}U_{f}CAGAAGGUGUCₘUₘUₘCₘUₘ |
| 57/338952 (s) | AGAAGACACCUUCUGAGUAU |
| 58/352827 (as) | UₛCₛUₛUAUCACCUUUAGCUₘCₘUₘ |
| 59/342764 (s) | AGAGCUAAAGGUGAUAAGA |
| 58/354604 (as) | UₛCₛUₛU_{f}A_{f}U_{f}C_{f}A_{f}C_{f}C_{f}U_{f}U_{f}U_{f}A_{f}G_{f}C_{f}UₘCₘUₘ |
| 59/342764 (s) | AGAGCUAAAGGUGAUAAGA |

| **SEQ ID NO**. **/ISIS NO.** | **Composition (5' to 3') Targeted to Survivin** |
|---|---|
| 24/355710 (as) | U_{f}U_{f}U_{f}G_{f}A_{f}A_{f}AAUGUUGAUₘCₘUₘCₘCₘ |
| 25/343868 (s) | GGAGAUCAACAUUUUCAAA |
| 24/353540 (as) | UₛUₛUₛGAAAAUGUUGAUCUₘCₘCₘ |
| 45/343868 (s) | GGAGAUCAACAUUUUCAAA |

The above constructs were tested in HeLa cells, MH-S cells or U-87 MG cells using transfection procedures and real-time PCR as described herein. The resulting IC₅₀'s for the duplexes were calculated and are shown below.

| **Construct** | **Species/cell line** | **Gene** | **IC₅₀** |
|---|---|---|---|
| 349894:338935 | Human/HeLa | eIF4E | 0.165 |
| 349895:338939 | Human/HeLa | eIF4E | 0.655 |
| 349896:338943 | Human/HeLa | eIF4E | 0.277 |
| 349896:338943 | Mouse/MH-S | eIF4E | 0.05771 |
| 349897:338952 | Human/HeLa | eIF4E | 0.471 |
| 352827:342764 | Human/HeLa | eIF4E | 2.033 |
| 352827:342764 | Mouse/MH-S | eIF4E | 0.34081 |
| 354604:342764 | Human/HeLa | eIF4E | 2.5765 |
| 355710:343868 | Human/HeLa | Survivin | 0.048717 |
| 353540:343868 | Human/HeLa | Survivin | 0.11276 |
| 353540:343868 | Human/U-87 MG | Survivin | 0.0921 |

### Example 33: Positionally Modified Compositions

The table below shows exemplary positionally modified compositions prepared in accordance with the present invention. Target descriptors are: P=PTEN; S=Survivin; E=eIF4E and are indicated following the antisense strand designation.

| **SEQ ID NO. /ISIS NO.** | **Composition (5' to 3')** |
|---|---|
| 52/345838 (as-P) | UCCUGGₘAUCCUUₘCACₘCAAₘUₘGₘ |
| 53/338939 (s) | CAUUGGUGAAGGAUCCAGGA |
| 60/345839 (as-E) | CCUGGₘAₘUCCₘUₘUCACCAAₘUₘGₘ |
| 53/338939 (s) | CAUUGGUGAAGGAUCCAGGA |
| 56/345853 (as-E) | AUACUCₘAₘGAAₘGₘGUGUCUUₘCₘUₘ |
| 57/338952 (s) | AGAAGACACCUUCUGAGUAU |
| 24/352505 (as-S) | UUUGAₘAAAₘUGUₘUGAₘUCUₘCₘCₘ |
| 25/343868 (s) | GGAGAUCAACAUUUUCAAA |
| 24/352506 (as-S) | UUUGAAₘAₘAUGₘUₘUGAUCUₘCₘCₘ |
| 25/343868 (s) | GGAGAUCAACAUUUUCAAA |
| 24/352506 (as-S) | UUUGAAₘAₘAUGₘUₘUGAUCUₘCₘCₘ |
| 25/346287 (s) | GGAGAUCAACAUUUUCAAA |
| 24/352505 (as-S) | UUUGAₘAAAₘUGUₘUGAₘUCUₘCₘCₘ |
| 25346287 (s) | GGAGAUCAACAUUUUCAAA |
| 24/352505 (as-S) | UUUGAₘAAAₘUGUₘUGAₘUCUₘCₘCₘ |
| 25/352511 (s) | GGₘAGₘAUₘCAₘACₘAUₘUUₘUCₘAAₘA |
| 24/352505 (as-S) | UUUGAₘAAAₘUGUₘUGAₘUCUₘCₘCₘ |
| 25/352513 (s) | GGₘAₘGₘAₘUₘCₘAₘAₙᵢCₘAₘUₘUₘUₘUₘCₘAₘAₘA |
| 24/352506 (as-S) | UUUGAAₘAₘAUGₘUₘUGAUCUₘCₘCₘ |
| 25/352511 (s) | GGₘAGₘAUₘCAₘACₙᵢAUₘUUₘUCₘAAₘA |
| 24/352505 (as-S) | UUUGAₘAAAₘUGUₘUGAₘUCUₘCₘCₘ |
| 25/352514 (s) | GGₑAGₑAUₑCAₑACₑAUₑUUₑUCₑAAₑA |
| 24/352506 (as-S) | UUUGAAₘAₘAUGₘUₘUGAUCUₘCₘCₘ |
| 25/352514 (s) | GGₑAGₑAUₑCAₑACₑAUₑUUₑUCₑAAₑA |
| 24/352505 (as-S) | UUUGAₘAAAₘUGUₘUGAₘUCUₘCₘCₘ |
| 25/352512 (s) | GₘGₘAₘGₘAₘUₘCₘAₘAₘCₘAₘUₘUₘUₘUₘCₘAₘAₘAₘ |
| 56/345853 (as-E) | AUACUCₘAₘGAAₘGₘGUGUCUUₘCₘUₘ |
| 57/345857 (s) | AGₘAₘAₘGₘAₘCₘAₘCₘCₘUₘUₘCₘUₘGₘAₘGₘUₘAₘU |
| 24/352506 (as-S) | UUUGAAₘAₘAUGₘUₘUGAUCUₘCₘCₘ |
| 25/352512 (s) | GⱼₙGₘAₘGₘAₘUₘCₘAₘAₘCₘAₘUₘUₘUₘUₘCₘAₘAₘAₘ |
| 24/352506 (as-S) | UUUGAAₘAₘAUGₘUₘUGAUCUₘCₘCₘ |
| 25/352513 (s) | GGₘAₘGₘAₘUₘCₘAₘAₘCₘAₘUₘUₘUₘUₘCₘAₘAₘA |
| 40/335225 (as-P) | UUUGUCUCUₘGₘGₘUCCUUACUU |
| 39/308746 (s) | AAGUAAGGACCAGAGACAAA |
| 40/335226 (as-P) | UUUGUCₘUₘCₘUGGUCCUUACUU |
| 39/308746 (s) | AAGUAAGGACCAGAGACAAA |
| 40/345711 (as-P) | UUUGₗUCUCUGₗGUCCUUACUₗU |
| 39/308746 (s) | AAGUAAGGACCAGAGACAAA |
| 40/345712 (as-P) | UUUₗGUCUCUGₗGₗUCCUUAₗCₗUU |
| 39/308746 (s) | AAGUAAGGACCAGAGACAAA |
| 40/347348 (as-P) | UₗUₗUₗGUCₗUCUₗGGUₗCCUₗUACₗUₗUₗ |
| 39/308746 (s) | AAGUAAGGACCAGAGACAAA |
| 40/348467 (as-P) | U_{!}U_{!}U_{!}GUCₗUCUₗGGUₗCCUₗUACₗUₗUₗ |
| 39/308746 (s) | AAGUAAGGACCAGAGACAAA |
| 24/355715 (as-S) | UUUGₗAAAAUₗGUUGAUCUCₗC |
| 25/343868 (s) | GGAGAUCAACAUUUUCAAA |
| 40/331426 (as-P) | UUUGUCUCUₗGₗGₗUCCUUACUU |
| 39/308746 (s) | AAGUAAGGACCAGAGACAAA |
| 40/331695 (as-P) | UUUGUCUCUGGUCCUUACₗUₗUₗ |
| 39/308746 (s) | AAGUAAGGACCAGAGACAAA |
| 40/332231 (as-P) | UUUGUCUCUGGUCCUUACUₜU |
| 39/308746 (s) | AAGUAAGGACCAGAGACAAA |
| 24/355712 (as-S) | UUUGAₗAAAₗUGUₗUGAₗUCUₘCₘCₘ |
| 25/343868 (s) | GGAGAUCAACAUUUUCAAA |
| 24/353538 (as-S) | UUUₜGAAAAUₜGUUₜGAUCUₜCₜCs |
| 25/343868 (s) | GGAGAUCAACAUUUUCAAA |
| 40/336671 (as-P) | UUUGUCUCUGGUCCUUACₜUₜUs |
| 39/308746 (s) | AAGUAAGGACCAGAGACAAA |
| 40/336674 (as-P) | UUUGUCUCUGGUCCUUₜACₜUₜUs |
| 39/308746 (s) | AAGUAAGGACCAGAGACAAA |
| 40/336675 (as-P) | UUUGUCUCUGGUCCUUACUUs |
| 39/308746 (s) | AAGUAAGGACCAGAGACAAA |
| 40/336672 (as-P) | UUUGUCUCUGGUCₜCₜUₜUACUU |
| 39/308746 (s) | AAGUAAGGACCAGAGACAAA |
| 40/336673 (as-P) | UUUGUCUCUGGUₜCₜCₜUUACUU |
| 39/308746 (s) | AAGUAAGGACCAGAGACAAA |
| 40/336676 (as-P) | UUUGUCUₜCₜUₜGGUCCUUACUU |
| 39/308746 (s) | AAGUAAGGACCAGAGACAAA |
| 40/336678 (as-P) | UₜUₜUₜGUCUCUGGUCCUUACUU |
| 39/308746 (s) | AAGUAAGGACCAGAGACAAA |
| 24/352515 (as-S) | UUUGAAAAUGUUGAUₘCₘUₘCₘCₘ |
| 25/343868 (s) | GGAGAUCAACAUUUUCAAA |
| 61/330919 (as-P) | UUTₑGₑTₑCUCUGGUCCUUACUU |
| 39/308746 (s) | AAGUAAGGACCAGAGACAAA |
| 62/330997 (as-P) | TₑTₑTₑGTCUCUGGUCCUUACUU |
| 39/308746 (s) | AAGUAAGGACCAGAGACAAA |
| 40/333749 (as-P) | UUUGUCUCUGGUCCU_{f}U_{f}A_{f}CUU |
| 39/308746 (s) | AAGUAAGGACCAGAGACAAA |
| 40/333750 (as-P) | UUUGUCUCUGGU_{f}C_{f}C_{f}UUACUU |
| 39/308746 (s) | AAGUAAGGACCAGAGACAAA |
| 40/333752 (as-P) | UUUGUC_{f}U_{f}C_{f}UGGUCCUUACUU |
| 39/308746 (s) | AAGUAAGGACCAGAGACAAA |
| 40/333756 (as-P) | UUUGUCUCUGGUCCUUAC_{f}U_{f}U_{f} |
| 39/308746 (s) | AAGUAAGGACCAGAGACAAA |
| 40/334253 (as-P) | UUUGUCUCUfG_{f}G_{f}UCCUUACUU |
| 39/308746 (s) | AAGUAAGGACCAGAGACAAA |
| 24/353539 (as-S) | UₜUₜUₜGAAAAUₜGUUₜGAUCUₘCₘCₘ |
| 25/343868 (s) | GGAGAUCAACAUUUUCAAA |

The above constructs were tested in HeLa cells, MH-S cells or U-87 MG cells using methods described herein. Resulting IC₅₀'s were calculated and are shown below. Also shown are the species to which the compounds were targeted and the cell line in which they were assayed.

| **Construct** | **Species/Cell Line** | **Gene** | **IC50** |
|---|---|---|---|
| 345838:338939 | Mouse/MH-S | eIF4E | 0.022859 |
| 345839:338939 | Mouse/MH-S | eIF4E | 0.01205 |
| 345853:338952 | Mouse/MH-S | eIF4E | 0.075517 |
| 352505:343868 | Human/HeLA | Survivin | 0.17024 |
| 352506:343868 | Human/HeLA | Survivin | 0.055386 |
| 352506:346287 | Human/HeLA | Survivin | 0.11222 |
| 352505:346287 | Human/HeLA | Survivin | 0.96445 |
| 352505:352511 | Human/HeLA | Survivin | 0.21527 |
| 352505:352513 | Human/HeLA | Survivin | 0.12453 |
| 352506:352511 | Human/HeLA | Survivin | 0.045167 |
| 352505:352514 | Human/HeLA | Survivin | 0.47593 |
| 352506:352514 | Human/HeLA | Survivin | 0.11759 |
| 352506:352514 | Human/HeLA | Survivin | 0.376 |
| 352506:352514 | Human/U-87 MG | Survivin | 0.261 |
| 352505:352512 | Human/HeLA | Survivin | 0.075608 |
| 345853:345857 | Mouse/MH-S | eIF4E | 0.025677 |
| 352506:352512 | Human/HeLA | Survivin | 0.11093 |
| 352506:352513 | Human/HeLA | Survivin | 0.24503 |
| 335225:308746 | Human/HeLA | PTEN | 0.809 |
| 335226:308746 | Human/HeLA | PTEN | 1.57 |
| 308746:345711 | Human/HeLA | PTEN | 1.13 |
| 308746:345712 | Human/HeLA | PTEN | 0.371 |
| 308746:347348 | Human/HeLA | PTEN | 0.769 |
| 308746:348467 | Human/HeLA | PTEN | 18.4 |
| 355715:343868 | Human/HeLA | Survivin | 0.020825 |
| 331426:308746 | Human/HeLA | PTEN | 0.5627 |
| 331695:308746 | Human/HeLA | PTEN | 0.27688 |
| 332231:308746 | Human/HeLA | PTEN | 5.58 |
| 355712:343868 | Human/HeLA | Survivin | 0.022046 |
| 353538:343868 | Human/HeLA | Survivin | 0.491 |
| 353538:343868 | Human/U87-MG | Survivin | 0.46 |
| 336671:308746 | Human/HeLA | PTEN | 0.273 |
| 336674:308746 | Human/HeLA | PTEN | 0.363 |
| 336675:308746 | Human/HeLA | PTEN | 0.131 |
| 336672:308746 | Human/HeLA | PTEN | 0.428 |
| 336673:308746 | Human/HeLA | PTEN | 0.122 |
| 336676:308746 | Human/HeLA | PTEN | 7.08 |
| 336678:308746 | Human/HeLA | PTEN | 0.144 |
| 352515:343868 | Human/HeLA | Survivin | 0.031541 |
| 330919:308746 | Human/HeLA | PTEN | 29.4 |
| 330997:308746 | Human/HeLA | PTEN | 3.39 |
| 333749:308746 | Human/HeLA | PTEN | 1.3 |
| 333750:308746 | Human/HeLA | PTEN | 0.30815 |
| 333752:308746 | Human/HeLA | PTEN | 1.5416 |
| 333756:308746 | Human/HeLA | PTEN | 1.0933 |
| 334253:308746 | Human/HeLA | PTEN | 0.68552 |
| 353539:343868 | Human/HeLA | Survivin | 0.13216. |

### Example 34: Suitable positional compositions of the invention

The following table describes some suitable positional compositions of the invention. In the listed constructs, the 5'-terminal nucleoside or the sense (upper) strand is hybridized to the 3'-terminal nucleoside of the antisense (lower) strand.

| Compound (sense/antisense) | Construct (sense 5'->3' / antisense) |
|---|---|
| sense RNA | 5'-XXXXXXXXXXXXXXXXXXX-3' |
| 4'thio (bold) dispersed antisense | 3'-XX**X₁₇**XXXX**X₁₂**XX**X₉**XXXXX**X₃X₂X₁**-5' |
| Sense RNA | 5'-XXXXXXXXXXXXXXXXXXX-3' |
| 2'-OMe (italic)/ 4'-thio (bold) dispersed antisense | 3'-*X₁₉X₁₈X₁₇*XXXX**X**XX**X**XXXXXX**XXX**-5' |
| Sense RNA | 5'-XXXXXXXXXXXXXXXXXXXX-3' |
| Chimeric 2'-OMe (italic)/2'-fluoro(bold italic) antisense | 3'-*XXX***XXXXX**XX**XXXXXXXX**-5' |
| Alternate MOE(underline)/OH sense | 5'-XXXXXXXXXXXXXXXXXXX-3' |
| | 3*'-X₂₀X₁₉X₁₈XXXXXXX₁₁X₁₀XXX₇X₆*XXXXX-5' |
| Chimeric OMe (italic) / OH antisense | |
| OMe Gapmer Sense / Chimeric OMe (italic) / OH antisense | 5*'*-X*XXXXXXXXXXXXXXXXX*X-3' |
| | *3'-X₂₀X₁₉X₁₈XXX₁₅XXX₁₂XXXXXX₆*XXXXX-5' |
| Sense RNA | 5'-XXXXXXXXXXXXXXXXXXX-3' |
| Chimeric OMe/OH antisense. | 3'*-XXX₁₇XXX₁₄XXX₁₁*XXX₈XXX₅XXXX-5' |

### Example 35: Alternating 2'-O-MethyV2'-F 20mer siRNAs Targeting PTEN in T-24 cells

A dose response experiment was performed in the PTEN system to examine the positional effects of alternating 2'-O-Methyl/2'-F siRNAs. The nucleosides are annotated as to chemical modification as per the legend at the beginning of the examples.

| **SEQ ID NO. /ISIS NO.** | **Composition (5' to 3')** |
|---|---|
| 40/303912 (as) | UUUGUCUCUGGUCCUUACUU |
| 39/308746 (s) | P-AAGUAAGGACCAGAGACAAA |
| 40/340569 (as) | P-U_{f}UₘU_{f}GₘUₜCₘUₜCₘU_{f}GₘG_{f}UₘC_{f}CₘU_{f}UₘA_{f}CₘU_{f}Uₘ |
| 39/340573 (s) | P-A_{f}AₘG_{f}UₘA_{f}AₘG_{f}GₘA_{f}CₘC_{f}AₘG_{f}AₘG_{f}AₘC_{f}AₘA_{f}Aₘ |
| 40/340569 (as) | P-U_{f}UₘU_{f}GₘU_{f}CₘU_{f}CₘU_{f}GₘG_{f}UₘC_{f}CₘU_{f}UₘA_{f}CₘU_{f}Uₘ |
| 39/340574 (s) | P-AₘAᵣGₘU_{f}AₘA_{f}GₘGᵣAₘC_{f}CₘA_{f}GₘA_{f}GₘA_{f}CₘA_{f}AₘA_{f} |
| 40/340569 (as) | P-U_{f}UₘU_{f}GₘU_{f}CₘU_{f}CₘU_{f}GₘG_{f}UₘC_{f}CₘU_{f}UₘA_{f}CₘU_{f}Uₘ |
| 39/308746 (s) | P-AAGUAAGGACCAGAGACAAA |
| 40/340570 (as) | P-U_{f}UₘU_{f}GₘU_{f}CₘU_{f}CₘU_{f}GₘG_{f}UₘC_{f}CₘU_{f}UₘA_{f}CₘU_{f}Uₘ |
| 39/340573 (s) | P-A_{f}AₘG_{f}UₘA_{f}AₘGₜC_{Tm}AₜCₘC_{t'}AₘG_{f}AₘG_{f}AₘC_{f}AₘA_{f}Aₘ |
| 40/340570 (as) | P-U_{f}UₘU_{f}GₘU_{f}CₘU_{f}CₘU_{f}CₘG_{f}UₘC_{f}CₘU_{f}UₘA_{f}CₘU_{f}Uₘ |
| 39/340574 (s) | P-AₘA_{f}GₘU_{f}AₘA_{f}GₘGᵣAₘC_{f}CₘA_{f}GₘA_{f}GₘA_{f}CₘA_{f}AₘA_{f} |
| 40/340570 (as) | P-U_{f}UₘU_{f}GₘU_{f}CₘU_{f}CₘU_{f}GₘG_{f}UₘC_{f}CₘU_{f}UₘA_{f}CₘU_{f}Uₘ |
| 39/308746 (s) | P-AAGUAAGGACCAGAGACAAA |

The above siRNA constructs were assayed to determine the effects of the full alternating 2'-O-methyl/2'-F antisense strands (PO or PS) where the 5'-terminus of the antisense strands are 2'-F modified nucleosides with the remaining positions alternating. The sense strands were prepared with the positioning of the modified nucleosides in both orientations such that for each siRNA tested with 2'-O-methyl modified nucleosides beginning at the 3'-terminus of the sense strand another identical siRNA was prepared with 2'-F modified nucleosides beginning at the 3'-terminus of the sense strand. Another way to describe the differences between these two siRNAs is that the register of the sense strand is in both possible orientations with the register of the antisense strand being held constant in one orientation. Activity of the constructs (at 150 nM) is presented below as a percentage of untreated control.

| **siRNA** | **Activity (% untreated control 150 nM)** | | |
|---|---|---|---|
| **Construct** | | **Sense** | **Antisense** |
| 308746/303912 | 28% | PO unmodified RNA | PS unmodified RNA |
| 340574/340569 | 46% | PO (2'-F, 3'-0) | PO (2'-F, 5'-0) |
| 340574/340570 | 62% | PO (2'-F, 3'-0) | PS (2'-F, 5'-0) |
| 340573/340569 | 84% | PO (2'-O-methyl; 3'-0) | PO (2'-F, 5'-0) |
| 340573/340570 | 23% | PO (2'-O-methyl, 3'-0) | PS (2'-F, 5'-0) |
| 308746/340569 | 23% | PO unmodified RNA | PO (2'-F, 5'-0) |
| 308746/340570 | 38% | PO unmodified RNA | PS (2'-F, 5'-0) |

Within the alternating motif for this assay the antisense strands were prepared beginning with a 2'-F group at the 5'-terminal nucleoside. The sense strands were prepared with the alternating motif beginning at the 3'-terminal nucleoside with either the 2'-F modified nucleoside or a 2'-O-methyl modified nucleoside. The siRNA constructs were prepared with the internucleoside linkages for the sense strand as full phosphodiester and the internucleoside linkages for the antisense strands as either full phosphodiester or phosphorothioate.

### Example 36: Effect of modified phosphate moieties on alternating 2'-O-methyl/2'-F siRNAs Targeting eIF4E

A dose response was performed targeting eIF4E in HeLa cells to determine the effects of selected terminal groups on activity. More specifically the reduction of eIF4E mRNA in HeLa cells by 19-basepair siRNA containing alternating 2'-OMe/2'-F modifications is shown in this example. The nucleosides are annotated as to chemical modification as per the legend at the beginning of the examples. 5'-P(S) is a 5'-thiophosphate group (5'-O-P(=S)(OH)OH), 5'-P(H) is a 5'-H-phosphonate group (5'-O-P(=O)(H)OH) and 5'-P(CH₃) is a methylphosphonate group (5'-O-P(=O)(CH₃)OH). All of the constructs in this assay were full phosphodiester linked.

HeLa cells were plated at 4000/well and transfected with siRNA in the presence of LIPOFECTIN™ (6µL/mL OPTI-MEM) and treated for about 4 hours, re-fed, lysed the following day and analyzed using real-time PCR methods as described herein. The maximum % reduction is the amount of mRNA reduction compared to untreated control cells at the highest concentration (100 nM), with IC50 indicating the interpolated concentration at which 50% reduction is achieved.

| **SEQ ID NO /ISIS NO** | **SEQUENCES 5'-3' targeted to eIF4E** |
|---|---|
| 26/341391 (as) | UUGUCUCUGGUCCUUACUU |
| 27/341401 (s) | AAGUAAGGACCAGAGACAA |
| 58/342744 (as) | UCUUAUCACCUUUAGCUCU |
| 59/342764 (s) | AGAGCUAAAGGUGAUAAGA |
| 58/351831 (as) | UₘC_{f}UₘU_{f}AₘU_{f}CₘA_{f}CₘC_{f}UₘU_{f}UₘA_{f}GₘC_{f}UₘC_{f}Uₘ |
| 59/351832 (s) | A_{f}GₘA_{f}GₘC_{f}UₘA_{f}AₘA_{f}GₘG_{f}UₘG_{f}AₘU_{f}AₘA_{f}GₘA_{f} |
| 58/368681 (as) | P-UₘC_{f}UₘU_{f}AₘU_{f}CₘA_{f}CₘC_{f}UₘU_{f}UₘA_{f}GₘC_{f}UₘC_{f}Uₘ |
| 59/351832 (s) | A_{f}GₘA_{f}GₘC_{f}UₘA_{f}AₘA_{f}GₘG_{f}UₘG_{f}AₘU_{f}AₘA_{f}GₘA_{f} |
| 58/379225 (as) | P(S)-UₘC_{f}UₘU_{f}AₘU_{f}CₘA_{f}CₘC_{f}UₘU_{f}UₘA_{f}GₘC_{f}UₘC_{f}Uₘ |
| 59/351832 (s) | A_{f}GₘA_{f}GₘC_{f}UₘA_{f}AₘA_{f}GₘG_{f}UₘG_{f}AₘU_{f}AₘA_{f}GₘA_{f} |
| 58/379712 (as) | P(H)-UₘC_{f}UₘU_{f}AmU_{f}CₘA_{f}CₘC_{f}UₘU_{f}UₘA_{f}GₘC_{f}UₘC_{f}Uₘ |
| 59/351832 (s) | A_{f}GₘA_{f}GₘC_{f}UₘA_{f}AₘA_{f}GₘG_{f}UₘG_{f}AₘU_{f}AₘA_{f}GₘA_{f} |
| 58/379226 (as) | P(CH₃)-UₘC_{f}UₘU_{f}AₘU_{f}CₘA_{f}CₘC_{f}UₘU_{f}UₘA_{f}GₘC_{f}UₘC_{f}Uₘ |
| 59/351832 (s) | A_{f}GₘA_{f}GₘC_{f}UₘA_{f}AₘA_{f}GₘG_{f}UₘG_{f}AₘU_{f}AₘA_{f}GₘA_{f} |

| **Double stranded construct** | | **Activity** | |
|---|---|---|---|
| **Antisense** | **Sense** | **% Control (100 nM)** | **IC50** (nM) |
| 341401 | 341391 | 103 | n/a (neg control) |
| 342764 | 342744 | 11.0 | 1.26 |
| 351832 | 351831 | 3.5 | 0.66 |
| 351832 | 368681 | 3.6 | 0.14 |
| 351832 | 379225 | 2.8 | 0.20 |
| 351832 | 379712 | 8.0 | 2.01 |
| 351832 | 379226 | 18.1 | 8.24. |

### Example 37: Assay of selected siRNAs targeting PTEN

The constructs listed below were assayed for activity by measuring the levels of human PTEN mRNA in HeLa cells against untreated control levels. The nucleosides are annotated as to chemical modification as per the legend at the beginning of the examples. "P(S)-" indicates a thiophosphate group (-O-P(=S)(OH)OH).

| **SEQ ID NO /ISIS NO** | **SEQUENCES 5'-3' targeted to PTEN** |
|---|---|
| 26/371789 (as) | P-UUGUCUCUGGUCCUUACUU |
| 27/341401 (s) | P-AAGUAAGGACCAGAGACAA |
| 26/383498 (as) | UₘU_{f}GₘU_{f}CₘU_{f}CₘU_{f}GₘG_{f}UₘC_{f}CₘU_{f}UₘA_{f}CₘU_{f}Uₘ |
| 27/359351 (s) | AₑAₑGₑUAAGGACCAGAGACₑAₑAₑ |
| 26/381671 (as) | P-UₘU_{f}GₘU_{f}CₘU_{f}CₘU_{f}GₘG_{f}UₘC_{f}CₘU_{f}UₘA_{f}CₘU_{f}Uₘ |
| 27/359351 (s) | AₑAₑGₑUAAGGACCAGAGACₑAₑAₑ |
| 26/382716 (as) | P(S)-UₘU_{f}GₘU_{f}CₘU_{f}CₘU_{f}GₘG_{f}UₘC_{f}CₘU_{f}UₘA_{f}CₘU_{f}Uₘ |
| 27/359351 (s) | AₑAₑGₑUAAGGACCAGAGACₑAₑAₑ |
| 26/381672 (as) | P-UₘU_{f}GₘU_{f}CₘU_{f}CₘU_{f}GₘG_{f}UₘC_{f}CₘU_{f}UₘA_{f}CₘU_{f}Uₘ |
| 27/359351 (s) | AₑAₑGₑUAAGGACCAGAGACₑAₑAₑ |
| 26/384758 (as) | P(S)-UₜUₜGUCUₘCₘUGGₘUₘCCUUACₘUₘUₘ |
| 27/359351 (s) | AₑAₑGₑUAAGGACCAGAGACₑAₑAₑ |
| 26/384759 (as) | P(S)-UₜUₜGUCUₘCₘUGGₘUₘCCUUACₘUₘUₘ |
| 27/359351 (s) | AₑAₑGₑUAAGGACCAGAGACₑAₑAₑ |
| 26/384760 (as) | P(S)-UₜUₜGUCUCUGGₘUₘCCUUACₘUₘUₘ |
| 27/359351 (s) | AₑAₑGₑUAAGGACCAGAGACₑAₑAₑ |
| 26/384761 (as) | P(S)-UₜUₜGUCUCUGGₘUₘCCUUACₘUₘUₘ |
| 27/359351 (s) | AₑAₑGₑUAAGGACCAGAGACₑAₑAₑ |
| 26/359455 (as) | UUGUCUCUGGUCCUUACUU |
| 27/359351 (s) | AₑAₑGₑUAAGGACCAGAGACₑAₑAₑ |
| 26/384754 (as) | P(S)-UUGUCUₘCₘUGGₘUₘCCUUACₘUₘUₘ |
| 27/359351 (s) | AₑAeGₑUAAGGACCAGAGACₑAₑAₑ |
| 26/384755 (as) | P(S)-UₜUₜGUCUCUGGUCCUUACₘUₘUₘ |
| 27/359351 (s) | AₑAₑGₑUAAGGACCAGAGACₑAₑAₑ |
| 26/384756 (as) | P(S)-UₜUₜGUCUCUGGUCCUUACₘUₘUₘ |
| 27/359351 (s) | AₑAₑGₑUAAGGACCAGAGACₑAₑAₑ |
| 26/384757 (as) | UₜUₜGUCUₘCₘUGGₘUₘCCUUACₘUₘUₘ |
| 27/359351 (s) | AₑAₑGₑUAAGGACCAGAGACₑAₑAₑ |
| 26/359455 (as) | UUGUCUCUGGUCCUUACUU |
| 27/384762 (s) | AₑAₑGₑUAAGGACCAGAGACₜAₜAₜ |
| 26/384754 (as) | P(S)-UUGUCUₘCₘUGGₘUₘCCUUACₘUₘUₘ |
| 27/384762 (s) | AₑAₑGₑUAAGGACCAGAGACₜAₜAₜ |
| 26/384755 (as) | P(S)-UₜUₜGUCUCUGGUCCUUACₘUₘUₘ |
| 27/384762 (s) | AₑAₑGₑUAAGGACCAGAGACₜAₜAₜ |
| 26/384756 (as) | P(S)-UₜUₜGUCUCUGGUCCUUACₘUₘUₘ |
| 27/384762 (s) | AₑAₑGₑUAAGGACCAGAGACₜAₜAₜ |
| 26/384757 (as) | UₜUₜGUCUₘCₘUGGₘUₘCCUUACₘUₘUₘ |
| 27/384762 (s) | AₑAₑGₑUAAGGACCAGAGACₜAₜAₜ |
| 26/383498 (as) | UₘU_{f}GₘU_{f}CₘU_{f}CₘU_{f}GₘG_{f}UₘC_{f}CₘU_{f}UₘA_{f}CₘU_{f}Uₘ |
| 27/384762 (s) | AₑAₑGₑUAAGGACCAGAGACₜAₜAₜ |
| 26/381671 (as) | P-UₘU_{f}GₘU_{f}CₘU_{f}CₘU_{f}GₘG_{f}UₘC_{f}CₘU_{f}UₘA_{f}CₘU_{f}Uₘ |
| 27/384762 (s) | AₑAₑGₑUAAGGACCAGAGACₜAₜAₜ |
| 26/382716 (as) | P(S)-UₘU_{f}GₘU_{f}CₘU_{f}CₘU_{f}GₘG_{f}UₘC_{f}CₘU_{f}UₘA_{f}CₘU_{f}Uₘ |
| 27/384762 (s) | AₑAeGₑUAAGGACCAGAGACₜAₜAₜ |
| 26/381672 (as) | P-UₘU_{f}GₘU_{f}CₘU_{f}CₘU_{f}GₘG_{f}UₘC_{f}CₘU_{f}UₘA_{f}CₘU_{f}Uₘ |
| 27/384762 (s) | AₑAₑGₑUAAGGACCAGAGACₜAₜAₜ |
| 26/384758 (as) | P(S)-UₜUₜGUCUₘCₘUGGₘUₘCCUUACₘUₘUₘ |
| 27/384762 (s) | AₑAₑGₑUAAGGACCAGAGACₜAₜAₜ |
| 26/384759 (as) | P(S)-UₜUₜGUCUₘCₘUGGₘUₘCCUUACₘUₘUₘ |
| 27/3 84762 (s) | AₑAₑGₑUAAGGACCAGAGACₜAₜAₜ |
| 26/384760 (as) | P(S)-UₜUₜGUCUCUGGₘUₘCCUUACₘUₘUₘ |
| 27/384762 (s) | AₑAₑGₑUAAGGACCAGAGACₜAₜAₜ |
| 26/384761 (as) | P(S)-UₜUₜGUCUCUGGₘUₘCCUUACₘUₘUₘ |
| 27/384762 (s) | AₑAₑG_{c}AAGGACCAGAGACₜAₜAₜ |
| 26/384758 (as) | P(S)-UₜUₜGUCUₘCₘUGGₘUₘCCUUACₘUₘUₘ |
| 27/366023 (s) | A_{f}AₘG_{f}U_{f}A_{f}AₘG_{f}GₘA_{f}CₘC_{f}AₘG_{f}AₘG_{f}AₘC_{f}AₘA_{f} |
| 26/384759 (as) | P(S)-UₜUₜGUCUₘCₘUGGₘUₘCCUUACₘUₘUₘ |
| 27/366023 (s) | A_{f}AₘG_{f}UₘA_{f}AₘG_{f}GₘA_{f}CₘAₘG_{f}AₘG_{f}AₘC_{f}AₘA_{f} |
| 26/384760 (as) | P(S)-UₜUₜGUCUCUGGₘUₘCCUUACₘUₘUₘ |
| 27/366023 (s) | A_{f}AₘG_{f}UₘA_{f}AₘG_{f}GₘA_{f}CₘC_{f}AₘG_{f}AₘG_{f}AₘC_{f}A_{mA}f |
| 26/384761 (as) | P(S)-UtU,GUCUCUGGₘU",CCUUACₘUₘUₘ, |
| 27/366023 (s) | A_{f}AₘG_{f}UₘA_{f}AₘG_{f}G_{f}A_{f}CₘC_{f}AₘG_{f}AₘG_{f}AₘC_{f}AₘA_{f} |
| 26/384754 (as) | P(S)-UUGUCUₘCₘUGGₘUₘCCUUACₘUₘUₘ |
| 27/359351 (s) | A_{f}AₘG_{f}UₘA_{f}AₘG_{f}GA_{f}CₘC_{f}AₘG_{f}AₘG_{f}AₘC_{f}AₘA_{f} |
| 26/384755 (as) | P(S)-UₜUₜGUCUCUGGUCCUUACₘUₘUₘ |
| 27/359351 (s) | A_{f}AₘG_{f}UₘA_{f}AₘG_{f}GₘA_{f}CₘC_{f}AₘG_{f}AₘG_{f}AₘC_{f}AₘA_{f} |
| 26/384756 (as) | P(S)-UₜUₜGUCUCUGGUCCUUACₘUₘUₘ |
| 27/359351 (s) | A_{f}AₘG_{f}UₘA_{f}AₘG_{f}GₘAₘC_{f}AₘG_{f}AₘG_{f}AₘC_{f}AₘA_{f} |
| 26/384757 (as) | UₜUₜGUCUₘCₘUGGₘUₘUUACₘUₘUₘ |
| 27/359351 (s) | A_{f}AₘG_{f}UₘA_{f}AₘG_{f}GₘA_{f}CₘC_{f}AₘG_{f}AₘG_{f}AₘC_{f}AₘA_{f} |
| 26/359345 (as) | UₜUₜGUCUCUGGUCCUUACUₜUₜ |
| 27/384762 (s) | AₑAₑGₑUAAGGACCAGAGACₜAₜAₜ |
| 26/381671 (as) | UₜUₜGUCUCUGGUCCUUACₘUₘUₘ |
| 27/384762 (s) | AₑAₑGₑUAAGGACCAGAGACₜAₜAₜ |
| 26/352820 (as) | P-UₘU_{f}GₘU_{f}CₘU_{f}CₘU_{f}GₘG_{f}UₘC_{f}CₘU_{f}UₘA_{f}CₘU_{f}Uₘ |
| 27/384762 (s) | AₑAₑGₑUAAGGACCAGAGACₜAₜAₜ |
| 26/352820 (as) | P-UₘU_{f}GₘU_{f}CₘU_{f}GₘG_{f}UₘC_{f}CₘU_{f}UₘA_{f}CₘU_{f}Uₘ |
| 27/359351 (s) | AₑAₑGₑUAAGGACCAGAGACₑAₑAₑ |
| 26/384754 (as) | P(S)-UUGUCUₘCₘUGGₘUₘCCUUACₘUₘUₘ |
| 27/359351(s) | A_{f}AₘG_{f}UₘA_{f}AₘG_{f}GₘA_{f}CₘC_{f}AₘG_{f}AₘG_{f}AₘC_{f}AₘA_{f} |

| **Double stranded construct** | | **Activity** |
|---|---|---|
| **Antisense** | **Sense** | **IC50** (nM) |
| 341391 | 341401 | 0.152 |
| 359980 | 359351 | 0.042 |
| 384758 | 359351 | 0.095 |
| 384759 | 359351 | 0.08 |
| 384760 | 359351 | 0.133 |
| 384761 | 359351 | 0.13 |
| 384754 | 359351 | 0.203 |
| 384757 | 359351 | 0.073 |
| 352820 | 359351 | 0.214 |
| 359980 | 384762 | 0.16 |
| 384754 | 384762 | 0.245 |
| 384755 | 384762 | 0.484 |
| 384756 | 384762 | 0.577 |
| 384757 | 384762 | 0.131 |
| 384758 | 384762 | 0.361 |
| 384759 | 384762 | 0.332 |
| 384760 | 384762 | 0.566 |
| 384761 | 384762 | 0.362 |
| 359345 | 384762 | 0.155 |
| 359346 | 384762 | 0.355 |
| 352820 | 384762 | 0.474. |

### Example 38: Alternating 2'-MOE/2'-OH siRNAs Targeting PTEN

The constructs listed below targeting PTEN were duplexed as shown (antisense strand followed by the sense strand of the duplex) and assayed for activity using methods described herein. The nucleosides are annotated as to chemical modification as per the legend at the beginning of the examples.

| **SEQ ID NO /ISIS NO** | **SEQUENCES 5'-3' targeted to PTEN** | **IC50** (nM) |
|---|---|---|
| 27/355771 (s) | P-AAₑGUₑAAₑGGeACₑCAₑGAₑGAₑCAₑA | 273 |
| 40/357276 (as) | P-UUUGₑUCUCₑUGGUCCUUₑACUU | |
| 27/355771 (s) | P-AAₑGUₑAAₑGGₑACₑCAₑGAₑGAₑCAₑA | 5.5 |
| 40/357276 (as) | P-UUUGₑUCUCUGGₑUCCUUACUₑU. | |

### Example 39: In vitro assay of selected differentially modified siRNAs targeted to PTEN

The constructs listed below are being prepared and will be assayed for activity by measuring the levels of human PTEN mRNA in HeLa cells against untreated control levels. The The nucleosides are annotated as to chemical modification as per the legend at the beginning of the examples.

| **SEQ ID NO /ISIS NO** | **SEQUENCES 5'-3' targeted to PTEN** |
|---|---|
| 64/398239 (s) | A_{f}AₘG_{f}UₘA_{f}AₘG_{f}GₘA_{f}GₘC_{f}AₘG_{f}AₘG_{f}AₘC_{f}AₘA_{f}UₑUₑ |
| 65/398259 (as) | P-U_{f}U_{f}G_{f}U_{f}C_{f}UₘCₘU_{f}G_{f}GₘUₘC_{f}C_{f}U_{f}U_{f}A_{f}CₘUₘUₘUₑUₑ |
| 65/398260 (as) | P-UₜUₜG_{f}U_{f}C_{f}UₘCₘU_{f}G_{f}GₘUₘC_{f}C_{f}U_{f}U_{f}A_{f}CₘUₘUₘUₑUₑ |
| 65/398266 (as) | P-U_{f}U_{f}G_{f}U_{f}C_{f}UₘCₘU_{f}G_{f}GₘUₘC_{f}C_{f}U_{f}U_{f}A_{f}CₘUₘUₘUₑUₑ |

| **SEQ ID NO /ISIS NO** | | **IC50** (nM) |
|---|---|---|
| **Sense** | **antisense** | |
| 64/398239 (s) | 65/398259 (as) | 0.15 |
| 64/398239 (s) | 65/398260 (as) | 0.15 |
| 64/398239 (s) | 65/398266 (as) | not determined. |

### Example 40: In vitro assay of selected differentially modified siRNAs targeted to eIF4E

In accordance with the present invention, a series of oligomeric compounds were synthesized and tested for their ability to reduce eIF4E expression over a range of doses. The nucleosides are annotated as to chemical modification as per the legend at the beginning of the examples. HeLa cells were treated with the double stranded oligomeric compounds (siRNA constructs transfected with Lipofectamine 2000 for 4 hours) shown below at concentrations of 0.008 nM, 0.04 nM, 0.2 nM, 1.0 nM or 5 nM using methods described herein. Expression levels of human eIF4E were determined using real-time PCR methods as described herein (purified RNA for RT-PCR analysis 16 hours after transfection - HTS4890). Resulting dose-response curves were used to determine the IC50 for each pair as shown below.

| **SEQ ID NO /ISIS NO** | **SEQUENCES 5'-3' targeted to eIF4E** |
|---|---|
| 66/400539 (s) | GₘU_{f}GₘA_{f}CₘU_{f}AₘC_{f}UₘU_{f}UₘU_{f}UₘA_{f}AₘUₑUₑ |
| 66/400733 (s) | _{d}GU_{ed}GA_{ed}CU_{ed}AC_{ed}UC_{ed}AC_{ed}UU_{ed}UU_{ed}UU_{ed}AUₑUₑ |
| 35/400734 (s) | _{d}GU_{ed}GA_{ed}CU_{ed}AC_{ed}UC_{ed}AC_{ed}UU_{ed}UU_{ed}UA_{ed}A-L-C₁₆ |
| 35/400540 (s) | GₘU_{f}GₘA_{f}CₘU_{f}AₘC_{f}UₘC_{f}AₘC_{f}UₘU_{f}UₘU_{f}UₘA_{f}Aₘ-L-C₁₆ |
| 67/400546 (as) | U_{f}U_{f}A_{f}A_{f}A_{f}AₘAₘG_{f}U_{f}GₘAₘG_{f}U_{f}A_{f}G_{f}U_{f}CₘAₘCₘUₑUₑ |

| **SEQ ID NO /ISIS NO** | | **IC50** (pM) |
|---|---|---|
| **Sense** | **antisense** | |
| 66/400539 (s) | 67/400546 (as) | 0.040 |
| 66/400733 (s) | 67/400546 (as) | 0.031 |
| 35/400734 (s) | 67/400546 (as) | not determined |
| 35/400540 (s) | 67/400546 (as) | 0.052 |

The sense strands 400734 and 400540 have a C₁₆ lipophilic conjugate group attached at the 3'-end via a bivalent linking group (L) which is further attached to a phosphorothioate group. The structure of the entire group which is attached to the 3'-carbon of the terminal nucleoside is shown below:

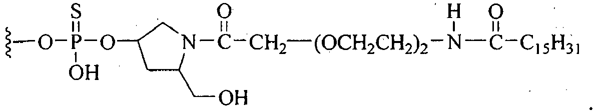

### Example 41: In vitro assay of selected differentially modified siRNAs targeted to eIF4E

In accordance with the present invention, a series of oligomeric compounds were synthesized and tested for their ability to reduce eIF4E expression over a range of doses. The nucleosides are annotated as to chemical modification as per the legend at the beginning of the examples. HeLa cells were treated with the double stranded oligomeric compounds shown below at concentrations of 0.0 nM, 0.0032 nM, 0.016 nM, 0.08 nM, 0.4 nM, 2.0 nM or 10 nM using methods described herein. Expression levels of human eIF4E were determined using real-time PCR methods as described herein. Resulting dose-response curves were used to determine the IC50 for each pair as shown below.

| **SEQ ID NO /ISISNO** | **SEQUENCES 5'-3' targeted to eIF4E** |
|---|---|
| 35/371303 (s) | _{d}GU_{ed}GA_{ed}CU_{ed}AC_{ed}UC_{ed}AC_{ed}UU_{ed}UU_{ed}UA_{ed}A |
| 35/385868 (s) | GU_{ed}GA_{ed}CU_{ed}AC_{ed}UC_{ed}AC_{ed}UU_{ed}UU_{ed}U_{Ae}Aₑ |
| 35/385869 (s) | GₑUₑG_{ed}A_{d}C_{d}U_{d}A_{d}C_{d}U_{d}C_{d}A_{d}C_{d}U_{d}U_{d}U_{d}UUₜAₜAₜ |
| 35/371306 (s) | GₑUₑGe_{d}A_{d}CdU_{d}A_{d}C_{d}U_{d}C_{d}A_{d}C_{d}U_{d}U_{d}U_{d}UUₑAₑAₑ |
| 34/385866 (as) | P-U_{f}U_{f}A_{f}A_{f}A_{f}A_{f}AₘG_{f}U_{f}GₘAₘG_{f}U_{f}A_{f}G_{f}U_{f}CₘAₘCₘ |
| 34/385867 (as) | P-UₜUₜA_{f}A_{f}A_{f}AₘAₘG_{f}U_{f}GₘAₘG_{f}U_{f}A_{f}G_{f}U_{f}CₘAₘCₘ |

| **SEQ ID NO /ISIS NO** | | **IC50** (nM) |
|---|---|---|
| **Sense** | **antisense** | |
| 35/371303 (s) | 34/385866 (as) | 0.017 |
| 35/385868 (s) | 34/385866 (as) | 0.026 |
| 35/371303 (s) | 34/385867 (as) | 0.016 |
| 35/385868 (s) | 34/385867 (as) | 0.043 |
| 35/385869 (s) | 34/385866 (as) | 0.052 |
| 35/371306 (s) | 34/385866 (as) | 0.071 |
| 35/385869 (s) | 34/385867 (as) | 0.086 |
| 35/371306 (s) | 34/385867 (as) | 0.037. |

### Example 42: Chemically modified siRNA targeted to PTEN: in vivo study

Six- to seven-week old Balb/c mice (Jackson Laboratory, Bar Harbor, ME) were injected with single strand and double strand compositions targeted to PTEN. The nucleosides are annotated as to chemical modification as per the legend at the beginning of the examples. Each treatment group was comprised of four animals. Animals were dosed via intraperitoneal injection twice per day for 4.5 days, for a total of 9 doses per animal. Saline-injected animals served as negative controls. Animals were sacrificed 6 hours after the last dose was administered, and plasma samples and tissues were harvested. Target reduction in liver was also measured at the conclusion of the study.

| **SEQ ID NO /ISIS NO** | **SEQUENCES 5'-3' targeted to eIF4E** |
|---|---|
| 63/116847 | CₑTₑGₑCₑTPAGCCTCTGGATₑTₑTₑGₑAₑ single strand |
| 26/341391 (as) | UUGUCUCUGGUCCUUACUU |
| 27/341401 (s) | AAGUAAGGACCAGAGACAA |
| 26/359995 (as) | UₘU_{f}GₘU_{f}CₘU_{f}CₘU_{f}GₘG_{f}UₘC_{f}CₘU_{f}UₘA_{f}CₘU_{f}Uₘ |
| 27/359996 (s) | A_{f}AₘG_{f}UₘA_{f}AₘG_{f}GₘA_{f}CₘC_{f}AₘG_{f}AₘG_{f}AₘC_{f}AₘA_{f} |

Two different doses of each treatment were tested. Treatment with ISIS 116847, was administered at doses of 12.5 mg/kg twice daily or at 6.25 mg/kg twice daily.

The siRNA constructs described above (unmodified 341391/341401, 359995/359996 both strands modified) were administered at doses of 25 mg/kg twice daily or 6.25 mg/kg twice daily. Each siRNA is composed of an antisense strand and a complementary sense strand as per previous examples, with the antisense strand targeted to mouse PTEN. ISIS 116847 and all of the siRNAs of this experiment also have perfect complementarity with human PTEN.

PTEN mRNA levels in liver were measured at the end of the study using real-time PCR and RIBOGREEN™ RNA quantification reagent (Molecular Probes, Inc. Eugene, OR) as taught in previous examples above. Results are presented in the table below as the average % inhibition of mRNA expression for each treatment group, normalized to saline-injected control.

**Target reduction by modified siRNAs targeted to PTEN in mouse liver**

| **Treatment** | **Dose (mg/kg, administered 2x/day)** | **% Inhibition** | |
|---|---|---|---|
| | | **Ribogreen** | **GAPDH** |
| **ISIS 116847** | 12.5 | 92 | 95 |
| | 6.25 | 92 | 95 |
| **ISIS 341391/341401** | 25 | 12 | 21 |
| | 6.25 | 2 | 9 |
| **ISIS 359995/359996** | 25 | 6 | 13 |
| | 6.25 | 5 | 13 |

As shown in the Table above, all oligonucleotides targeted to PTEN caused a reduction in mRNA levels in liver as compared to saline-treated control. The mRNA levels measured for the ISIS 341391/341401 duplex are also suggestive of dose-dependent inhibition.

The effects of treatment with the RNA duplexes on plasma glucose levels were evaluated in the mice treated as described above. Glucose levels were measured using routine clinical analyzer instruments (eg. Ascencia Glucometer Elite XL, Bayer, Tarrytown, NY). Approximate average plasma glucose is presented in the Table below for each treatment group.

### Effects of modified siRNAs targeted to PTEN on plasma glucose levels in normal mice

| **Treatment** | **Dose (mg/kg, administered 2x/day)** | **Plasma glucose (mg/dL)** |
|---|---|---|
| **Saline** | N/A | 186 |
| **ISIS 116847** | 12.5 | 169 |
| | 6.25 | 166 |
| **ISIS 341391/341401** | 25 | 159 |
| | 6.25 | 182 |
| **ISIS 359996/359995** | 25 | 182 |
| | 625 | 169 |

To assess the physiological effects resulting from in vivo siRNA targeted to PTEN mRNA, the mice were evaluated at the end of the treatment period for plasma triglycerides, plasma cholesterol, and plasma transaminase levels. Routine clinical analyzer instruments (eg. Olympus Clinical Analyzer, Melville, NY) were used to measure plasma triglycerides, cholesterol, and transaminase levels. Plasma cholesterol levels from animals treated with either dose of ISIS 116847 were increased about 20% over levels measured for saline-treated animals. Conversely, the cholesterol levels measured for animals treated with either the 25 mg/kg or the 6.25 mg/kg doses of the ISIS 341391/341401 duplex were decreased about 12% as compared to saline-treated controls. The ISIS 359996/359995 duplex did not cause significant alterations in cholesterol levels. All of the treatment groups showed decreased plasma triglycerides as compared to saline-treated control, regardless of treatment dose.

Increases in the transaminases ALT and AST can indicate hepatotoxicity. The transaminase levels measured for mice treated with the siRNA duplexes were not elevated to a level indicative of hepatotoxicity with respect to saline treated control. Treatment with 12.5 mg/kg doses of ISIS 116847 caused approximately 7-fold and 3-fold increases in ALT and AST levels, respectively. Treatment with the lower doses (6.25 mg/kg) of ISIS 116847 caused approximately 4-fold and 2-fold increases in ALT and AST levels, respectively.

At the end of the study, liver, white adipose tissue (WAT), spleen, and kidney were harvested from animals treated with the oligomeric compounds and were weighed to assess gross organ alterations. Approximate average tissue weights for each treatment group are presented in the table below.

**Effects of chemically modified siRNAs targeted to PTEN on tissue weight in normal mice**

| **Treatment** | **Dose (mg/kg, administered 2x/day)** | **Liver** | **WAT** | **Spleen** | **Kidney** |
|---|---|---|---|---|---|
| | | **Tissue weight (g)** | | | |
| **Saline** | N/A | 1.0 | 0.5 | 0.1 | 0.3 |
| **ISIS 116847** | 12.5 | 1.1 | 0.4 | 0.1 | 0.3 |
| | 6.25 | 1.1 | 0.4 | 0.1 | 0.3 |
| **ISIS 341391/341401** | 25 | 1.0 | 0.3 | 0.1 | 0.3 |
| | 6.25 | 0.9 | 0.4 | 0.1 | 0.3 |
| **ISIS 359996/359995** | 25 | 1.1 | 0.4 | 0.1 | 0.3 |
| | 6.25 | 1.0 | 0.3 | 0.1 | 0.4 |

As shown, treatment with antisense oligonucleotides or siRNA duplexes targeted to PTEN did not substantially alter liver, WAT, spleen, or kidney weights in normal mice as compared to the organ weights of mice treated with saline alone.

### Example 43: Chemically modified siRNA targeted to PTEN: in vivo study

Six- to seven-week old Balb/c mice (Jackson Laboratory, Bar Harbor, ME) were injected with compounds targeted to PTEN. Each treatment group was comprised of four animals. Animals were dosed via intraperitoneal injection twice per day for 4.5 days, for a total of 9 doses per animal. Saline-injected animals served as negative controls. Animals were sacrificed 6 hours after the last dose of oligonucleotide was administered, and plasma samples and tissues were harvested. Target reduction in liver was also measured at the conclusion of the study.

Two doses of each treatment were tested. Treatment with ISIS 116847 (5'-CTGCTAGCCTCTGGATTTGA-3', SEQ ID NO: 63), a 5-10-5 gapmer was administered at doses of 12.5 mg/kg twice daily or at 6.25 mg/kg twice daily. The siRNA compounds described below were administered at doses of 25 mg/kg twice daily or 6.25 mg/kg twice daily. Each siRNA is composed of an antisense and complement strand as described in previous examples, with the antisense strand targeted to mouse PTEN. ISIS 116847 and all of the siRNAs of this experiment also have perfect complementarity with human PTEN.

An siRNA duplex targeted to PTEN is comprised of antisense strand ISIS 341391 (5'-UUGUCUCUGGUCCUUACUU-3', SEQ ID NO: 26) and the sense strand ISIS 341401 (5'-AAGUAAGGACCAGAGACAA-3', SEQ ID NO: 27). Both strands of the ISIS 341391/341401 duplex are comprised ofribonucleosides with phosphodiester internucleoside linkages.

Another siRNA duplex targeted to human PTEN is comprised of antisense strand ISIS 342851 (5'-**UUU**G**U**CUCUGGUCCU**U**A**CUU**-3', SEQ ID NO: 40) and the sense strand ISIS 308746 (5'-AAGUAAGGACCAGAGACAAA-3', SEQ ID NO: 39). The antisense strand, ISIS 342851, is comprised of a central RNA region with 4'-thioribose nucleosides at positions 1, 2, 3, 5, 16, 18, 19, and 20, indicated in **bold.** The sense strand, ISIS 308746, is comprised of ribonucleosides, and both strands of the ISIS 342851/308746 duplex have phosphodiester internucleoside linkages throughout.

PTEN mRNA levels in liver were measured at the end of the study using real-time PCR and RIBOGREEN™ RNA quantification reagent (Molecular Probes, Inc. Eugene, OR) as taught in previous examples above. PTEN mRNA levels were determined relative to total RNA or GAPDH expression, prior to normalization to saline-treated control. Results are presented in the following table as the average % inhibition of mRNA expression for each treatment group, normalized to saline-injected control.

**Target reduction by chemically modified siRNAs targeted to PTEN in mouse liver •**

| **Treatment** | **Dose (mg/kg, administered 2x/day)** | **% Inhibition** | |
|---|---|---|---|
| | | **Ribogreen** | **GAPDH** |
| **ISIS 116847** | 12.5 | 92 | 95 |
| | 6.25 | 92 | 95 |
| **ISIS 342851/308746** | 25 | 11 | 18 |
| | 6.25 | 7 | 15 |
| **ISIS 341391/341401** | 25 | 12 | 21 |
| | 6.25 | 2 | 9 |

As shown in the table, the oligonucleotides targeted to PTEN decreased mRNA levels relative to saline-treated controls. The mRNA levels measured for the ISIS 341391/341401 duplex are also suggestive of dose-dependent inhibition.

The effects of treatment with the RNA duplexes on plasma glucose levels were evaluated in the mice treated as described above. Glucose levels were measured using routine clinical analyzer instruments (eg. Ascencia Glucometer Elite XL, Bayer, Tarrytown, NY). Approximate average plasma glucose is presented in the following table for each treatment group.

**Effects of chemically modified siRNAs targeted to PTEN on plasma glucose levels in normal mice**

| **Treatment** | **Dose (mg/kg, administered 2x/day)** | **Plasma glucose (mg/dL)** |
|---|---|---|
| **Saline** | N/A | 186 |
| **ISIS 116847** | 12.5 | 169 |
| | 6.25 | 166 |
| **ISIS 342851/308746** | 25 | 167 |
| | 6.25 | 173 |
| **ISIS 341391/341401** | 25 | 159 |
| | 6.25 | 182 |

To assess the physiological effects resulting from in vivo siRNA targeted to PTEN mRNA, the mice were evaluated at the end of the treatment period for plasma triglycerides, plasma cholesterol, and plasma transaminase levels. Routine clinical analyzer instruments (eg. Olympus Clinical Analyzer, Melville, NY) were used to measure plasma triglycerides, cholesterol, and transaminase levels. Plasma cholesterol levels from animals treated with either dose of ISIS 116847 were increased about 20% over levels measured for saline-treated animals. Conversely, the cholesterol levels measured for animals treated with either the 25 mg/kg or the 6.25 mg/kg doses of the ISIS 341391/341401 duplex were decreased about 12% as compared to saline-treated controls. The other treatments did not cause substantial alterations in cholesterol levels. All of the treatment groups showed decreased plasma triglycerides as compared to saline-treated control, regardless of treatment dose.

Increases in the transaminases ALT and AST can indicate hepatotoxicity. The transaminase levels measured for mice treated with the siRNA duplexes were not elevated to a level indicative of hepatotoxicity with respect to saline treated control. Treatment with 12.5 mg/kg doses of ISIS 116847 caused approximately 7-fold and 3-fold increases in ALT and AST levels, respectively. Treatment with the lower doses (6.25 mg/kg) of ISIS 116847 caused approximately 4-fold and 2-fold increases in ALT and AST levels, respectively.

At the end of the study, liver, white adipose tissue (WAT), spleen, and kidney were harvested from animals treated with the oligomeric compounds and were weighed to assess gross organ alterations. Approximate average tissue weights for each treatment group are presented in the following table.

**Effects of chemically modified siRNAs targeted to PTEN on tissue weight in normal mice**

| **Treatment** | **Dose (mg/kg, administered 2x/day)** | **Liver** | **WAT** | **Spleen** | **Kidney** |
|---|---|---|---|---|---|
| | | **Tissue weight (g)** | | | |
| **Saline** | **N/A** | 1.0 | 0.5 | 0.1 | 0.3 |
| **ISIS 116847** | **12.5** | 1.1 | 0.4 | 0.1 | 0.3 |
| | **6.25** | 1.1 | 0.4 | 0.1 | 0.3 |
| **ISIS 342851/308746** | **25** | 1.0 | 0.3 | 0.1 | 0.3 |
| | **6.25** | 0.9 | 0.4 | 0.1 | 0.3 |
| **ISIS 341391/341401** | **25** | 1.0 | 0.3 | 0.1 | 0.3 |
| | **6.25** | 0.9 | 0.4 | 0.1 | 0.3 |

As shown, treatment with antisense oligonucleotides or siRNA duplexes targeted to PTEN did not substantially alter liver, WAT, spleen, or kidney weights in normal mice as compared to the organ weights of mice treated with saline alone.

### Example 44: Stability of alternating 2'-O-methyl/2'-fluoro siRNA constructs in mouse plasma

Intact duplex RNA was analyzed from diluted mouse-plasma using an extraction and capillary electrophoresis method similar to those previously described (Leeds et al., Anal. Biochem., 1996, 235, 36-43; Geary, Anal. Biochem., 1999, 274, 241-248. Heparin-treated mouse plasma, from 3-6 month old female Balb/c mice (Charles River Labs) was thawed from - 80 °C and diluted to 25% (v/v) with phosphate buffered saline (140 mM NaCl, 3 mM KCl, 2 mM potassium phosphate, 10 mM sodium phosphate). Approximately 10 nmol of pre-annealed siRNA, at a concentration of 100 µM, was added to the 25% plasma and incubated at 37 °C for 0, 15, 30, 45, 60, 120, 180, 240, 360, and 420 minutes. Aliquots were removed at the indicated time, treated with EDTA to a final concentration of 2 mM, and placed on ice at 0 °C until analyzed by capillary gel electrophoresis (Beckman P/ACE MDQ-UV with eCap DNA Capillary tube). The area of the siRNA duplex peak was measured and used to calculate the percent of intact siRNA remaining. Adenosine triphosphate (ATP) was added at a concentration of 2.5 mM to each injection as an internal calibration standard. A zero time point was taken by diluting siRNA in phosphate buffered saline followed by capillary electrophoresis. Percent intact siRNA was plotted against time, allowing the calculation of a pseudo first-order half-life. Results are shown in the Table below. ISIS 338918 (UCUUAUCACCUUUAGCUCUA, SEQ ID NO: 54) and ISIS 338943 are unmodified RNA strand with phosphodiester linkages throughout. ISIS 351831 is annotated as UₘC_{f}UₘU_{f}AₘU_{f}CₘAtCₘC_{f}UₘU_{f}UₘAtGₘC_{f}UₘC_{f}Uₘ (SEQ ID NO: 58) and ISIS 351832 as A_{f}GₘA_{f}GₘC_{f}UₘA_{f}AₘA_{f}GₘG_{f}UₘG_{f}AₘU_{f}AₘA_{f}GₘA_{f} (SEQ ID NO: 59) in other examples herein.

**Stability of alternating 2'-O-methyl/2'-fluoro siRNA constructs in mouse plasma**

| **Construct** | **SEQ ID NOs** | **% Intact siRNA** | | | | | | | | |
|---|---|---|---|---|---|---|---|---|---|---|
| | | **Time (minutes)** | | | | | | | | |
| | | **0** | **15** | **30** | **45** | **60** | **120** | **180** | **240** | **360** |
| 338918/ 338943 | 54 and 55 | 76.98 | 71.33 | 49.77 | 40.85 | 27.86 | 22.53 | 14.86 | 4.18 | 0 |
| 351831/ 351832 | 58 and 59 | 82.42 | 81.05 | 79.56 | 77.64 | 75.54 | 75.55 | 75.56 | 75.55 | 75 |

The parent (unmodified) construct is approximately 50% degraded after 30 minutes and nearly gone after 4 hours (completely gone at 6 hours). In contrast, the alternating 2'-O-methyl/2'-fluoro construct remains relatively unchanged and 75% remains even after 6 hours.

### Example 45: In vivo inhibition of survivin expression in a human glioblastoma xenograft tumor model

The U-87MG human glioblastoma xenograft tumor model (Kiaris et al., 2000, May-Jun; 2(3):242-50) was used to demonstrate the antitumor activity of selected compositions of the present invention. A total of 8 CD1 nu/nu (Charles River) mice were used for each group. For implantation, tumor cells were trypsinized, washed in PBS and resuspended in PBS at 4 X 10⁶ cells/mL in DMEM. Just before implantation, animals were irradiated (450 TBI) and the cells were mixed in Matrigel (1:1). A total of 4 X 10⁶ tumor cells in a 0.2 mL volume were injected subcutaneously (s.c.) in the left rear flank of each mouse. Treatment with the selected double stranded compositions (dissolved in 0.9% NaCl, injection grade), or vehicle (0.9% NaCl) was started 4 days post tumor cell implantation. The compositions were administered intravenously (i.v.) in a 0.2 mL volume eight hours apart on day one and four hours apart on day two. Tissues (tumor, liver, kidney, serum) were collected two hours after the last dose. Tumors from eight animals from each group were homogenized for western evaluation. Survivin levels were determined and compared to saline controls.

| **SEQ ID No/ISIS No** | **Sequence 5'-3'** |
|---|---|
| 24/343868 (as) | UUUGAAAAUGUUGAUCUCC |
| 25/343867 (s) | GGAGAUCAACAUUUUCAAA |
| 24/355713 (as) | UₘU_{f}UₘG_{f}AₘArAₘA_{f}UₘG_{f}UₘUtGₘA_{f}UₘC_{f}UₘC_{f}Cₘ |
| 25/355714 (s) | G_{f}GₘA_{f}GₘA_{f}UₘC_{f}AₘA_{f}CₘA_{f}UₘU_{f}UmU_{f}CₘA_{f}AₘA_{f} |
| 24/353537 (as) | UₜUₜUₜGAAAAUGUUGAUCUₜCₜCₜ |
| 25/343868 (s) | GGAGAUCAACAUUUUCAAA |
| 24/352506 (as) | UUUGAAₘAₘAUGₘUₘUGAUCUₘCₘCₘ |
| 25/352514 (s) | GGₑAGₑAUₑCAₑACₑAUₑUUₑUCₑAAₑA |

| **Double stranded construct** | | **Activity** |
|---|---|---|
| **Antisense** | **Sense** | **% Inhibition of Survivin** |
| 343868 | 343867 | none |
| 355713 | 355714 | 60 |
| 353537 | 343868 | 48 |
| 352506 | 352514 | 44 |

The data demonstrate that modified chemistries can be used to stabilize the constructs resulting in activity not seen with the unmodified construct.

### Example 46: Chemically modified siRNA targeted to PTEN: in vivo study

Six- to seven-week old Balb/c mice (Jackson Laboratory, Bar Harbor, ME) were treated with oligomeric compounds targeted to PTEN. Each treatment group was comprised of five animals. Animals were dosed via intraperitoneal injection daily for four days. Saline-injected animals served as negative controls. Animals were sacrificed 24 hours after the last dosing and liver and spleen samples were harvested. Target reduction in liver was also measured at the conclusion of the study.

Included in the study were chemically modified siRNA duplexes, one unmodified siRNA duplex and a single strand 5-10-5 MOE gapmer (116847, SEQ ID NO: 67). The animals were dosed at 80 mg/kg (duplexes) and 40 mg/kg (single strand) daily. The siRNA duplexes were composed of an antisense strand targeted to mouse PTEN paired with a sense strand. The single strand 5-10-5 MOE gapmer (116847, SEQ ID NO: 67) and the antisense strands of the siRNA duplexes used in this experiment also have perfect complementarity with human PTEN.

| **SEQ ID No/ S/AS ISIS No** | | **Sequence 5'-3'** |
|---|---|---|
| 63/116847 | AS | CₑTₑGₑCₑTₑAGCCTCTGGATₑTₑTₑGₑAₑ |
| 27/341401 | S | AAGUAAGGACCAGAGACAA |
| 26/341391 | AS | P-UUGUCUCUGGUCCUUACUU |
| 64/398249 | S | A_{f}AₘG_{f}UₘA_{f}AₘG_{f}GₘA_{f}CₘC_{f}AₘG_{f}AₘG_{f}AₘC_{f}AₘA_{f}UₑUₑ-L-C₁₆ |
| 65/398262 | AS | P-UₘU_{f}GₘU_{f}CₘU_{f}CₘU_{f}GₘG_{f}UₘC_{f}CₘU_{f}UₘA_{f}CₘU_{f}UₘUₑUₑ |
| 65/398256 | AS | P-UₘU_{f}GₘU_{f}CₘU_{f}CₘU_{f}GₘG_{f}UₘC_{f}CₘU_{f}UₘA_{f}CₘU_{f}UₘUₑUₑ |
| 64/398239 | S | A_{f}AₘG_{f}UₘAₑAₘCT_{f}GₘA_{f}CₘC_{f}AₘG_{f}AₘG_{f}AₘC_{f}AₘA_{f}UₑUₑ |

The sense strand (398249) has a C₁₆ lipophilic conjugate group attached at the 3'-end via a bivalent linking group (L) which is further attached to a phosphorothioate group. The structure of the entire group which is attached to the 3'-carbon of the terminal nucleoside is shown below:

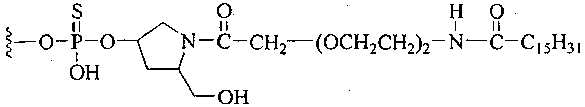

PTEN mRNA levels in liver were measured at the end of the study using real-time PCR and RIBOGREEN® RNA quantification reagent (Molecular Probes, Inc. Eugene, OR) according to standard protocols. PTEN mRNA levels were determined relative to total RNA (using Ribogreen), prior to normalization to saline-treated control. Results are presented in the table below as the average % inhibition of mRNA expression for each treatment group, normalized to saline-injected control.

| **Compound (sense/antisense)** | **Dose (mg/kg)** | **% Inhibition** |
|---|---|---|
| 116847 | 40 | 79 |
| 398249/398262 | 80 | 52 |
| 398249/398256 | 80 | 39 |
| 398239/398256 | 80 | 0 |
| 341401/341391 | 80 | 0 |

In vivo activity was demonstrated for siRNA duplexes having a linked conjugate at the 3'-end of the sense strand and an overhang of 2 nuclease resistant 2'-modified nucleosides at the 3'-end of the antisense strand. The use of at least two phosphorothioate internucleoside linkages at the 3'-end of the antisense strand may also be important for in vivo activity. When the number of phosphorothioate internucleoside linkages at the 3'-end of the antisense strand was increased from 2 (398249/398256) to 7 (398249/398262) a further increase in activity was observed. Removal of the conjugate group resulted in no measurable activity (398239/39825).

In addition to PTEN mRNA levels, liver transaminase levels, alanine aminotranferease (ALT) and aspartate aminotransferase (AST), in serum were also measured relative to saline injected mice. The approximate liver transaminase levels are listed in the table below.

| **Compound** | **Dose (mg/kg)** | **ALT (% change)** | **AST (% change)** |
|---|---|---|---|
| 116847 | 40 | 33 | 70 |
| 398249/398262 | 80 | -30 | 0 |
| 398249/398256 | 80 | -33 | 23 |
| 398239/398256 | 80 | -23 | 30 |
| 341401/341391 | 80 | -28 | -20 |

The measured transaminase levels for mice treated with the siRNA duplexes, 398249/398262, 398249/398256 and 341401/341391 were not elevated to a level indicative of hepatotoxicity.

The effects on liver and spleen weights were also determined. Significant changes in liver and spleen weight can indicate that a particular compound causes toxic effects. The data are expressed as percent change in body or organ weight ("+" indicates an increase, indicates a decrease). The results are listed in the table below.

| **Compound** | **Dose (mg/kg)** | **Liver weight** | **Spleen weight** |
|---|---|---|---|
| Saline | N/A | 1.12 | 0.08 |
| 116847 | 40 | 1.42 (+27%) | 0.08 |
| 398249/398262 | 80 | 1.08 (-4%) | 0.09 (+12%) |
| 398249/398256 | 80 | 1.20 (+7%) | 0.10 (+25%) |
| 398239/398256 | 80 | 1.30 (+16%) | 0.12 (+50%) |
| 341401/341391 | 80 | 1.10 (-2%) | 0.08 |

As listed in the table, treatment with siRNA duplexes, 398249/398262 and 398249/398256, did not alter spleen weights, within experimental error, in normal mice as compared to the organ weights of mice treated with saline alone to a level that would indicate a significant toxic effect. The changes in organ weight were within the normal range seen when animals are treated with oligonucleotides that are considered nontoxic.

The accumulation of siRNAs in liver was also determined histologically following routine procedures. Liver samples were procured, fixed in 10% neutral-buffered formalin and processed for staining with hematoxylin and eosin, to visualize nuclei and cytoplasm, and with an anti-oligonucleotide rabbit polyclonal antisera raised against a keyhole limpet hemocyanin-ISIS 116847 conjugate in order to assess oligonucleotide staining patterns. Hematoxylin-and eosin staining in most tissues exhibited no significant difference between saline- and oligonucleotide-treated animals. The rabbit polyclonal antibodies were recognized using an isospecific anti-IgG2 horse-radish peroxidase-conjugated secondary antibody (Zymed, San Francisco, CA) and immunostaining was developed with 3,3'-diaminobenzidene (DAKO, Carpenteria, CA). The results demonstrated that mice treated with the chemically modified siRNA 398249/398262, have significant increased liver accumulation of the siRNA as compared to the unmodified control, 341401/341391. 398249/398262 has also shown increased liver accumulation as compared to the other two chemically modified siRNAs, 398239/398256 and 398249/398256, and the MOE gapmer control 116847.

The PTEN protein levels were also determined for by Western blot analysis (immunoblot analysis) using standard methods. Liver samples from the treated mice were homogenized and diluted in Laemmli buffer (100 ul/well), boiled for 5 minutes and and equal amounts of protein were loaded on a 10-16% SDS-PAGE gel. Gels are run for 1-2 hours at 100-150 V, and transferred to membrane for western blotting. Appropriate primary antibody directed to PTEN (anti-rabbit IgG; HRP-conjugated from Upstate, cat # 07-016; lot 4 26560) is used, and detected with an enzyme-linked secondary antibody (Goat anti-rabbit IgG; HRP-conjugted from Upstate, cat # 12-348; lot # 32628) directed against the primary antibody species. Bands are visualized/quantified using an Image Quank, version 5.2 (Molecular Dynamics, Sunnyvale Calif.).

The protein levels were decreased by 24-fold, 7-fold and 2.8-fold with treatment of 116847, 398249/398262 and 398239/398256 respectively. The mean values for the protein levels correlated with the measured mRNA levels.

## Claims

1. A composition comprising first and second chemically synthesized oligomeric compounds, wherein:
the first oligomeric compound, the antisense strand, comprises a hybridizing region that is fully complementary to a nucleic acid target and has the formula:
5'-(Nᵢ-L)₂-(Nₘ-L)₃-(Nₙ-L)₂-(Nₘ-L)₂-(Nₙ-L)₂-(Nₘ-L)₅-(Nₙ-L)₃-3'
wherein
each Nᵢ is a 4'-thio modified nucleoside or a 2'-F modified nucleoside; each Nₘ is a 2'-F modified nucleoside;
each Nₙ is a 2'-OCH₃ modified nucleoside;
each L is an internucleoside linkage;
the second oligomeric compound, the sense strand, comprises a hybridizing region that is fully complementary to the hybridizing region of the first oligomeric compound and comprises a continuous sequence of nucleosides that define an alternating motif comprising 2'-O(CH₂)₂OCH₃;
each of the first and second oligomeric compounds can comprise one or more optional groups independently selected from a 3'-capping group, a 5'-capping group, a 5'-phosphate moiety, a 3'-linked conjugate group, a non hybridizing 3'-overhang region and a non hybridizing 3'-overhang region that is linked to a conjugate group; and
the first and second oligomeric compounds form an siRNA.

2. The composition of claim 1 wherein at least one Nᵢ is a 4'-thio modified nucleoside.

3. The composition of claim 2 wherein each Nᵢ is a 4'-thio modified nucleoside.

4. The composition of claim 1 wherein each Nᵢ is a 2'-F modified nucleoside.

5. The composition of claim 1 wherein each L is, independently, a phosphorothioate or phosphodiester internucleoside linkage.

6. The composition of claim 1 wherein the first oligomeric compound comprises a 5'-phosphate moiety.

7. The composition of claim 1 wherein the first oligomeric compound comprises a non hybridizing 3'-overhang region.

8. The composition of claim 7 wherein the first oligomeric compound further includes a 5'-phosphate moiety.

9. The composition of claim 7 wherein the non hybridizing 3'-overhang region of the first oligomeric compound comprises 2 2'-modified nucleosides.

10. The composition of claim 1 wherein the second oligomeric compound includes a non hybridizing 3'-overhang region.

11. The composition of claim 10 wherein the second oligomeric compound further includes a conjugate group linked to the 3'-overhang region.

12. The composition of claim 1 wherein from 2 to 10 of the internucleoside linkages of the first oligomeric compound are phosphorothioate internucleoside linkages.

13. The composition of claim 1 wherein from 1 to 10 of the internucleoside linkages of the second oligomeric compound are phosphorothioate internucleoside linkages.

14. The composition of claim 1 wherein each 2'-modified nucleoside, of each non hybridizing overhang region, comprises a 2'-substituent group independently selected from -O-C₁₋₆-alkyl, substituted -O-C₁₋₆-alkyl, substituted -O-C₁₋₆-alkyl-O-C₁₋₆-alkyl, -O-C₂₋₆-alkenyl, substituted -O-C₂₋₆-alkenyl, -O-C₂₋₆-alkynyl, substituted -O-C₂₋₆-alkynyl, substituted -O-acetamide (-O-CH₂C)=O)-N(-)₂) or allyl;
each substituent group is, independently, halogen, -O-, -N(R₁)- or -S- C₁₋₆-alkyl; substituted -O-, -N(R₁)-, or -S- C₁₋₆-alkyl, -O-, -N(R₁)-, or -S- C₂₋₆-alkenyl, substituted - O-, -N(R₁)-, or -S- C₂₋₆-alkenyl; -O-N(R₁)(R₂) or -N(R₁)(R₂); and
each R₁ and R₂ is, independently, H, -C₁₋₆-alkyl, substituted -C₁₋₆-alkyl or an amino protecting group.

15. The composition of claim 14 wherein each 2'-substituent group is, independently, -O-CH₃, -O-(CH₂)₂-OCH₃, -O-(CH₂)₂-O-N(CH₃)₂ or O-CH₂-C(=O)N(H)(CH₃).

16. The composition of claim 1 comprising at least one conjugate group selected from peptides, proteins, sterols, lipids, phospholipids, biotin, phenoxazines, an active drug substance or folates.

17. The composition of claim 16 wherein the conjugate is cholesterol or a lipid.

18. The composition of claim 16 wherein the lipid is a C₈-C₁₈ lipid.

19. The composition of claim 16 wherein the conjugate is an active drug substance.

20. An in vitro method of inhibiting gene expression comprising contacting one or more cells or a tissue with a composition of claim 1.

## Patentansprüche

1. Zusammensetzung, umfassend erste und zweite chemisch synthetisierte oligomere Verbindungen, wobei:
die erste oligomere Verbindung, der Antisense-Strang, einen Hybridisierungsbereich umfasst, der vollständig komplementär zu einem Nukleinsäureziel ist und die Formel
5'-(Nᵢ-L)₂-(Nₘ-L)₃₋(Nₙ-L)₂-(Nₘ-L)₂₋(Nₙ-L)₂-(Nₘ-L)₅-(Nₙ-L)₃-3'
aufweist, wobei
Nᵢ jeweils für ein 4'-thio-modifiziertes Nukleosid oder ein 2'-F-modifiziertes Nukleosid steht;
Nₘ jeweils für ein 2'-F-modifiziertes Nukleosid steht;
Nₙ jeweils für ein 2'-OCH₃-modifiziertes Nukleosid steht;
L jeweils für eine Internukleosid-Verknüpfung steht;
die zweite oligomere Verbindung, der Sense-Strang, einen Hybridisierungsbereich umfasst, der vollständig komplementär zum Hybridisierungsbereich der ersten oligomeren Verbindung ist und eine fortlaufende Sequenz von Nukleosiden umfasst, die ein 2'-O(CH₂)₂OCH₃ umfassendes alternierendes Motiv definieren;
wobei die ersten und zweiten oligomeren Verbindungen jeweils eine oder mehrere beliebige, unabhängig aus einer 3'-Capping-Gruppe, einer 5'-Capping-Gruppe, einer 5'-Phosphat-Gruppierung, einer 3'-verknüpften Konjugatgruppe, einem nicht hybridisierenden 3'-Überhangbereich und einem nicht hybridisierenden 3'-Überhangbereich, der mit einer Konjugatgruppe verknüpft ist, ausgewählte Gruppen umfassen können; und
die ersten und zweiten oligomeren Verbindungen eine siRNA bilden.

2. Zusammensetzung nach Anspruch 1, wobei wenigstens ein Nᵢ für ein 4'-thio-modifiziertes Nukleosid steht.

3. Zusammensetzung nach Anspruch 2, wobei Nᵢ jeweils für ein 4'-thio-modifiziertes Nukleosid steht.

4. Zusammensetzung nach Anspruch 1, wobei Nᵢ jeweils für ein 2'-F-modifiziertes Nukleosid steht.

5. Zusammensetzung nach Anspruch 1, wobei L jeweils unabhängig für eine Phosphorothioat- oder Phosphodiester-Internukleosid-Verknüpfung steht.

6. Zusammensetzung nach Anspruch 1, wobei die erste oligomere Verbindung eine 5'-Phosphat-Gruppierung umfasst.

7. Zusammensetzung nach Anspruch 1, wobei die erste oligomere Verbindung einen nicht hybridisierenden 3'-Überhangbereich umfasst.

8. Zusammensetzung nach Anspruch 7, wobei die erste oligomere Verbindung ferner eine 5'-Phosphat-Gruppierung enthält.

9. Zusammensetzung nach Anspruch 7, wobei der nicht hybridisierende 3'-Überhangbereich der ersten oligomeren Verbindung 2 2'-modifizierte Nukleoside umfasst.

10. Zusammensetzung nach Anspruch 1, wobei die zweite oligomere Verbindung einen nicht hybridisierenden 3'-Überhangbereich enthält.

11. Zusammensetzung nach Anspruch 10, wobei die zweite oligomere Verbindung ferner eine mit dem 3'-Überhangbereich verknüpfte Konjugatgruppe enthält.

12. Zusammensetzung nach Anspruch 1, wobei es sich bei 2 bis 10 der Internukleosid-Verknüpfungen der ersten oligomeren Verbindung um Phosphorothioat-Internukleosid-Verknüpfungen handelt.

13. Zusammensetzung nach Anspruch 1, wobei es sich bei 1 bis 10 der Internukleosid-Verknüpfungen der zweiten oligomeren Verbindung um Phosphorothioat-Internukleosid-Verknüpfungen handelt.

14. Zusammensetzung nach Anspruch 1, wobei jedes 2'-modifizierte Nukleosid eines jeden nicht hybridisierenden Überhangbereichs eine unabhängig aus -O-C₁₋₆-Alkyl, substituiertem -O-C₁₋₆-Alkyl, substituiertem -O-C₁₋₆-Alkyl-O-C₁₋₆-alkyl, -O-C₂₋₆-Alkenyl, substituiertem -O-C₂₋₆-Alkenyl, -O-C₂₋₆-Alkinyl, substituiertem -O-C₂-₆-Alkinyl, substituiertem -0-Acetamid (-O-CH₂C)=O)-N(-)₂) oder Allyl ausgewählte 2'-Substituentengruppe umfasst;
es sich bei der Substituentengruppe jeweils unabhängig um Halogen, -O-, -N(R₁)- oder -S-C₁₋₆-Alkyl; substituiertes -O-, -N(R₁)- oder -S-C₁₋₆-Alkyl, -O-, -N(R₁)- oder -S-C₂₋₆-Alkenyl, substituiertes -0-, -N(R₁)- oder -S-C₂₋₆-Alkenyl; -O-N(R₁) (R₂) oder -N(R₁) (R₂) handelt; und
R₁ und R₂ jeweils unabhängig für H, -C₁₋₆-Alkyl, substituiertes -C₁₋₆-Alkyl oder eine Amino-Schutzgruppe stehen.

15. Zusammensetzung nach Anspruch 14, wobei es sich bei der 2'-Substituentengruppe jeweils unabhängig um -O-CH₃, -O-(CH₂)₂-OCH₃, -O- (CH₂)₂-O-N (CH₃)₂ oder O-CH₂-C (=0) -N (H) (CH₃) handelt.

16. Zusammensetzung nach Anspruch 1, umfassend wenigstens eine aus Peptiden, Proteinen, Sterinen, Lipiden, Phospholipiden, Biotin, Phenoxazinen, einem Arzneimittelwirkstoff oder Folaten ausgewählte Konjugatgruppe.

17. Zusammensetzung nach Anspruch 16, wobei es sich bei dem Konjugat um Cholesterin oder ein Lipid handelt.

18. Zusammensetzung nach Anspruch 16, wobei es sich bei dem Lipid um ein C₈-C₁₈-Lipid handelt.

19. Zusammensetzung nach Anspruch 16, wobei es sich bei dem Konjugat um einen Arzneimittelwirkstoff handelt.

20. In-vitro-Verfahren zur Hemmung der Genexpression, umfassend Inkontaktbringen einer oder mehrerer Zellen oder eines Gewebes mit einer Zusammensetzung nach Anspruch 1.

## Revendications

1. Composition comprenant des premier et deuxième composés oligomères chimiquement synthétisés, dans laquelle :
le premier composé oligomère, le brin antisens, comprend une région d'hybridation qui est totalement complémentaire d'un acide nucléique cible et a la formule : 5'-(Ni-L)₂-(Nₘ-L)₃-(Nₙ-L)₂-(Nₘ-L)₂-(Nₙ-L)₂-(Nₘ-L)₅-(Nₙ-L)₃-3' où chaque Nᵢ est un nucléoside modifié 4'-thio ou un nucléoside modifié 2'-F ;
chaque Nₘ est un nucléoside modifié 2'-F ;
chaque Nₙ est un nucléoside modifié 2'-OCF₃ ;
chaque L est un lieur internucléosidique ;
le deuxième composé oligomère, le brin sens, comprend une région d'hybridation qui est totalement complémentaire de la région d'hybridation du premier composé oligomère et comprend une séquence continue de nucléosides qui définit un motif alterné comprenant 2'-O(CH₂)₂OCH₃ ;
chacun des premier et deuxième composés oligomères peut comprendre un ou plusieurs groupes facultatifs indépendamment choisis parmi un groupe de coiffe 3', un groupe de coiffe 5', un fragment 5'-phosphate, un groupe conjugué 3'-lié, une région d'extrémité saillante 3' non hybridante et une région d'extrémité saillante 3' non hybridante qui est liée à un groupe conjugué ; et les premier et deuxième composés oligomères forment un ARNsi.

2. Composition de la revendication 1 dans laquelle au moins un Ni est un nucléoside modifié 4'-thio.

3. Composition de la revendication 2 dans laquelle chaque Ni est un nucléoside modifié 4'-thio.

4. Composition de la revendication 1 dans laquelle chaque Ni est un nucléoside modifié 2'-F.

5. Composition de la revendication 1 dans laquelle chaque L est, indépendamment, un lieur internucléosidique phosphorothioate ou phosphodiester.

6. Composition de la revendication 1 dans laquelle le premier composé oligomère comprend un fragment 5'-phosphate.

7. Composition de la revendication 1 dans laquelle le premier composé oligomère comprend une région d'extrémité saillante 3' non hybridante.

8. Composition de la revendication 7 dans laquelle le premier composé oligomère comprend en outre un fragment 5'-phosphate.

9. Composition de la revendication 7 dans laquelle la région d'extrémité saillante 3' non hybridante du premier composé oligomère comprend 2 nucléosides 2'-modifiés.

10. Composition de la revendication 1 dans laquelle le deuxième composé oligomère comprend une région d'extrémité saillante 3' non hybridante.

11. Composition de la revendication 10 dans laquelle le deuxième composé oligomère comprend en outre un groupe conjugué groupe lié à la région d'extrémité saillante 3'.

12. Composition de la revendication 1 dans laquelle de 2 à 10 des lieurs internucléosidiques du premier composé oligomère sont des lieurs internucléosidiques phosphorothioate.

13. Composition de la revendication 1 dans laquelle de 1 à 10 des lieurs internucléosidiques du deuxième composé oligomère sont des lieurs internucléosidiques phosphorothioate.

14. Composition de la revendication 1 dans laquelle chaque nucléoside 2'-modifié, de chaque région d'extrémité saillante non hybridante, comprend un groupe substituant 2' indépendamment choisi parmi -O-(alkyle en C1-6), -O-(alkyle en C1-6) substitué, -O-(alkyle en C1-6)-O-(alkyle en C1-6) substitué, -O-(alcényle en C2-6), -0-(alcényle en C2-6) substitué, - O-(alcynyle en C2-6), -0-(alcynyle en C2-6) substitué, -0-acétamide substitué (-O-CH₂C)=O)-N(-)₂) ou allyle ;
chaque groupe substituant est, indépendamment, halogène, -0-, -N(R₁)- ou -S-(alkyle en C1-6) ; -O-, - N(R₁)- ou -S-(alkyle en C1-6) substitué, -0-, -N(R₁)- ou -S-(alcényle en C2-6), -O-, -N(R₁)- ou -S- (alcényle en C2-6) substitué ; -O-N(R₁)(R₂) ou -N(R₁)(R₂) ; et
chaque R₁ et R₂ est, indépendamment, H, - (alkyle en C1-6), -(alkyle en C1-6) substitué ou un groupe protecteur d'amino.

15. Composition de la revendication 14 dans laquelle chaque groupe substituant 2' est, indépendamment, -O-CH₃, -O- (CH₂)₂-OCH₃, -O- (CH₂) ₂-O-N(CH₃)₂ ou O-CH₂-C(=O)-N(H)(CH₃).

16. Composition de la revendication 1 comprenant au moins un groupe conjugué choisi parmi des peptides, des protéines, des stérols, des lipides, des phospholipides, la biotine, des phénoxazines, une substance pharmaceutique active ou des folates.

17. Composition de la revendication 16 dans laquelle le conjugué est le cholestérol ou un lipide.

18. Composition de la revendication 16 dans laquelle le lipide est un lipide en C₈-C₁₈.

19. Composition de la revendication 16 dans laquelle le conjugué est une substance pharmaceutique active.

20. Procédé *in vitro* d'inhibition d'expression génique comprenant la mise en contact d'une ou plusieurs cellules ou d'un tissu avec une composition de la revendication 1.
